# EUROPEAN PATENT APPLICATION

(11) **EP 3 456 327 A1**
(43) Date of publication of application: **20.03.2019**
(21) Application number: 17796233.9
(22) Date of filing: 11.05.2017
(51) Int. Cl.: A61K 31/421, A61K 31/422, A61K 31/426, A61K 31/427, A61P 17/02, A61P 43/00

(54) **WOUND-HEALING AGENT**

(30) Priority: 12.05.2016 JP 2016095793
(71) Applicant: Nippon Chemiphar Co., Ltd., Tokyo 101-0032 (JP)
(72) Inventor: HIRAI, Toshitake, Misato-shi Saitama 341-0005 (JP); HAMANO, Takaichi, Tokyo 1010032 (JP); YAMAKAWA, Tomio, Misato-shi Saitama 341-0005 (JP)
(74) Representative: Beckmann, Claus
(86) International application number: PCT/JP2017/017945
(87) International publication number: WO 2017/195875

(57) **Abstract**

A wound-healing agent is provided by using a benzisoxazole compound represented by the following general formula (I), a tautomer, a stereoisomer, or a pharmaceutically acceptable salt of the compound, a solvate of any of the foregoing, or the like, which exerts a PPARδ agonist effect.

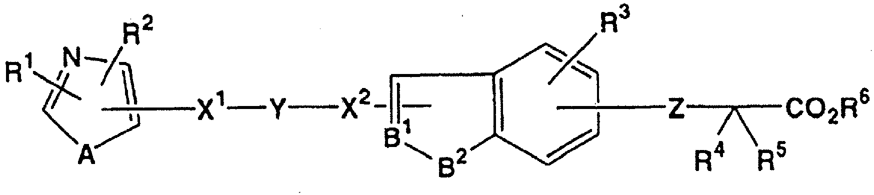

## Description

### Technical Field

The present invention relates to a wound-healing agent comprising a PPARδ agonist.

### Background Art

Intractable wounds are wounds that do not heal within 3 to 4 weeks, and they include pressure ulcer, diabetic ulcer, venous stasis ulcer, and the like, as well as burn wounds accompanied by infection, and intractable open wounds that developed due to wound infection (Non-patent document 1). Among these, as for intractable wounds caused by diabetes, it has been reported that they are caused due to delay of neovascularization, dysfunction of macrophages and fibroblasts, decrease of migration ability of epidermal cells, and the like (Non-patent document 2).

The wound healing process refers to a series of repair processes where damaged skins and subcutaneous tissues are reconstructed repeatedly as a barrier, and it is divided into an inflammation phase, a proliferation phase and a maturation phase (Non-patent document 1). In the first inflammation phase, bleeding and clotting and tissue debridement take place. Granulation and epithelization are promoted, and wounds are closed in the proliferation phase. In the maturation phase, which is the final phase of the wound healing process, reconstruction of the extracellular matrix and apoptosis take place, and wounds become matured scars (Non-patent document 1).

During the respective phases of wound healing, humoral growth factors including specific cytokines are released. In the inflammation phase, cells such as thrombocytes, polynuclear leucocytes, and macrophages are predominant. Cytokines such as TGF-β and PDGF are released, and cause accumulation of inflammatory cells such as lymphocytes and monocytes, migration of macrophages, and phagocytosis (Non-patent document 1). The humoral growth factors released from macrophages promote proliferation of vascular endothelial cells, fibroblasts, and epidermal cells to shift the wound healing process to the proliferation phase. When the process advances to the proliferation phase, apoptosis of monocytes and macrophages is induced, and granulation gradually is formed. In this phase, fibroblasts, vascular endothelial cells, and keratinocytes are predominant, and fibroblasts secrete collagen, which promotes the synthesis of the extracellular matrix together with the action of TGF-β. The vascular endothelial cells cause neovascularization, granulation tissues are thereby formed, wound constriction is caused by myofibroblasts, and epithelialization by keratinocytes advances. When the process reached the maturation phase, reconstruction of scar tissues is caused by cell death of the myofibroblasts and vascular endothelial cells, and crosslinking of collagen fibers. As a result, redness of the scar decreases, and the scar is flattened, and finally becomes a white scar (Non-patent document 3).

There has so far been known presence of three subtypes of the peroxisome proliferator activated receptor (PPAR) according to broad classification, and they are referred to as PPARα, PPARγ, and PPARδ (Non-patent document 4). PPARα is expressed in fat, liver, heart, and the like, and mainly involved in the lipid metabolism. PPARγ exists in fat cells, sebaceous gland cells, immunocytes such as macrophages and dendritic cells, and the like, and is involved in the immunological responses such as inflammation, cell proliferation and differentiation, apoptosis, and the like (Non-patent document 3). On the other hand, PPARδ is expressed more in the skin compared with PPARα and PPARγ, and it has been reported to participate in the differentiation of keratinocytes and restoration of the skin barrier function (Non-patent document 3). It has also been reported that the roles of PPARs are important also for wound healing, and it is considered that PPARα is involved in the skin healing via modulation of the inflammation phase, PPARδ protects keratinocytes from TNF-α-induced apoptosis and thus it is involved in cell survival in the wound healing process (Non-patent document 3). By such protection from apoptosis, migration of keratinocytes is maintained for the re-epithelialization phase of the wound healing process (Non-patent document 5).

So far to date, as a PPARδ-selective agonist, benzisoxazole derivatives and the like have been reported (Patent documents 1 to 4).

### Background art references

### Patent documents

Patent document 1: WO03/033493
Patent document 2: WO03/016291
Patent document 3: WO2007/119887
Patent document 4: WO2009/128558
Non-patent documents

Non-patent document 1: Journal of clinical and experimental medicine (Igaku No Ayumi), Vol. 237, No. 1
Non-patent document 2: Brem H, Tomic-Canic M, Cellular and molecular basis of wound healing in diabetes, J. Clin. Invest., 117:1219-1222, 2007
Non-patent document 3: Lorenz HP, Longaker MT, Plastic Surgery 2nd ed. (ed. by Mathes, SJ), Saunders, Philadelphia, 2006, pp.209-217
Non-patent document 4: Gupta M, Mahajan VK, Mehta KS, Chauhan PS, Rawat R, Peroxisome proliferator-activated receptors (PPARs) and PPAR agonists: the 'future' in dermatology therapeutics, Arch. Dermatol. Res., 307:767-780, 2015
Non-patent document 5: Montagner A, Wahli W, Tan NS., Nuclear receptor peroxisome proliferator activated receptor (PPAR) 6/8 in skin wound healing and cancer, Eur. J. Dermatol. Suppl., 1:4-11, 2015

### Summary of the Invention

### Object to be Achieved by the Invention

An object of the present invention is to provide a pharmaceutical composition for wound treatment, which comprises a PPARδ agonist.

Another object of the present invention is to provide a pharmaceutical composition for wound treatment, which shortens a period required for recovery of wound.

Another object of the present invention is to provide a pharmaceutical composition for wound treatment, which suppresses exacerbation of wound.

### Means for Achieving the Object

The present invention provides a pharmaceutical composition for wound treatment, which comprises a compound represented by the general formula (I), a tautomer, a stereoisomer, or a pharmaceutically acceptable salt of the compound, or a solvate of any of the foregoing: 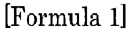 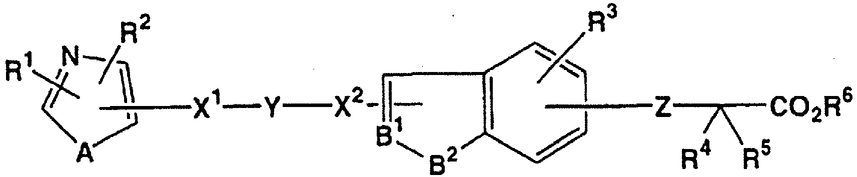
wherein A represents O, S, or NR⁷, wherein
R⁷ represents hydrogen atom, or an alkyl group having 1 to 8 carbon atoms,
B¹ represents CW or N, wherein
W represents hydrogen atom, or an atomic bond,
B² represents O, S, or NR⁸, wherein
R⁸ represents hydrogen atom, or an alkyl group having 1 to 8 carbon atoms,
X¹ and X² represent O, S, NH, NHC(=O), C(=O), C(=N-OR⁹), CH(OR¹⁰), C=C, C≡C, or an atomic bond, wherein
R⁹ and R¹⁰ represent hydrogen atom, or an alkyl group having 1 to 8 carbon atoms,
Y represents an alkylene chain having 1 to 8 carbon atoms which may have an alkyl group having 1 to 8 carbon atoms, or an alkyl group having 1 to 8 carbon atoms and substituted with 1 to 3 halogen atoms as a substituent,
Z represents NH, O, or S,
R¹ represents an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent, or a heterocyclic group having a 5- to 8-membered ring comprising 1 to 3 heteroatoms selected from nitrogen atom, oxygen atom, and sulfur atom, and the remainder carbon atoms as ring-constituting atoms (benzene ring may further condense to this heterocyclic ring),
R² represents an alkyl group having 2 to 8 carbon atoms, an alkyl group having 1 to 8 carbon atoms and substituted with 1 to 3 halogen atoms, a cycloalkyl group having 3 to 7 carbon atoms, an alkenyl group having 2 to 8 carbon atoms, an alkynyl group having 2 to 8 carbon atoms, an alkyl group substituted with an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent (the alkyl moiety thereof has 1 to 4 carbon atoms), or an alkyl group substituted with a 5- to 8-membered heterocyclic ring (the heterocyclic ring thereof comprises 1 to 3 heteroatoms selected from nitrogen atom, oxygen atom, and sulfur atom, and the remainder carbon atoms as ring-constituting atoms, and the alkyl moiety thereof has 1 to 4 carbon atoms),
R³ represents hydrogen atom, a halogen atom, trifluoromethyl group, an alkyl group having 1 to 8 carbon atoms, an alkenyl group having 2 to 8 carbon atoms, or an alkynyl group having 2 to 8 carbon atoms,
R⁴ and R⁵ represent hydrogen atom, an alkyl group having 1 to 8 carbon atoms, or an alkyl group having 1 to 8 carbon atoms and substituted with 1 to 3 halogen atoms, and
R⁶ represents hydrogen atom, an alkyl group having 1 to 8 carbon atoms and substituted with an amino group, an alkyl group having 1 to 8 carbon atoms, or an alkali metal,
provided that Z and R³ bond to the benzene ring, and X² does not bond to the benzene ring;
a compound represented by the general formula (II), a tautomer, a stereoisomer, or a pharmaceutically acceptable salt of the compound, or a solvate of any of the foregoing:

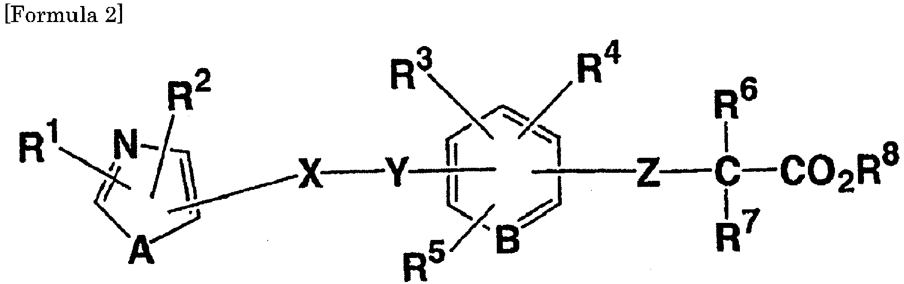

wherein R¹ represents a phenyl group, naphthyl group, pyridyl group, thienyl group, furyl group, quinolyl group, or benzothienyl group which may have a group or an atom selected from an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent, R² represents an alkyl group having 1 to 8 carbon atoms, an alkyl group having 1 to 8 carbon atoms and substituted with a halogen atom, an alkenyl group having 2 to 8 carbon atoms, an alkynyl group having 2 to 8 carbon atoms, a cycloalkyl group having a 3- to 7-membered ring, an alkyl group having 1 to 8 carbon atoms and substituted with a cycloalkyl group having a 3- to 7-membered ring, a phenyl group which may have a group or an atom selected from an alkoxy group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkynyl group having 2 to 8 carbon atoms, a halogen atom, an acyl group having 2 to 7 carbon atoms, a benzoyl group, a hydroxyl group, a nitro group, an amino group, a phenyl group, and a pyridyl group, a naphthyl group, or an alkyl group having 1 to 6 carbon atoms substituted with a pyridyl group, A represents oxygen atom, sulfur atom, or NR⁹, wherein R⁹ represents hydrogen atom, or an alkyl group having 1 to 8 carbon atoms, X represents an alkylene chain having 1 to 8 carbon atoms which may have a group selected from an alkyl group having 1 to 8 carbon atoms, an alkoxy group having 1 to 8 carbon atoms, and hydroxyl group as a substituent, and may contain a double bond, Y represents C(=O), C(=NOR¹⁰), CH(OR¹¹), CH=CH, C≡C, or C(=CH₂), wherein R¹⁰ and R¹¹ represent hydrogen atom, an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, or pyridyl group, B represents CH or nitrogen atom, Z represents oxygen atom or sulfur atom, R⁶ and R⁷ represent hydrogen atom, an alkyl group having 1 to 8 carbon atoms, or an alkyl group having 1 to 8 carbon atoms and substituted with a halogen atom, and R⁸ represents hydrogen atom, or an alkyl group having 1 to 8 carbon atoms,
provided that at least one of R³, R⁴, or R⁵ is not hydrogen atom;
a compound represented by the general formula (III-I), a tautomer, a stereoisomer, or a pharmaceutically acceptable salt of the compound, or a solvate of any of the foregoing:

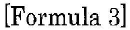

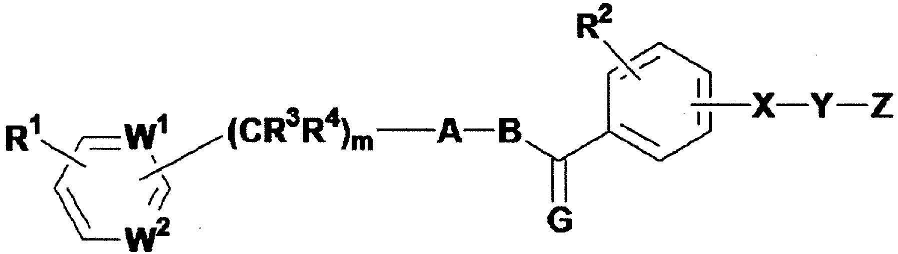

wherein W¹ and W² may be the same or different, and represent CH or nitrogen atom,
X represents NR⁵ or CR⁶R⁷, wherein R⁵ represents hydrogen atom, an alkyl group having 1 to 8 carbon atoms, an alkyl group having 1 to 8 carbon atoms and substituted with a halogen atom, an alkyl group having 1 to 8 carbon atoms and substituted with an alkoxy group having 1 to 8 carbon atoms, a cycloalkyl group having a 3- to 7-membered ring, an alkyl group having 1 to 8 carbon atoms and substituted with a cycloalkyl group having a 3- to 7-membered ring, an alkyl group having 1 to 8 carbon atoms and substituted with phenyl group, an acyl group having 2 to 8 carbon atoms, or an alkenyl group having 2 to 8 carbon atoms, and
R⁶ and R⁷ may be the same or different, and represent hydrogen atom, or an alkyl group having 1 to 8 carbon atoms,
Y represents -(CR⁸R⁹)ₙ-, wherein R⁸ and R⁹ may be the same or different, and represent hydrogen atom, or an alkyl group having 1 to 8 carbon atoms, and n represents an integer of 1 to 4, or
X and Y may bind together to represent -CR¹⁰=CR¹¹- or ethynylene, wherein R¹⁰ and R¹¹ may be the same or different, and represent hydrogen atom, or an alkyl group having 1 to 8 carbon atoms,
Z represents carboxyl group, or tetrazolyl group,
G represents O, S, or CR¹²R¹³, wherein R¹² and R¹³ may be the same or different, and represent hydrogen atom, or an alkyl group having 1 to 8 carbon atoms,
A represents a 5-membered heterocyclic ring selected from thiazole, oxazole, imidazole, pyrazole, thiophene, furan, and pyrrole which may have a substituent selected from an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms and substituted with a halogen atom, an alkoxy group having 1 to 8 carbon atoms and substituted with a halogen atom, hydroxyl group, nitro group, an acyl group having 2 to 8 carbon atoms, an aryl group having 6 to 10 carbon atoms, and a heterocyclic group having a 5- or 6-membered ring,
B represents an alkylene chain having 1 to 8 carbon atoms which may have a substituent selected from an alkyl group having 1 to 8 carbon atoms, a cycloalkyl group having a 3- to 7-membered ring, an alkoxy group having 1 to 8 carbon atoms, and a halogen atom, wherein if this alkylene chain contains 2 or more carbon atoms, it may contain a double bond or a triple bond,
R¹ and R² may be the same or different, and represent hydrogen atom, an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms and substituted with a halogen atom, an alkoxy group having 1 to 8 carbon atoms and substituted with a halogen atom, hydroxyl group, nitro group, an acyl group having 2 to 8 carbon atoms, an aryl group having 6 to 10 carbon atoms, or a heterocyclic group having a 5- or 6-membered ring,
R³ and R⁴ may be the same or different, and represent hydrogen atom, or an alkyl group having 1 to 8 carbon atoms, and
m represents an integer of 0 to 3; or
a compound represented by the general formula (IV-I), a tautomer, a stereoisomer, or a pharmaceutically acceptable salt of the compound, or a solvate of any of the foregoing:

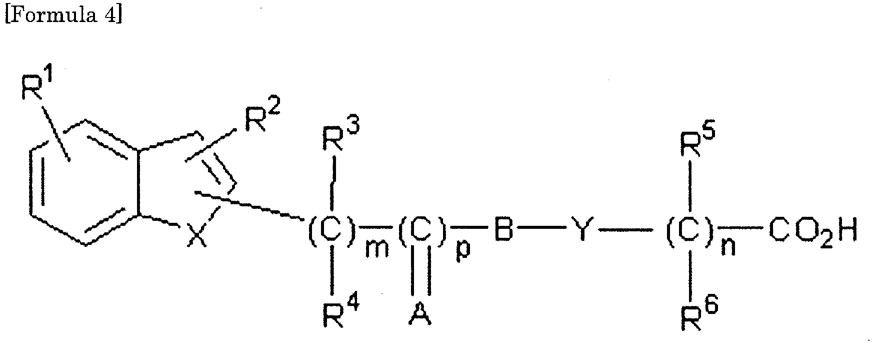

wherein R¹ represents hydrogen atom, a halogen atom, hydroxyl group, nitro group, amino group, cyano group, carboxyl group, an alkyl group having 1 to 8 carbon atoms, a cycloalkyl group having a 3- to 7-membered ring, an alkenyl group having 2 to 8 carbon atoms, an alkynyl group having 2 to 8 carbon atoms, an alkoxy group having 1 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an alkoxy group having 1 to 8 carbon atoms and substituted with a halogen atom, an acyl group having 2 to 8 carbon atoms, an aryl group having 6 to 10 carbon atoms, a heterocyclic group having a 5- or 6-membered ring, an aralkyl group (the aryl moiety thereof has 6 to 10 carbon atoms, and the alkylene moiety thereof has 1 to 8 carbon atoms), or an alkyl group having 1 to 8 carbon atoms and substituted with a heterocyclic group having a 5- or 6-membered ring,
R² represents hydrogen atom, an alkyl group having 1 to 8 carbon atoms, an alkenyl group having 2 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an acyl group having 2 to 8 carbon atoms, an aryl group having 6 to 10 carbon atoms, or an aralkyl group (the aryl moiety thereof has 6 to 10 carbon atoms, and the alkylene moiety thereof has 1 to 8 carbon atoms),
R3, R⁴, R⁵, and R⁶ may be the same or different, and represent hydrogen atom, an alkyl group having 1 to 8 carbon atoms, or an alkyl group having 1 to 8 carbon atoms and substituted with a halogen atom,
X represents oxygen atom, sulfur atom, or NR⁷, wherein
R⁷ represents hydrogen atom, an alkyl group having 1 to 8 carbon atoms, an alkyl group having 1 to 8 carbon atoms and substituted with a halogen atom, an aralkyl group (the aryl moiety thereof has 6 to 10 carbon atoms, and the alkylene moiety thereof has 1 to 8 carbon atoms), an acyl group having 2 to 8 carbon atoms, or an alkenyl group having 2 to 8 carbon atoms,
Y represents oxygen atom, sulfur atom, NR⁸, or an atomic bond, wherein
R⁸ represents hydrogen atom, an alkyl group having 1 to 8 carbon atoms, an alkyl group having 1 to 8 carbon atoms and substituted with a halogen atom, an acyl group having 2 to 8 carbon atoms, or an alkenyl group having 2 to 8 carbon atoms,
p represents 0 or 1,
A represents oxygen atom, CH₂, N-NH₂, or N-OR⁹, wherein
R⁹ represents hydrogen atom, an alkyl group having 1 to 8 carbon atoms, an alkyl group having 1 to 8 carbon atoms and substituted with a halogen atom, an acyl group having 2 to 8 carbon atoms, an alkenyl group having 2 to 8 carbon atoms, or an aralkyl group (the aryl moiety thereof has 6 to 10 carbon atoms, and the alkylene moiety thereof has 1 to 8 carbon atoms),
B represents, when p is 1, a benzene ring which may have a substituent selected from a halogen atom, hydroxyl group, nitro group, amino group, an alkyl group having 1 to 8 carbon atoms, a cycloalkyl group having a 3- to 7-membered ring, an alkenyl group having 2 to 8 carbon atoms, an alkynyl group having 2 to 8 carbon atoms, an alkoxy group having 1 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an alkoxy group having 1 to 8 carbon atoms and substituted with a halogen atom, an acyl group having 2 to 8 carbon atoms, an aryl group having 6 to 10 carbon atoms, and an aralkyl group (the aryl moiety thereof has 6 to 10 carbon atoms, and the alkylene moiety thereof has 1 to 8 carbon atoms), or
B represents, when p is 0, a condensed ring selected from indole ring, benzofuran ring, 1,2-benzisoxazole ring, and 1,2-benzisothiazole ring which may have a substituent selected from a halogen atom, hydroxyl group, nitro group, amino group, an alkyl group having 1 to 8 carbon atoms, a cycloalkyl group having a 3- to 7-membered ring, an alkenyl group having 2 to 8 carbon atoms, an alkynyl group having 2 to 8 carbon atoms, an alkoxy group having 1 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an alkoxy group having 1 to 8 carbon atoms and substituted with a halogen atom, an acyl group having 2 to 8 carbon atoms, an aryl group having 6 to 10 carbon atoms, and an aralkyl group (the aryl moiety thereof has 6 to 10 carbon atoms, and the alkylene moiety thereof has 1 to 8 carbon atoms),
provided that Y bonds to the benzene ring moiety of B, and -(C(R³)(R⁴))ₘ-bonds to the condensed ring of B at the 3-position,
m represents an integer of 1 to 4, and
n represents an integer of 0 to 5,
provided that when n is 0, Y is an atomic bond.

In one embodiment, the present invention provides a pharmaceutical composition for wound treatment, which comprises a compound represented by the general formula (I), (II), (III-I), or (IV-I), a tautomer, a stereoisomer, or a pharmaceutically acceptable salt of the compound, or a solvate of any of the foregoing, wherein the pharmaceutical composition shortens time required for heating of wound.

In one embodiment, the present invention provides a pharmaceutical composition for wound treatment, which comprises a compound represented by the general formula (I), (II), (III-I), or (IV-I), a tautomer, a stereoisomer, or a pharmaceutically acceptable salt of the compound, or a solvate of any of the foregoing, wherein the pharmaceutical composition not only promotes healing of wound, but also suppresses exacerbation of wound (for example, expansion of wound surface during the inflammation phase and/or proliferation phase).

In one embodiment, the present invention provides a pharmaceutical composition for wound treatment, which comprises a compound represented by the general formula (I), (II), (III-I), or (IV-I), a tautomer, a stereoisomer, or a pharmaceutically acceptable salt of the compound, or a solvate of any of the foregoing, wherein the pharmaceutical composition suppresses exacerbation of wound (for example, expansion of wound surface during the inflammation phase and/or proliferation phase).

In one embodiment, the present invention provides a pharmaceutical composition for wound treatment, which comprises a compound represented by the general formula (I), (II), (III-I), or (IV-I), a tautomer, a stereoisomer, or a pharmaceutically acceptable salt of the compound, or a solvate of any of the foregoing, wherein the pharmaceutical composition promotes wound healing during the inflammation phase and proliferation phase in the wound healing process.

In one embodiment, the present invention provides a pharmaceutical composition for wound treatment, which comprises a compound represented by the general formula (I), (II), (III-I), or (IV-I), a tautomer, a stereoisomer, or a pharmaceutically acceptable salt of the compound, or a solvate of any of the foregoing, wherein the pharmaceutical composition suppresses aggravation of wound surface caused by exudate (for example, abnormal granulation).

Proliferation of granulation tissues is suppressed by excessive exudate, thereby wound surface is aggravated, and wound surface is expanded. In one embodiment, the present invention provides a pharmaceutical composition for wound treatment, which comprises a compound represented by the general formula (I), (II), (III-I), or (IV-I), a tautomer, a stereoisomer, or a pharmaceutically acceptable salt of the compound, or a solvate of any of the foregoing, wherein the pharmaceutical composition suppresses expansion of wound surface caused by excessive exudate. In one embodiment, the present invention provides a pharmaceutical composition for wound treatment, which comprises a compound represented by the general formula (I), (II), (III-I), or (IV-I), a tautomer, a stereoisomer, or a pharmaceutically acceptable salt of the compound, or a solvate of any of the foregoing, wherein the pharmaceutical composition suppresses expansion of wound surface caused by excessive exudate during the inflammation phase and/or proliferation phase.

### Effect of the Invention

According to the present invention, a wound can be treated by administration of a compound that is a PPARδ agonist.

In one embodiment of the present invention, by administration of a compound that is a PPARδ agonist, not only healing of wound can be promoted, but also exacerbation of wound can be suppressed (for example, expansion of wound surface can be suppressed during the inflammation phase and/or proliferation phase).

In one embodiment of the present invention, by administration of a compound that is a PPARδ agonist, exacerbation of wound can be suppressed (for example, expansion of wound surface can be suppressed during the inflammation phase and/or proliferation phase).

In one embodiment of the present invention, by administration of a compound that is a PPARδ agonist, wound healing during the inflammation phase and proliferation phase in the wound healing process can be promoted.

In one embodiment of the present invention, by administration of a compound that is a PPARδ agonist, aggravation of wound surface caused by excessive exudate (for example, abnormal granulation during the inflammation phase and proliferation phase), and/or expansion of wound surface can be suppressed.

### Modes for Carrying out the Invention

Hereafter, the present invention will be explained in detail.

Examples of the compounds usable for the present invention include a compound represented by the following general formula (I): 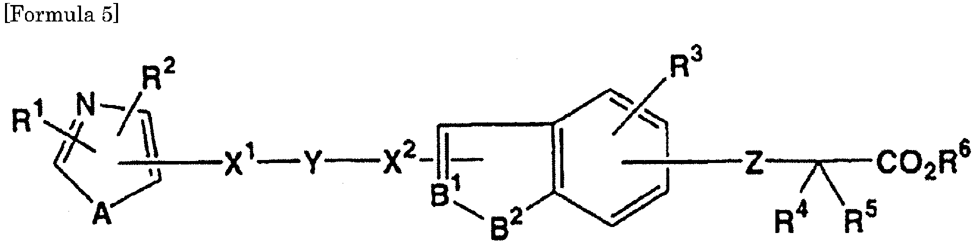
wherein A represents O, S, or NR⁷, wherein
R⁷ represents hydrogen atom, or an alkyl group having 1 to 8 carbon atoms,
B¹ represents CW or N, wherein
W represents hydrogen atom, or an atomic bond,
B² represents O, S, or NR⁸, wherein
R⁸ represents hydrogen atom, or an alkyl group having 1 to 8 carbon atoms,
X¹ and X² represent O, S, NH, NHC(=O), C(=O), C(=N-OR⁹), CH(OR¹⁰), C=C, C≡C, or an atomic bond, wherein
R⁹ and R¹⁰ represent hydrogen atom, or an alkyl group having 1 to 8 carbon atoms,
Y represents an alkylene chain having 1 to 8 carbon atoms which may have an alkyl group having 1 to 8 carbon atoms, or an alkyl group having 1 to 8 carbon atoms and substituted with 1 to 3 halogen atoms as a substituent,
Z represents NH, O, or S,
R¹ represents an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent, or a heterocyclic group having a 5- to 8-membered ring comprising 1 to 3 heteroatoms selected from nitrogen atom, oxygen atom, and sulfur atom, and the remainder carbon atoms as ring-constituting atoms (benzene ring may further condense to this heterocyclic ring),
R² represents an alkyl group having 2 to 8 carbon atoms, an alkyl group having 1 to 8 carbon atoms and substituted with 1 to 3 halogen atoms, a cycloalkyl group having 3 to 7 carbon atoms, an alkenyl group having 2 to 8 carbon atoms, an alkynyl group having 2 to 8 carbon atoms, an alkyl group substituted with an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent (the alkyl moiety thereof has 1 to 4 carbon atoms), or an alkyl group substituted with a 5- to 8-membered heterocyclic ring (the heterocyclic ring thereof comprises 1 to 3 heteroatoms selected from nitrogen atom, oxygen atom, and sulfur atom, and the remainder carbon atoms as ring-constituting atoms, and the alkyl moiety thereof has 1 to 4 carbon atoms,
R³ represents hydrogen atom, a halogen atom, trifluoromethyl group, an alkyl group having 1 to 8 carbon atoms, an alkenyl group having 2 to 8 carbon atoms, or an alkynyl group having 2 to 8 carbon atoms,
R⁴ and R⁵ represent hydrogen atom, an alkyl group having 1 to 8 carbon atoms, or an alkyl group having 1 to 8 carbon atoms and substituted with 1 to 3 halogen atoms, and
R⁶ represents hydrogen atom, an alkyl group having 1 to 8 carbon atoms and substituted with an amino group, an alkyl group having 1 to 8 carbon atoms, or an alkali metal,
provided that Z and R³ bond to the benzene ring, and X² does not bond to the benzene ring);
a compound represented by the following general formula (II):

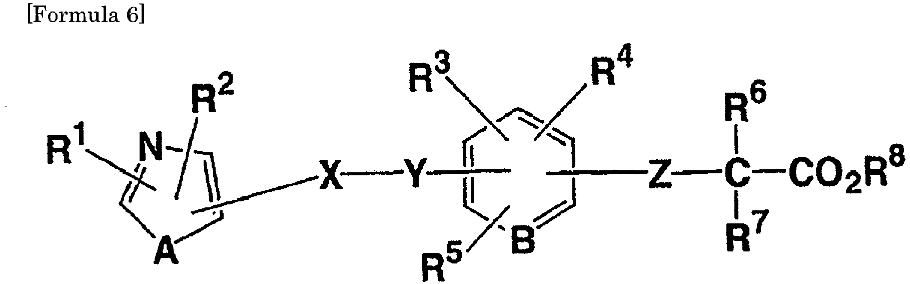

wherein R¹ represents a phenyl group, naphthyl group, pyridyl group, thienyl group, furyl group, quinolyl group, or benzothienyl group which may have a group or an atom selected from an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent, R² represents an alkyl group having 1 to 8 carbon atoms, an alkyl group having 1 to 8 carbon atoms and substituted with a halogen atom, an alkenyl group having 2 to 8 carbon atoms, an alkynyl group having 2 to 8 carbon atoms, a cycloalkyl group having a 3- to 7-membered ring, an alkyl group having 1 to 8 carbon atoms and substituted with a cycloalkyl group having a 3- to 7-membered ring, a phenyl group which may have a group or an atom selected from an alkoxy group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkynyl group having 2 to 8 carbon atoms, a halogen atom, an acyl group having 2 to 7 carbon atoms, a benzoyl group, a hydroxyl group, a nitro group, an amino group, a phenyl group, and a pyridyl group, a naphthyl group, or an alkyl group having 1 to 6 carbon atoms and substituted with a pyridyl group, A represents oxygen atom, sulfur atom, or NR⁹, wherein R⁹ represents hydrogen atom, or an alkyl group having 1 to 8 carbon atoms, X represents an alkylene chain having 1 to 8 carbon atoms which may have a group selected from an alkyl group having 1 to 8 carbon atoms, an alkoxy group having 1 to 8 carbon atoms, and hydroxyl group as a substituent, and may contain a double bond, Y represents C(=O), C(=NOR¹⁰), CH(OR¹¹), CH=CH, C≡C, or C(=CH₂), wherein R¹⁰ and R¹¹ represent hydrogen atom, an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, or pyridyl group, B represents CH or nitrogen atom, Z represents oxygen atom or sulfur atom, R⁶ and R⁷ represents hydrogen atom, an alkyl group having 1 to 8 carbon atoms, or an alkyl group having 1 to 8 carbon atoms and substituted with a halogen atom, and R⁸ represents hydrogen atom, or an alkyl group having 1 to 8 carbon atoms,
provided that at least one of R³, R⁴, or R⁵ is not hydrogen atom;
a compound represented by the following general formula (III-I):

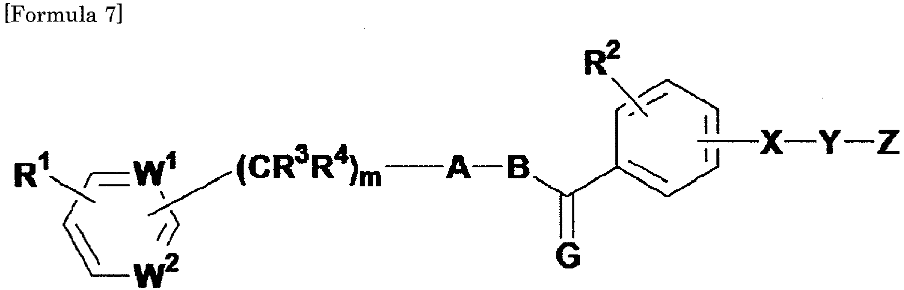

wherein W¹ and W² may be the same or different, and represent CH or nitrogen atom,
X represents NR⁵ or CR⁶R⁷, wherein R⁵ represents hydrogen atom, an alkyl group having 1 to 8 carbon atoms, an alkyl group having 1 to 8 carbon atoms and substituted with a halogen atom, an alkyl group having 1 to 8 carbon atoms and substituted with an alkoxy group having 1 to 8 carbon atoms, a cycloalkyl group having a 3- to 7-membered ring, an alkyl group having 1 to 8 carbon atoms and substituted with a cycloalkyl group having a 3- to 7-membered ring, an alkyl group having 1 to 8 carbon atoms and substituted with phenyl group, an acyl group having 2 to 8 carbon atoms, or an alkenyl group having 2 to 8 carbon atoms, and
R⁶ and R⁷ may be the same or different, and represent hydrogen atom, or an alkyl group having 1 to 8 carbon atoms,
Y represents -(CR⁸R⁹)ₙ-, wherein R⁸ and R⁹ may be the same or different, and represent hydrogen atom, or an alkyl group having 1 to 8 carbon atoms, and n represents an integer of 1 to 4, or
X and Y may bind together to represent -CR¹⁰=CR¹¹- or ethynylene, wherein R¹⁰ and R¹¹ may be the same or different, and represent hydrogen atom, or an alkyl group having 1 to 8 carbon atoms,
Z represents carboxyl group, or tetrazolyl group,
G represents O, S, or CR¹²R¹³, wherein R¹² and R¹³ may be the same or different, and represent hydrogen atom, or an alkyl group having 1 to 8 carbon atoms,
A represents a 5-membered heterocyclic ring selected from thiazole, oxazole, imidazole, pyrazole, thiophene, furan, and pyrrole which may have a substituent selected from an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms and substituted with a halogen atom, an alkoxy group having 1 to 8 carbon atoms and substituted with a halogen atom, hydroxyl group, nitro group, an acyl group having 2 to 8 carbon atoms, an aryl group having 6 to 10 carbon atoms, and a 5- or 6-membered heterocyclic group,
B represents an alkylene chain having 1 to 8 carbon atoms which may have a substituent selected from an alkyl group having 1 to 8 carbon atoms, a cycloalkyl group having a 3- to 7-membered ring, an alkoxy group having 1 to 8 carbon atoms, and a halogen atom, wherein if the alkylene chain contains 2 or more carbon atoms, it may contain a double bond or triple bond,
R¹ and R² may be the same or different, and represent hydrogen atom, an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms and substituted with a halogen atom, an alkoxy group having 1 to 8 carbon atoms and substituted with a halogen atom, hydroxyl group, nitro group, an acyl group having 2 to 8 carbon atoms, an aryl group having 6 to 10 carbon atoms, or a heterocyclic group having a 5- or 6-membered ring,
R³ and R⁴ may be the same or different, and represent hydrogen atom, or an alkyl group having 1 to 8 carbon atoms, and
m represents an integer of 0 to 3; and
a compound represented by the following general formula (IV-I):

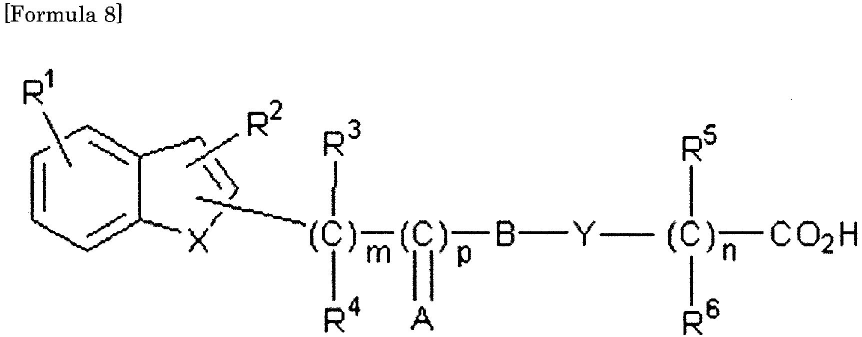

wherein R¹ represents hydrogen atom, a halogen atom, hydroxyl group, nitro group, amino group, cyano group, carboxyl group, an alkyl group having 1 to 8 carbon atoms, a cycloalkyl group having a 3- to 7-membered ring, an alkenyl group having 2 to 8 carbon atoms, an alkynyl group having 2 to 8 carbon atoms, an alkoxy group having 1 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an alkoxy group having 1 to 8 carbon atoms and substituted with a halogen atom, an acyl group having 2 to 8 carbon atoms, an aryl group having 6 to 10 carbon atoms, a heterocyclic group having a 5- or 6-membered ring, an aralkyl group (the aryl moiety thereof has 6 to 10 carbon atoms, and the alkylene moiety thereof has 1 to 8 carbon atoms), or an alkyl group having 1 to 8 carbon atoms and substituted with a heterocyclic group having a 5- or 6-membered ring,
R² represents hydrogen atom, an alkyl group having 1 to 8 carbon atoms, an alkenyl group having 2 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an acyl group having 2 to 8 carbon atoms, an aryl group having 6 to 10 carbon atoms, or an aralkyl group (the aryl moiety thereof has 6 to 10 carbon atoms, and the alkylene moiety thereof has 1 to 8 carbon atoms),
R³, R⁴, R⁵, and R⁶ may be the same or different, and represent hydrogen atom, an alkyl group having 1 to 8 carbon atoms, or an alkyl group having 1 to 8 carbon atoms and substituted with a halogen atom,
X represents oxygen atom, sulfur atom, or NR⁷, wherein
R⁷ represents hydrogen atom, an alkyl group having 1 to 8 carbon atoms, an alkyl group having 1 to 8 carbon atoms and substituted with a halogen atom, an aralkyl group (the aryl moiety thereof has 6 to 10 carbon atoms, and the alkylene moiety thereof has 1 to 8 carbon atoms), an acyl group having 2 to 8 carbon atoms, or an alkenyl group having 2 to 8 carbon atoms,
Y represents oxygen atom, sulfur atom, NR⁸, or an atomic bond, wherein
R⁸ represents hydrogen atom, an alkyl group having 1 to 8 carbon atoms, an alkyl group having 1 to 8 carbon atoms and substituted with a halogen atom, an acyl group having 2 to 8 carbon atoms, or an alkenyl group having 2 to 8 carbon atoms,
p represents 0 or 1,
A represents oxygen atom, CH₂, N-NH₂, or N-OR⁹, wherein
R⁹ represents hydrogen atom, an alkyl group having 1 to 8 carbon atoms, an alkyl group having 1 to 8 carbon atoms and substituted with a halogen atom, an acyl group having 2 to 8 carbon atoms, an alkenyl group having 2 to 8 carbon atoms, or an aralkyl group (the aryl moiety thereof has 6 to 10 carbon atoms, and the alkylene moiety thereof has 1 to 8 carbon atoms),
B represents, when p is 1, a benzene ring which may have a substituent selected from a halogen atom, hydroxyl group, nitro group, amino group, an alkyl group having 1 to 8 carbon atoms, a cycloalkyl group having a 3- to 7-membered ring, an alkenyl group having 2 to 8 carbon atoms, an alkynyl group having 2 to 8 carbon atoms, an alkoxy group having 1 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an alkoxy group having 1 to 8 carbon atoms and substituted with a halogen atom, an acyl group having 2 to 8 carbon atoms, an aryl group having 6 to 10 carbon atoms, and an aralkyl group (the aryl moiety thereof has 6 to 10 carbon atoms, and the alkylene moiety thereof has 1 to 8 carbon atoms), or
B represents, when p is 0, a condensed ring selected from indole ring, benzofuran ring, 1,2-benzisoxazole ring, and 1,2-benzisothiazole ring which may have a substituent selected from a halogen atom, hydroxyl group, nitro group, amino group, an alkyl group having 1 to 8 carbon atoms, a cycloalkyl group having a 3- to 7-membered ring, an alkenyl group having 2 to 8 carbon atoms, an alkynyl group having 2 to 8 carbon atoms, an alkoxy group having 1 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an alkoxy group having 1 to 8 carbon atoms and substituted with a halogen atom, an acyl group having 2 to 8 carbon atoms, an aryl group having 6 to 10 carbon atoms, and an aralkyl group (the aryl moiety thereof has 6 to 10 carbon atoms, and the alkylene moiety thereof has 1 to 8 carbon atoms),
provided that Y bonds to the benzene ring moiety of B, and -(C(R³)(R⁴))ₘ-bonds to the condensed ring of B at the 3-position.
m represents an integer of 1 to 4, and
n represents an integer of 0 to 5,
provided that when n is 0, Y represents an atomic bond.
A tautomer, a stereoisomer, or a pharmaceutically acceptable salt of the compounds represented by the general formula (I), (II), (III-I), or (IV-I), or a solvate of any of the foregoing can also be used for the present invention.

Examples of the pharmaceutically acceptable salt include acid addition salts formed with an inorganic acid such as hydrochloric acid, hydrobromic acid, sulfuric acid, nitric acid, and phosphoric acid; or an organic acid such as formic acid, acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydrxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis(3-hydroxy-2-en-1-carboxylic acid), 3-phenylpropionic acid, trimethylacetic acid, tert-butylacetic acid, laurylsulfuric acid, gluconic acid, glutamic acid, hydroxynaphthoic acid, salicylic acid, stearic acid, and muconic acid; salts formed by replacement of an acidic proton of a base compound with a metal ion (for example, alkali metal ion (for example, sodium ion and potassium ion), an alkaline earth metal ion (for example, calcium ion), and aluminum ion) when a parent compound has an acidic portion; and salts formed with an organic base such as ethanolamine, diethanolamine, triethanolamine, tromethamine, and N-methylglucamine.

Examples of the stereoisomer include cis- and trans-isomers, racemates, and optically active substances.

### Examples of the solvate include hydrate.

As for the general formula (I), examples of the alkyl group having 1 to 8 carbon atoms as R³, R⁴, R⁵, R⁶, R⁷, R⁸, R⁹, and R¹⁰, as well as the substituent which the alkylene chain as Y may have, the substituent which the aryl group, and heterocyclic group as R¹ may have, and the substituent which the alkyl group substituted with an aryl group, and the alkyl group substituted with a heterocyclic ring as R² may have include methyl group, ethyl group, propyl group, isopropyl group, butyl group, i-butyl group, t-butyl group, pentyl group, and hexyl group.

As for the general formula (I), examples of the alkyl group having 2 to 8 carbon atoms as R² include ethyl group, propyl group, isopropyl group, butyl group, i-butyl group, t-butyl group, pentyl group, and hexyl group.

As for the general formula (I), examples of the alkyl group having 1 to 8 carbon atoms and substituted with 1 to 3 halogen atoms as R², R⁴, and R⁵ as well as the substituent which the alkylene chain as Y may have, the substituent which the aryl group and heterocyclic group as R¹ may have, and the substituent which the alkyl group substituted with an aryl group, and alkyl group substituted with a heterocyclic ring as R² may have include methyl group, ethyl group, propyl group, isopropyl group, butyl group, and t-butyl group substituted with 1 to 3 halogen atoms such as fluorine atom, chlorine atom, and bromine atom, and preferred examples include trifluoromethyl group, chloromethyl group, 2-chloroethyl group, 2-bromoethyl group, and 2-fluoroethyl group.

As for the general formula (I), examples of the alkenyl group having 2 to 8 carbon atoms as R² and R³ include vinyl group, and allyl group.

As for the general formula (I), examples of the alkynyl group having 2 to 8 carbon atoms as R² and R³ include propargyl group.

As for the general formula (I), examples of the halogen atom as R³ include fluorine atom, chlorine atom, and bromine atom.

As for the general formula (I), examples of the cycloalkyl group having 3 to 7 carbon atoms as R² include cyclopropyl group, cyclopentyl group, and cyclohexyl group.

As for the general formula (I), examples of the alkoxy group having 1 to 8 carbon atoms as the substituent which the aryl group and heterocyclic group as R¹ may have, and the substituent which the alkyl group substituted with an aryl group, and alkyl group substituted with a heterocyclic ring as R² may have include methoxy group, ethoxy group, propoxy group, isopropoxy group, butoxy group, i-butoxy group, t-butoxy group, pentyloxy group, and hexyloxy group.

As for the general formula (I), examples of the aryl moiety of the aryl group as R¹, and the alkyl group substituted with an aryl group as R² include phenyl group, and naphthyl group.

As for the general formula (I), examples of the heterocyclic ring moiety of the heterocyclic ring in the heterocyclic group having a 5- to 8-membered ring as R¹, and the alkyl group substituted with a 5- to 8-membered alkyl group as R² include pyridyl group, thienyl group, furyl group, thiazolyl group, and quinolyl group.

As for the general formula (I), examples of the heterocyclic group consisting of condensed heterocyclic group having a 5- to 8-membered ring comprising 1 to 3 heteroatoms selected from nitrogen atom, oxygen atom, and sulfur atom, and the remainder carbon atoms as ring-constituting atoms and benzene ring as R¹ include quinoline ring, and benzothienyl ring.

As for the general formula (I), examples of the alkylene chain having 1 to 8 carbon atoms as Y include methylene, and ethylene.

As for the general formula (I), R³ may consist of 1 to 3 of the same or different groups or atoms.

As for the general formula (I), examples of the alkyl group having 1 to 8 carbon atoms and substituted with an amino group as R⁶ include methyl group, ethyl group, propyl group, isopropyl group, butyl group, i-butyl group, t-butyl group, pentyl group, and hexyl group substituted with an amino group such as piperidino group, pyrrolidino group, dimethylamino group, and diethylamino group.

As for the general formula (II), examples of the alkyl group having 1 to 8 carbon atoms include methyl group, ethyl group, propyl group, isopropyl group, butyl group, i-butyl group, t-butyl group, and pentyl group.

As for the general formula (II), examples of the alkyl group having 1 to 8 carbon atoms and substituted with a halogen atom include methyl group, ethyl group, propyl group, isopropyl group, butyl group, and t-butyl group substituted with 1 to 3 halogen atoms such as fluorine atom, chlorine atom, and bromine atom, and preferred examples include trifluoromethyl group, chloromethyl group, 2-chloroethyl group, 2-bromoethyl group, 2-fluoroethyl group, and the like.

As for the general formula (II), examples of the alkoxy group having 1 to 8 carbon atoms include methoxy group, ethoxy group, propoxy group, isopropoxy group, butoxy group, i-butoxy group, t-butoxy group, and pentyloxy group.

As for the general formula (II), examples of the alkoxy group having 1 to 8 carbon atoms and substituted with a halogen atom include methoxy group, ethoxy group, propoxy group, isopropoxy group, butoxy group, and t-butoxy group substituted with 1 to 3 halogen atoms such as fluorine atom, chlorine atom, and bromine atom, and preferred examples include trifluoromethoxy group, chloromethoxy group, 2-chloroethoxy group, 2-bromoethoxy group, 2-fluoroethoxy group, and the like.

As for the general formula (II), examples of the alkenyl group having 2 to 8 carbon atoms include vinyl group, and allyl group.

As for the general formula (II), examples of the alkynyl group having 2 to 8 carbon atoms include propargyl group.

As for the general formula (II), examples of the cycloalkyl group having a 3- to 7-membered ring include cyclohexyl group, cyclopentyl group, and the like.

As for the general formula (II), examples of the halogen atom include fluorine atom, chlorine atom, bromine atom, and the like.

As for the general formula (II), examples of the acyl group having 2 to 7 carbon atoms include acetyl group, propionyl group, and the like.

As for the general formula (II), examples of the alkyl group having 1 to 8 carbon atoms and substituted with a cycloalkyl group having a 3- to 7-membered ring include cyclohexylmethyl group, cyclopentylmethyl group, and the like.

As for the general formula (III-I), examples of the alkyl group having 1 to 8 carbon atoms as R¹, R², R³, R⁴, R⁵, R⁶, R⁷, R⁸, R⁹, R¹⁰, R¹¹, R¹², R¹³, the substituent which the 5-membered heterocyclic ring asA may have, and the substituent which the alkylene chain having 1 to 8 carbon atoms as B may have include methyl group, ethyl group, propyl group, isopropyl group, butyl group, i-butyl group, t-butyl group, pentyl group, hexyl group, and the like.

As for the general formula (III-I), examples of the alkenyl group having 2 to 8 carbon atoms as R¹, R², R⁵, and the substituent which the 5-membered heterocyclic ring as A may have include vinyl group, allyl group, and the like.

As for the general formula (III-I), examples of the alkynyl group having 2 to 8 carbon atoms as R¹, R², and the substituent which the 5-membered heterocyclic ring as A may have include propargyl group, and the like.

As for the general formula (III-I), examples of the alkoxy group having 1 to 8 carbon atoms as R¹, R², the substituent which the 5-membered heterocyclic ring as A may have, and the substituent which the alkylene chain having 1 to 8 carbon atoms as B may have include methoxy group, ethoxy group, propoxy group, isopropoxy group, butoxy group, i-butoxy group, t-butoxy group, pentyloxy group, hexyloxy group, and the like.

As for the general formula (III-I), examples of the halogen atom as R¹, R², the substituent which the 5-membered heterocyclic ring as A may have, and the substituent which the alkylene chain having 1 to 8 carbon atoms as B may have include fluorine atom, chlorine atom, bromine atom, and the like.

As for the general formula (III-I), examples of the alkyl group having 1 to 8 carbon atoms and substituted with a halogen atom as R¹, R², R⁵, and the substituent which the 5-membered heterocyclic ring as A may have include methyl group, ethyl group, propyl group, isopropyl group, butyl group, t-butyl group substituted with 1 to 3 halogen atoms such as fluorine atom, chlorine atom, and bromine atom, and the like, and preferred examples include trifluoromethyl group, chloromethyl group, 2-chloroethyl group, 2-bromoethyl group, 2-fluoroethyl group, and the like.

As for the general formula (III-I), examples of the alkoxy group having 1 to 8 carbon atoms and substituted with a halogen atom as R¹, R², and the substituent which the 5-membered heterocyclic ring as A may have include methoxy group, ethoxy group, propoxy group, isopropyloxy group, butyloxy group, t-butyloxy group substituted with 1 to 3 halogen atoms such as fluorine atom, chlorine atom, and bromine atom, and the like, and preferred examples include trifluoromethyloxy group, chloromethyloxy group, 2-chloroethyloxy group, 2-bromoethyloxy group, 2-fluoroethyloxy group, and the like.

As for the general formula (III-I), examples of the acyl group having 2 to 8 carbon atoms as R¹, R², R⁵, and the substituent which the 5-membered heterocyclic ring as A may have include acetyl group, propionyl group, and the like.

As for the general formula (III-I), examples of the aryl group having 6 to 10 carbon atoms as R¹, R², and the substituent which 5-membered heterocyclic ring as A may have include phenyl group, and the like.

As for the general formula (III-I), examples of the heterocyclic group having a 5- or 6-membered ring as R¹, R², and the substituent which the 5-membered heterocyclic ring as A may have include pyridyl group, and the like.

As for the general formula (III-I), examples of the alkyl group having 1 to 8 carbon atoms and substituted with an alkoxy group having 1 to 8 carbon atoms as R⁵ include methyl group, ethyl group, propyl group, isopropyl group, butyl group, i-butyl group, t-butyl group, pentyl group, hexyl group substituted with methoxy group, ethoxy group, propoxy group, isopropoxy group, butoxy group, i-butoxy group, t-butoxy group, pentyloxy group, hexyloxy group, or the like, and the like.

As for the general formula (III-I), examples of the cycloalkyl group having a 3-to 7-membered ring as R⁵ include cyclopropyl group, cyclobutyl group, cyclopentyl group, cyclohexyl group, and the like.

As for the general formula (III-I), examples of the alkyl group having 1 to 8 carbon atoms and substituted with a cycloalkyl group having a 3- to 7-membered ring as R⁵ include methyl group, ethyl group, propyl group, isopropyl group, butyl group, i-butyl group, t-butyl group, pentyl group, hexyl group substituted with cyclopropyl group, cyclobutyl group, cyclopentyl group, cyclohexyl group, or the like, and the like.

As for the general formula (III-I), examples of the alkyl group having 1 to 8 carbon atoms substituted with phenyl group as R⁵ include benzyl group, phenethyl group, and the like.

As for the general formula (III-I), examples of the cycloalkyl group having a 3-to 7-membered ring as the substituent which the alkylene chain having 1 to 8 carbon atoms as B may have include cyclopropyl group, cyclobutyl group, cyclopentyl group, cyclohexyl group, and the like.

The compound represented by the general formula (III-I) may be a compound represented by the general formula (III-II): 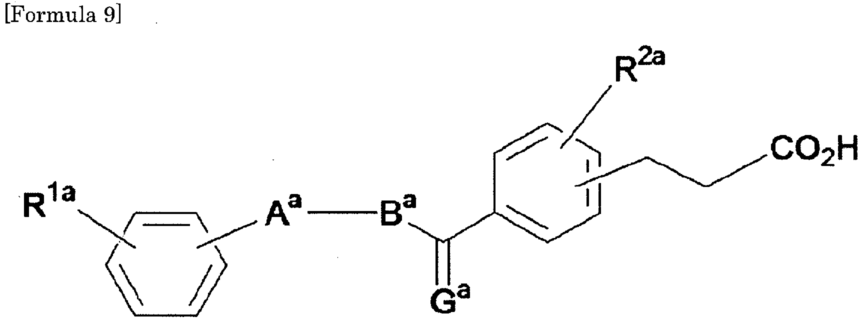
wherein G^{a} represents O, S, or CH₂, A^{a} represents a 5-membered heterocyclic ring selected from thiazole, oxazole, and thiophene which may have a substituent selected from an alkyl group having 1 to 8 carbon atoms, an alkoxy group having 1 to 8 carbon atoms, a halogen atom, an alkyl group having 1 to 8 carbon atoms and substituted with a halogen atom, an alkoxy group having 1 to 8 carbon atoms and substituted with a halogen atom, hydroxyl group, nitro group, and an acyl group having 2 to 8 carbon atoms,
B^{a} represents an alkylene chain having 1 to 8 carbon atoms, which may have a double bond when it is an alkylene chain having 2 or more carbon atoms, and R^{1a} and R^{2a} may be the same or different, and represent hydrogen atom, an alkyl group having 1 to 8 carbon atoms, an alkoxy group having 1 to 8 carbon atoms, a halogen atom, an alkyl group having 1 to 8 carbon atoms and substituted with a halogen atom, an alkoxy group having 1 to 8 carbon atoms and substituted with a halogen atom, hydroxyl group, nitro group, or an acyl group having 2 to 8 carbon atoms; or
a compound represented by the general formula (III-III): 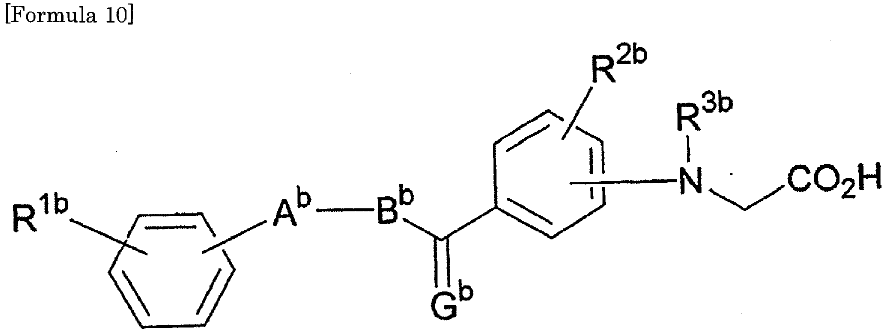
wherein G^{b} represents O, S, or CH₂, A^{b} represents a 5-membered heterocyclic ring selected from thiazole, oxazole, and thiophene which may have a substituent selected from an alkyl group having 1 to 8 carbon atoms, an alkoxy group having 1 to 8 carbon atoms, a halogen atom, an alkyl group having 1 to 8 carbon atoms and substituted with a halogen atom, an alkoxy group having 1 to 8 carbon atoms and substituted with a halogen atom, hydroxyl group, nitro group, and an acyl group having 2 to 8 carbon atoms,
B^{b} represents an alkylene chain having 1 to 8 carbon atoms, wherein if the alkylene chain contains 2 or more carbon atoms, it may contain a double bond, R^{1b} and R^{2b} may be the same or different, and represent hydrogen atom, an alkyl group having 1 to 8 carbon atoms, an alkoxy group having 1 to 8 carbon atoms, a halogen atom, an alkyl group having 1 to 8 carbon atoms and substituted with a halogen atom, an alkoxy group having 1 to 8 carbon atoms and substituted with a halogen atom, hydroxyl group, nitro group, or an acyl group having 2 to 8 carbon atoms, and R^{3b} represents hydrogen atom, or an alkyl group having 1 to 8 carbon atoms.

As for the general formula (III-II), examples of the alkyl group having 1 to 8 carbon atoms, alkoxy group having 1 to 8 carbon atoms, halogen atom, alkyl group having 1 to 8 carbon atoms and substituted with a halogen atom, alkoxy group having 1 to 8 carbon atoms and substituted with a halogen atom, and acyl group having 2 to 8 carbon atoms as R^{1a}, R^{2a}, and the substituent which the 5-membered heterocyclic ring as A^{a} may have include those exemplified above for R¹, R², and the substituent which the 5-membered heterocyclic ring as A may have as for the general formula (III-I).

As for the general formula (III-III), examples of the alkyl group having 1 to 8 carbon atoms, alkoxy group having 1 to 8 carbon atoms, halogen atom, alkyl group having 1 to 8 carbon atoms and substituted with a halogen atom, alkoxy group having 1 to 8 carbon atoms and substituted with a halogen atom, and acyl group having 2 to 8 carbon atoms as R^{1b}, R^{2b}, and the substituent which the 5-membered heterocyclic ring as A^{b} may have include those exemplified above for R¹, R², and the substituent which the 5-membered heterocyclic ring as A may have as for the general formula (III-I).

As for the general formula (III-III), examples of the alkyl group having 1 to 8 carbon atoms as R^{3b} include those exemplified above for R⁵ as for the general formula (III-I).

R¹ and R² included in the general formula (III-I), R^{1a} and R^{2a} included in the general formula (III-II), and R^{1b} and R^{2b} included in the general formula (III-II) may each consist of 1 to 3 of the same or different groups or atoms on the benzene ring on which R¹ or the like substitutes.

As for the general formula (IV-I), examples of the alkyl group having 1 to 8 carbon atoms as R¹, R², R³, R⁴, R⁵, R⁶, R⁷, R⁸, and R⁹ as well as the substituent which the phenyl group and condensed ring as B may have include methyl group, ethyl group, propyl group, isopropyl group, butyl group, i-butyl group, t-butyl group, pentyl group, hexyl group, and the like.

As for the general formula (IV-I), examples of the alkenyl group having 2 to 8 carbon atoms as R¹, R², R⁷, R⁸, and R⁹ as well as the substituent which the phenyl group and condensed ring as B may have include vinyl group, allyl group, and the like.

As for the general formula (IV-I), examples of the alkynyl group having 2 to 8 carbon atoms as R¹ and the substituent which the phenyl group and condensed ring as B may have include propargyl group, and the like.

As for the general formula (IV-I), examples of the cycloalkyl group having a 3-to 7-membered ring as R¹ and the substituent which the phenyl group and condensed ring as B may have include cyclopropyl group, cyclopentyl group, cyclohexyl group, and the like.

As for the general formula (IV-I), examples of the alkoxy group having 1 to 8 carbon atoms as R¹ and the substituent which the phenyl group and condensed ring as B may have include methoxy group, ethoxy group, propoxy group, isopropoxy group, butoxy group, i-butoxy group, t-butoxy group, pentyloxy group, hexyloxy group, and the like.

As for the general formula (IV-I), examples of the halogen atom as R¹ and the substituent which the phenyl group and condensed ring as B may have include fluorine atom, chlorine atom, bromine atom, and the like.

As for the general formula (IV-I), examples of the alkyl group having 1 to 8 carbon atoms and substituted with a halogen atom as R¹, R², R³, R⁴, R⁵, R⁶, R⁷, R⁸, and R⁹ as well as the substituent which the phenyl group and condensed ring as B may have include methyl group, ethyl group, propyl group, isopropyl group, butyl group, t-butyl group substituted with 1 to 3 halogen atoms such as fluorine atom, chlorine atom, and bromine atom, and the like, and preferred examples include trifluoromethyl group, chloromethyl group, 2-chloroethyl group, 2-bromoethyl group, 2-fluoroethyl group, and the like. Examples of the alkyl group having 1 to 8 carbon atoms and substituted with an alkoxy group having 1 to 8 carbon atoms as R¹ and R² as well as the substituent which the phenyl group and condensed ring as B may have include methyl group, ethyl group, propyl group, isopropyl group, butyl group, i-butyl group, t-butyl group, pentyl group, hexyl group substituted with methoxy group, ethoxy group, propoxy group, isopropoxy group, butoxy group, i-butoxy group, t-butoxy group, pentyloxy group, hexyloxy group, or the like, and the like, and preferred examples include ethoxyethyl group, and the like.

As for the general formula (IV-I), examples of the alkoxy group having 1 to 8 carbon atoms and substituted with a halogen atom as R¹ and the substituent which the phenyl group and condensed ring as B may have include methoxy group, ethoxy group, propoxy group, isopropyloxy group, butyloxy group, t-butyloxy group substituted with 1 to 3 halogen atoms such as fluorine atom, chlorine atom, and bromine atom, and the like, and preferred examples include trifluoromethyloxy group, chloromethyloxy group, 2-chloroethyloxy group, 2-bromoethyloxy group, 2-fluoroethyloxy group, and the like.

As for the general formula (IV-I), examples of the aryl group having 6 to 10 carbon atoms as R¹ and R² as well as the substituent which the phenyl group and condensed ring as B may have include phenyl group, and the like.

As for the general formula (IV-I), examples of the acyl group having 2 to 8 carbon atoms as R¹, R², R⁷, R⁸, and R⁹ as well as the substituent which the phenyl group and condensed ring as B may have include acetyl group.

As for the general formula (IV-I), examples of the heterocyclic group having a 5- or 6-membered ring as R¹ include pyridyl group, and the like.

As for the general formula (IV-I), examples of the alkyl group having 1 to 8 carbon atoms and substituted with a cycloalkyl group having a 3- to 7-membered ring as R¹ and R² as well as the substituent which the phenyl group and condensed ring as B may have include methyl group, ethyl group, propyl group, isopropyl group, butyl group, i-butyl group, t-butyl group, pentyl group, hexyl group substituted with cyclopropyl group, cyclopentyl group, cyclohexyl group, or the like, and the like.

As for the general formula (IV-I), examples of the aralkyl group (the aryl moiety thereof has 6 to 10 carbon atoms, and the alkylene moiety thereof has 1 to 8 carbon atoms) as R¹, R², R⁷, R⁹, and the substituent which the phenyl group and condensed ring as B may have include benzyl group, phenethyl group, and the like.

Examples of the alkyl group having 1 to 8 carbon atoms and substituted with a heterocyclic group having a 5- or 6-membered ring as R¹ include methyl group, ethyl group, propyl group, isopropyl group, butyl group, i-butyl group, t-butyl group, pentyl group, hexyl group substituted with pyridyl group, or the like, and the like.

R¹ (except for hydrogen atom) and the substituent which the phenyl group and condensed ring as B may have may consist of 1 to 3 of the same or different atoms or groups.

As for the general formula (IV-I), R¹ may be a dialkylamino group having 2 to 12 carbon atoms such as dimethylamino group, and diethylamino group, and R² may be a cycloalkyl group having a 3- to 7-membered ring such as cyclopropyl group, cyclopentyl group, and cyclohexyl group.

As for the general formula (IV-I), R¹ is preferably a group or an atom other than hydrogen atom, and R² is preferably an alkyl group having 2 to 6 carbon atoms.

The compounds represented by the general formula (IV-I) may be a compound represented by the general formula (IV-II): 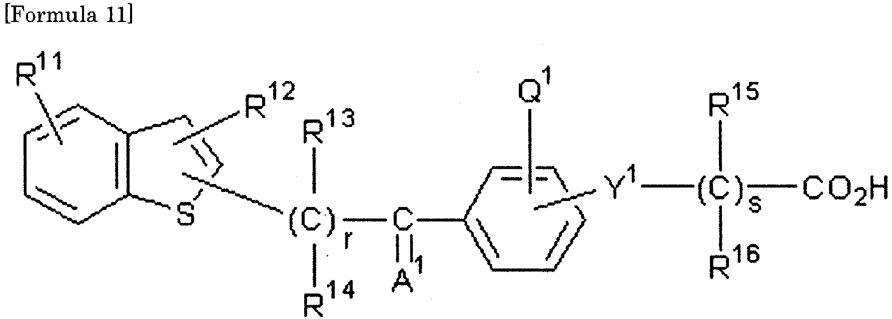
wherein R¹¹ represents hydrogen atom, a halogen atom, hydroxyl group, nitro group, amino group, cyano group, carboxyl group, an alkyl group having 1 to 8 carbon atoms, a cycloalkyl group having a 3- to 7-membered ring, an alkenyl group having 2 to 8 carbon atoms, an alkynyl group having 2 to 8 carbon atoms, an alkoxy group having 1 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an alkoxy group having 1 to 8 carbon atoms and substituted with a halogen atom, an acyl group having 2 to 8 carbon atoms, an aryl group having 6 to 10 carbon atoms, a heterocyclic group having a 5- or 6-membered ring, an aralkyl group (the aryl moiety thereof has 6 to 10 carbon atoms, and the alkylene moiety thereof has 1 to 8 carbon atoms), or an alkyl group having 1 to 8 carbon atoms and substituted with a heterocyclic group having a 5- or 6-membered ring,
R¹² represents hydrogen atom, an alkyl group having 1 to 8 carbon atoms, an alkenyl group having 2 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an acyl group having 2 to 8 carbon atoms, an aryl group having 6 to 10 carbon atoms, or an aralkyl group (the aryl moiety thereof has 6 to 10 carbon atoms, and the alkylene moiety thereof has 1 to 8 carbon atoms),
R¹³, R¹⁴, R¹⁵, and R¹⁶ may be the same or different, and represent hydrogen atom, an alkyl group having 1 to 8 carbon atoms, or an alkyl group having 1 to 8 carbon atoms and substituted with a halogen atom,
Y¹ represents oxygen atom, sulfur atom, NR¹⁸, or an atomic bond, wherein
R¹⁸ represents hydrogen atom, an alkyl group having 1 to 8 carbon atoms, an alkyl group having 1 to 8 carbon atoms and substituted with a halogen atom, an acyl group having 2 to 8 carbon atoms, or an alkenyl group having 2 to 8 carbon atoms,
A¹ represents oxygen atom, CH₂, N-NH₂, or N-OR¹⁹, wherein
R¹⁹ represents hydrogen atom, an alkyl group having 1 to 8 carbon atoms, an alkyl group having 1 to 8 carbon atoms and substituted with a halogen atom, an acyl group having 2 to 8 carbon atoms, an alkenyl group having 2 to 8 carbon atoms, or an aralkyl group (the aryl moiety thereof has 6 to 10 carbon atoms, and the alkylene moiety thereof has 1 to 8 carbon atoms),
Q¹ represents hydrogen atom, a halogen atom, hydroxyl group, nitro group, amino group, an alkyl group having 1 to 8 carbon atoms, a cycloalkyl group having a 3-to 7-membered ring, an alkenyl group having 2 to 8 carbon atoms, an alkynyl group having 2 to 8 carbon atoms, an alkoxy group having 1 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an alkoxy group having 1 to 8 carbon atoms and substituted with a halogen atom, an acyl group having 2 to 8 carbon atoms, an aryl group having 6 to 10 carbon atoms, or an aralkyl group (the aryl moiety thereof has 6 to 10 carbon atoms, and the alkylene moiety thereof has 1 to 8 carbon atoms),
r represents an integer of 1 to 4, and
s represents an integer of 1 to 5; or
a compound represented by the general formula (IV-III):

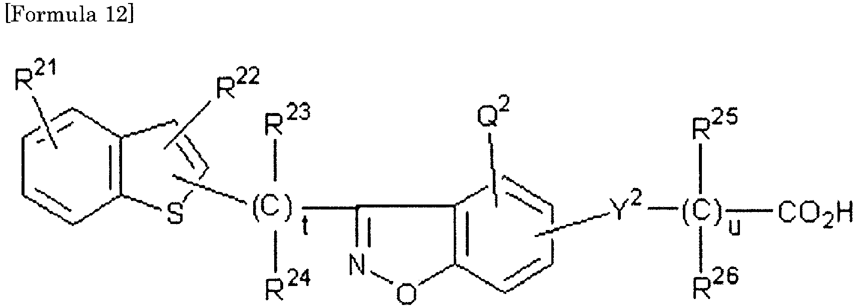

wherein R²¹ represents hydrogen atom, a halogen atom, hydroxyl group, nitro group, amino group, cyano group, carboxyl group, an alkyl group having 1 to 8 carbon atoms, a cycloalkyl group having a 3- to 7-membered ring, an alkenyl group having 2 to 8 carbon atoms, an alkynyl group having 2 to 8 carbon atoms, an alkoxy group having 1 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an alkoxy group having 1 to 8 carbon atoms and substituted with a halogen atom, an acyl group having 2 to 8 carbon atoms, an aryl group having 6 to 10 carbon atoms, a heterocyclic group having a 5- or 6-membered ring, an aralkyl group (the aryl moiety thereof has 6 to 10 carbon atoms, and the alkylene moiety thereof has 1 to 8 carbon atoms), or an alkyl group having 1 to 8 carbon atoms and substituted with a heterocyclic group having a 5- or 6-membered ring,
R²² represents hydrogen atom, an alkyl group having 1 to 8 carbon atoms, an alkenyl group having 2 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an acyl group having 2 to 8 carbon atoms, an aryl group having 6 to 10 carbon atoms, or an aralkyl group (the aryl moiety thereof has 6 to 10 carbon atoms, and the alkylene moiety thereof has 1 to 8 carbon atoms),
R²³, R²⁴, R²⁵ and R²⁶ may be the same or different, and represent hydrogen atom, an alkyl group having 1 to 8 carbon atoms, or an alkyl group having 1 to 8 carbon atoms and substituted with a halogen atom,
Y² represents oxygen atom, sulfur atom, NR²⁸, or an atomic bond, wherein
R²⁸ represents hydrogen atom, an alkyl group having 1 to 8 carbon atoms, an alkyl group having 1 to 8 carbon atoms and substituted with a halogen atom, an acyl group having 2 to 8 carbon atoms, or an alkenyl group having 2 to 8 carbon atoms,
Q² represents hydrogen atom, a halogen atom, hydroxyl group, nitro group, amino group, an alkyl group having 1 to 8 carbon atoms, a cycloalkyl group having a 3-to 7-membered ring, an alkenyl group having 2 to 8 carbon atoms, an alkynyl group having 2 to 8 carbon atoms, an alkoxy group having 1 to 8 carbon atoms, an alkyl group having 1 to 8 carbon atoms and substituted with a cycloalkyl group having a 3- to 7-membered ring, an alkyl group having 1 to 8 carbon atoms and substituted with a halogen atom, an alkyl group having 1 to 8 carbon atoms and substituted with an alkoxy group having 1 to 8 carbon atoms, an alkoxy group having 1 to 8 carbon atoms and substituted with a halogen atom, an acyl group having 2 to 8 carbon atoms, an aryl group having 6 to 10 carbon atoms, or an aralkyl group (the aryl moiety thereof has 6 to 10 carbon atoms, and the alkylene moiety thereof has 1 to 8 carbon atoms),
t represents an integer of 1 to 4, and
u represents an integer of 1 to 5.

As for the general formula (IV-II), examples of the alkyl group having 1 to 8 carbon atoms as R¹¹, R¹², R¹³, R¹⁴, R¹⁵, R¹⁶, R¹⁸, R¹⁹, and Q¹ include methyl group, ethyl group, propyl group, isopropyl group, butyl group, i-butyl group, t-butyl group, pentyl group, hexyl group, and the like.

As for the general formula (IV-II), examples of the alkenyl group having 2 to 8 carbon atoms as R¹¹, R¹², R¹⁸, R¹⁹, and Q¹ include vinyl group, allyl group, and the like.

As for the general formula (IV-II), examples of the alkynyl group having 2 to 8 carbon atoms as R¹¹ and Q¹ include propargyl group, and the like.

As for the general formula (IV-II), examples of the cycloalkyl group having a 3-to 7-membered ring as R¹¹ and Q¹ include cyclopropyl group, cyclopentyl group, cyclohexyl group, and the like.

As for the general formula (IV-II), examples of the alkoxy group having 1 to 8 carbon atoms as R¹¹ and Q¹ include methoxy group, ethoxy group, propoxy group, isopropoxy group, butoxy group, i-butoxy group, t-butoxy group, pentyloxy group, hexyloxy group, and the like.

As for the general formula (IV-II), examples of the halogen atom as R¹¹ and Q¹ include fluorine atom, chlorine atom, bromine atom, and the like.

As for the general formula (IV-II), examples of the alkyl group having 1 to 8 carbon atoms and substituted with a halogen atom as R¹¹, R¹², R¹³, R¹⁴, R¹⁵, R¹⁶, R¹⁸, R¹⁹, and Q¹ include methyl group, ethyl group, propyl group, isopropyl group, butyl group, t-butyl group substituted with 1 to 3 halogen atoms such as fluorine atom, chlorine atom, and bromine atom, and the like, and preferred examples include trifluoromethyl group, chloromethyl group, 2-chloroethyl group, 2-bromoethyl group, 2-fluoroethyl group, and the like.

As for the general formula (IV-II), examples of the alkyl group having 1 to 8 carbon atoms and substituted with an alkoxy group having 1 to 8 carbon atoms as R¹¹, R¹², and Q¹ include methyl group, ethyl group, propyl group, isopropyl group, butyl group, i-butyl group, t-butyl group, pentyl group, hexyl group substituted with methoxy group, ethoxy group, propoxy group, isopropoxy group, butoxy group, i-butoxy group, t-butoxy group, pentyloxy group, hexyloxy group, or the like, and the like, and preferred examples include ethoxyethyl group, and the like.

As for the general formula (IV-II), examples of the alkoxy group having 1 to 8 carbon atoms and substituted with a halogen atom as R¹¹ and Q¹ include methoxy group, ethoxy group, propoxy group, isopropyloxy group, butyloxy group, t-butyloxy group substituted with 1 to 3 halogen atoms such as fluorine atom, chlorine atom, and bromine atom, and the like, and preferred examples include trifluoromethyloxy group, chloromethyloxy group, 2-chloroethyloxy group, 2-bromoethyloxy group, 2-fluoroethyloxy group, and the like.

As for the general formula (IV-II), examples of the aryl group having 6 to 10 carbon atoms as R¹¹, R¹², and Q¹ include phenyl group, and the like.

As for the general formula (IV-II), examples of the acyl group having 2 to 8 carbon atoms as R¹¹, R¹², R¹⁸, R¹⁹, and Q¹ include acetyl group.

As for the general formula (IV-II), examples of the heterocyclic group having a 5- or 6-membered ring as R¹¹ include pyridyl group, and the like.

As for the general formula (IV-II), examples of the alkyl group having 1 to 8 carbon atoms and substituted with a cycloalkyl group having a 3- to 7-membered ring as R¹¹, R¹², and Q¹ include methyl group, ethyl group, propyl group, isopropyl group, butyl group, i-butyl group, t-butyl group, pentyl group, hexyl group substituted with cyclopropyl group, cyclopentyl group, cyclohexyl group, or the like, and the like.

As for the general formula (IV-II), examples of the aralkyl group (the aryl moiety thereof has 6 to 10 carbon atoms, and the alkylene moiety thereof has 1 to 8 carbon atoms) as R¹¹, R¹², R¹⁹, and Q¹ include benzyl group, phenethyl group, and the like.

As for the general formula (IV-II), examples of the alkyl group having 1 to 8 carbon atoms and substituted with a heterocyclic group having a 5- or 6-membered ring as R¹¹ include methyl group, ethyl group, propyl group, isopropyl group, butyl group, i-butyl group, t-butyl group, pentyl group, hexyl group substituted with pyridyl group, or the like, and the like.

As for the general formula (IV-II), each of R¹¹ (except for hydrogen atom) and Q¹ may consist of 1 to 3 of the same or different atoms or groups.

As for the general formula (IV-III), examples of the alkyl group having 1 to 8 carbon atoms as R²¹, R²², R²³, R²⁴, R²⁵, R²⁶, R²⁸, and Q² include methyl group, ethyl group, propyl group, isopropyl group, butyl group, i-butyl group, t-butyl group, pentyl group, hexyl group, and the like.

As for the general formula (IV-III), examples of the alkenyl group having 2 to 8 carbon atoms as R²¹, R²², R²⁸, and Q² include vinyl group, allyl group, and the like.

As for the general formula (IV-III), examples of the alkynyl group having 2 to 8 carbon atoms as R²¹ and Q² include propargyl group, and the like.

As for the general formula (IV-III), examples of the cycloalkyl group having a 3- to 7-membered ring as R²¹ and Q² include cyclopropyl group, cyclopentyl group, cyclohexyl group, and the like.

As for the general formula (IV-III), examples of the alkoxy group having 1 to 8 carbon atoms as R²¹ and Q² include methoxy group, ethoxy group, propoxy group, isopropoxy group, butoxy group, i-butoxy group, t-butoxy group, pentyloxy group, hexyloxy group, and the like.

As for the general formula (IV-III), examples of the halogen atom as R²¹ and Q² include fluorine atom, chlorine atom, bromine atom, and the like.

As for the general formula (IV-III), examples of the alkyl group having 1 to 8 carbon atoms and substituted with a halogen atom as R²¹, R²², R²³, R²⁴, R²⁵, R²⁶, R²⁸, and Q² include methyl group, ethyl group, propyl group, isopropyl group, butyl group, t-butyl group substituted with 1 to 3 halogen atoms such as fluorine atom, chlorine atom, and bromine atom, and the like, and preferred examples include trifluoromethyl group, chloromethyl group, 2-chloroethyl group, 2-bromoethyl group, 2-fluoroethyl group, and the like.

As for the general formula (IV-III), examples of the alkyl group having 1 to 8 carbon atoms and substituted with an alkoxy group having 1 to 8 carbon atoms as R²¹, R²², and Q² include methyl group, ethyl group, propyl group, isopropyl group, butyl group, i-butyl group, t-butyl group, pentyl group, hexyl group substituted with methoxy group, ethoxy group, propoxy group, isopropoxy group, butoxy group, i-butoxy group, t-butoxy group, pentyloxy group, hexyloxy group, or the like, and the like, and preferred examples include ethoxyethyl group, and the like.

As for the general formula (IV-III), examples of the alkoxy group having 1 to 8 carbon atoms and substituted with a halogen atom as R²¹ and Q² include methoxy group, ethoxy group, propoxy group, isopropyloxy group, butyloxy group, t-butyloxy group substituted with 1 to 3 halogen atoms such as fluorine atom, chlorine atom, and bromine atom, and the like, and preferred examples include trifluoromethyloxy group, chloromethyloxy group, 2-chloroethyloxy group, 2-bromoethyloxy group, 2-fluoroethyloxy group, and the like.

As for the general formula (IV-III), examples of the aryl group having 6 to 10 carbon atoms as R²¹, R²², and Q² include phenyl group, and the like.

As for the general formula (IV-III), examples of the acyl group having 2 to 8 carbon atoms as R²¹, R²², R²⁸, and Q² include acetyl group.

As for the general formula (IV-III), examples of the heterocyclic group having a 5- or 6-membered ring as R²¹ include pyridyl group, and the like.

As for the general formula (IV-III), examples of the alkyl group having 1 to 8 carbon atoms and substituted with a cycloalkyl group having a 3- to 7-membered ring as R²¹, R²², and Q² include methyl group, ethyl group, propyl group, isopropyl group, butyl group, i-butyl group, t-butyl group, pentyl group, hexyl group substituted with cyclopropyl group, cyclopentyl group, cyclohexyl group, or the like, and the like.

As for the general formula (IV-III), examples of the aralkyl group (the aryl moiety thereof has 6 to 10 carbon atoms, and the alkylene moiety thereof has 1 to 8 carbon atoms) as R²¹, R²², and Q² include benzyl group, phenethyl group, and the like.

As for the general formula (IV-III), examples of the alkyl group having 1 to 8 carbon atoms and substituted with a heterocyclic group having a 5- or 6-membered ring as R²¹ include methyl group, ethyl group, propyl group, isopropyl group, butyl group, i-butyl group, t-butyl group, pentyl group, hexyl group substituted with pyridyl group or the like, and the like.

In the general formula (IV-III), each of R²¹ (except for hydrogen atom) and Q² may consist of 1 to 3 of the same or different atoms or groups.

In a preferred embodiment, the compound usable for the present invention is any one of the compounds of 1) to 39) described below:
1) a compound represented by the general formula (I), a tautomer, a stereoisomer, or a pharmaceutically acceptable salt of the compound, or a solvate of any of the foregoing, wherein B¹ is N, and B² is O;
2) a compound represented by the general formula (I), a tautomer, a stereoisomer, or a pharmaceutically acceptable salt of the compound, or a solvate of any of the foregoing, wherein A is S;
3) the compound represented by the general formula (I), a tautomer, a stereoisomer, or a pharmaceutically acceptable salt of the compound, or a solvate of any of the foregoing according to 1) described above, wherein A is S;
4) a compound represented by the general formula (I), a tautomer, a stereoisomer, or a pharmaceutically acceptable salt of the compound, or a solvate of any of the foregoing, wherein A is O;
5) the compound represented by the general formula (I), a tautomer, a stereoisomer, or a pharmaceutically acceptable salt of the compound, or a solvate of any of the foregoing according to 1) described above, wherein A is O;
6) a compound represented by the general formula (I), a tautomer, a stereoisomer, or a pharmaceutically acceptable salt of the compound, or a solvate of any of the foregoing, wherein Z is O;
7) the compound represented by the general formula (I), a tautomer, a stereoisomer, or a pharmaceutically acceptable salt of the compound, or a solvate of any of the foregoing according to any one of 1) to 6) described above, wherein Z is O;
8) a compound represented by the general formula (I), a tautomer, a stereoisomer, or a pharmaceutically acceptable salt of the compound, or a solvate of any of the foregoing, wherein Z is NH;
9) the compound represented by the general formula (I), a tautomer, a stereoisomer, or a pharmaceutically acceptable salt of the compound, or a solvate of any of the foregoing according to any one of 1) to 6) described above, wherein Z is NH;
10) a compound represented by the general formula (I), a tautomer, a stereoisomer, or a pharmaceutically acceptable salt of the compound, or a solvate of any of the foregoing, wherein Z is S;
11) the compound represented by the general formula (I), a tautomer, a stereoisomer, or a pharmaceutically acceptable salt of the compound, or a solvate of any of the foregoing according to any one of 1) to 9) described above, wherein Z is S;
12) a compound represented by the general formula (I), a tautomer, a stereoisomer, or a pharmaceutically acceptable salt of the compound, or a solvate of any of the foregoing, wherein R¹ is a phenyl group which may have an alkyl group substituted with 1 to 3 halogen atoms;
13) the compound represented by the general formula (I), a tautomer, a stereoisomer, or a pharmaceutically acceptable salt of the compound, or a solvate of any of the foregoing according to any one of 1) to 11) described above, wherein R¹ is a phenyl group which may have an alkyl group substituted with 1 to 3 halogen atoms;
14) a compound represented by the general formula (I), a tautomer, a stereoisomer, or a pharmaceutically acceptable salt of the compound, or a solvate of any of the foregoing, wherein X¹ and X² are atomic bonds, and Y is ethylene group;
15) the compound represented by the general formula (I), a tautomer, a stereoisomer, or a pharmaceutically acceptable salt of the compound, or a solvate of any of the foregoing according to any one of 1) to 13) described above, wherein X¹ and X² are atomic bonds, and Y is ethylene group;
16) a compound represented by the general formula (I), a tautomer, a stereoisomer, or a pharmaceutically acceptable salt of the compound, or a solvate of any of the foregoing, wherein R² is isopropyl group;
17) the compound represented by the general formula (I), a tautomer, a stereoisomer, or a pharmaceutically acceptable salt of the compound, or a solvate of any of the foregoing according to any one of 1) to 15) described above, wherein R² is isopropyl group;
18) a compound represented by the general formula (I), a tautomer, a stereoisomer, or a pharmaceutically acceptable salt of the compound, or a solvate of any of the foregoing, wherein R³ is an alkyl group having 1 to 3 carbon atoms;
19) the compound represented by the general formula (I), a tautomer, a stereoisomer, or a pharmaceutically acceptable salt of the compound, or a solvate of any of the foregoing according to any one of 1) to 17) described above, wherein R³ is an alkyl group having 1 to 3 carbon atoms;
20) a compound represented by the general formula (I), a tautomer, a stereoisomer, or a pharmaceutically acceptable salt of the compound, or a solvate of any of the foregoing, wherein R⁴ and R⁵ are hydrogen atoms;
21) the compound represented by the general formula (I), a tautomer, a stereoisomer, or a pharmaceutically acceptable salt of the compound, or a solvate of any of the foregoing according to any one of 1) to 19) described above, wherein R⁴ and R⁵ are hydrogen atoms;
22) a compound represented by the general formula (I), a tautomer, a stereoisomer, or a pharmaceutically acceptable salt of the compound, or a solvate of any of the foregoing, wherein R⁶ is hydrogen atom;
23) the compound represented by the general formula (I), a tautomer, a stereoisomer, or a pharmaceutically acceptable salt of the compound, or a solvate of any of the foregoing according to any one of 1) to 21) described above, wherein R⁶ is hydrogen atom,
24) a compound represented by the general formula (II), a tautomer, a stereoisomer, or a pharmaceutically acceptable salt of the compound, or a solvate of any of the foregoing wherein R¹ is a phenyl group which may have a group or an atom selected from an alkyl group having 1 to 8 carbon atoms, an alkyl group having 1 to 8 carbon atoms and substituted with 1 to 3 halogen atoms, an alkoxy group having 1 to 8 carbon atoms, an alkoxy group having 1 to 8 carbon atoms and substituted with 1 to 3 halogen atoms, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent;
25) a compound represented by the general formula (II), a tautomer, a stereoisomer, or a pharmaceutically acceptable salt of the compound, or a solvate of any of the foregoing, wherein R² is an alkyl group having 2 to 8 carbon atoms, or the compound, a tautomer, a stereoisomer, or a pharmaceutically acceptable salt of the compound, or a solvate of any of the foregoing according to 24) described above, wherein R² is an alkyl group having 2 to 8 carbon atoms;
26) a compound represented by the general formula (II), a tautomer, a stereoisomer, or a pharmaceutically acceptable salt of the compound, or a solvate of any of the foregoing, wherein the substitution position of R¹ is the 2-position (when the substitution position of R¹ is the 2-position, the substitution position of R² is the 4-position, and the substitution position of -X-Y- is the 5-position, or the substitution position of R² is the 5-position, and the substitution position of -X-Y- is the 4-position), or the compound, a tautomer, a stereoisomer, or a pharmaceutically acceptable salt of the compound, or a solvate of any of the foregoing according to 24) or 25) described above, wherein the substitution position of R¹ is the 2-position (when the substitution position of R¹ is the 2-position, the substitution position of R² is the 4-position, and the substitution position of -X-Y- is the 5-position, or the substitution position of R² is the 5-position, and the substitution position of -X-Y- is the 4-position);
27) a compound represented by the general formula (II), a tautomer, a stereoisomer, or a pharmaceutically acceptable salt of the compound, or a solvate of any of the foregoing, wherein A is oxygen atom or sulfur atom, or the compound, a tautomer, a stereoisomer, or a pharmaceutically acceptable salt of the compound, or a solvate of any of the foregoing according to any one of 24) to 26) described above, wherein A is oxygen atom or sulfur atom;
28) a compound represented by the general formula (II), a tautomer, a stereoisomer, or a pharmaceutically acceptable salt of the compound, or a solvate of any of the foregoing, wherein X is an alkylene chain having 1 to 8 carbon atoms, or the compound, a tautomer, a stereoisomer, or a pharmaceutically acceptable salt of the compound, or a solvate of any of the foregoing according to any one of 24) to 27) described above, wherein X is an alkylene chain having 1 to 8 carbon atoms;
29) a compound represented by the general formula (II), a tautomer, a stereoisomer, or a pharmaceutically acceptable salt of the compound, or a solvate of any of the foregoing, wherein Y is C(=O), or the compound, a tautomer, a stereoisomer, or a pharmaceutically acceptable salt of the compound, or a solvate of any of the foregoing according to any one of 24) to 28) described above, wherein Y is C(=O);
30) a compound represented by the general formula (II), a tautomer, a stereoisomer, or a pharmaceutically acceptable salt of the compound, or a solvate of any of the foregoing, wherein R³, R⁴, and R⁵ are hydrogen atom, an alkyl group having 1 to 8 carbon atoms, or an alkyl group having 1 to 8 carbon atoms and substituted with a halogen atom, or the compound, a tautomer, a stereoisomer, or a pharmaceutically acceptable salt of the compound, or a solvate of any of the foregoing according to any one of 24) to 29) described above, wherein R³, R⁴, and R⁵ are hydrogen atom, an alkyl group having 1 to 8 carbon atoms, or an alkyl group having 1 to 8 carbon atoms and substituted with a halogen atom;
31) a compound represented by the general formula (II), a tautomer, a stereoisomer, or a pharmaceutically acceptable salt of the compound, or a solvate of any of the foregoing, wherein B is CH, or the compound, a tautomer, a stereoisomer, or a pharmaceutically acceptable salt of the compound, or a solvate of any of the foregoing according to any one of 24) to 30) described above, wherein B is CH;
32) a compound represented by the general formula (II), a tautomer, a stereoisomer, or a pharmaceutically acceptable salt of the compound, or a solvate of any of the foregoing, wherein Z is oxygen atom, or the compound, a tautomer, a stereoisomer, or a pharmaceutically acceptable salt of the compound, or a solvate of any of the foregoing according to any one of 24) to 31) described above, wherein Z is oxygen atom;
33) a compound represented by the general formula (II), a tautomer, a stereoisomer, or a pharmaceutically acceptable salt of the compound, or a solvate of any of the foregoing, wherein R⁶ and R⁷ are hydrogen atom, or an alkyl group having 1 to 4 carbon atoms, or the compound, a tautomer, a stereoisomer, or a pharmaceutically acceptable salt of the compound, or a solvate of any of the foregoing according to any one of 24) to 32) described above, wherein R⁶ and R⁷ are hydrogen atom, or an alkyl group having 1 to 4 carbon atoms;
34) a compound represented by the general formula (II), a tautomer, a stereoisomer, or a pharmaceutically acceptable salt of the compound, or a solvate of any of the foregoing, wherein R⁸ is hydrogen atom, or the compound, a tautomer, a stereoisomer, or a pharmaceutically acceptable salt of the compound, or a solvate of any of the foregoing according to any one of 24) to 33) described above, wherein R⁸ is hydrogen atom;
35) a compound represented by the general formula (II), a tautomer, a stereoisomer, or a pharmaceutically acceptable salt of the compound, or a solvate of any of the foregoing, wherein R¹ is a phenyl group or naphthyl group which may have a group or an atom selected from an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent, R² is an alkyl group having 2 to 8 carbon atoms, A is oxygen atom or sulfur atom, X is an alkylene chain having 1 to 8 carbon atoms which may have an alkyl group having 1 to 8 carbon atoms as a substituent, and may contain a double bond, Y is C(=O), CH=CH, or C(=CH₂), R³, R⁴, and R⁵ are hydrogen atom, an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, or pyridyl group, B is CH, Z is oxygen atom or sulfur atom, R⁶ and R⁷ are hydrogen atom, or an alkyl group having 1 to 8 carbon atoms, and R⁸ is hydrogen atom, or an alkyl group having 1 to 8 carbon atoms;
36) the compound represented by the general formula (II), a tautomer, a stereoisomer, or a pharmaceutically acceptable salt of the compound, or a solvate of any of the foregoing according to 35) described above, wherein X is an alkylene chain having 1 to 8 carbon atoms;
37) the compound represented by the general formula (II), a tautomer, a stereoisomer, or a pharmaceutically acceptable salt of the compound, or a solvate of any of the foregoing according to 35) or 36) described above, wherein the substitution position of R¹ is the 2-position;
38) the compound represented by the general formula (II), a tautomer, a stereoisomer, or a pharmaceutically acceptable salt of the compound, or a solvate of any of the foregoing according to any one of 35) to 37) described above, wherein R⁸ is hydrogen atom; and
39) the compound represented by the general formula (II), a tautomer, a stereoisomer, or a pharmaceutically acceptable salt of the compound, or a solvate of any of the foregoing according to any one of 35) to 38) described above, wherein a substituent other than hydrogen atom as R³, R⁴, or R⁵ binds at the ortho-position of -Z-CR⁶R⁷CO₂R⁸.

In a preferred embodiment, the compound usable for the present invention is any one of the compounds of 40) to 79) described below:
40) a compound represented by the general formula (III-I), a tautomer, a stereoisomer, or a pharmaceutically acceptable salt of the compound, or a solvate of any of the foregoing, wherein W¹ and W² are both CH;
41) a compound represented by the general formula (III-I), or the compound according to 40) described above, a tautomer, a stereoisomer, or a pharmaceutically acceptable salt of the compound, or a solvate of any of the foregoing, wherein X is CR⁶R⁷;
42) a compound represented by the general formula (III-I), or the compound according to 40) described above, a tautomer, a stereoisomer, or a pharmaceutically acceptable salt of the compound, or a solvate of any of the foregoing, wherein X is CH₂;
43) a compound represented by the general formula (III-I), or the compound according to 40) described above, a tautomer, a stereoisomer, or a pharmaceutically acceptable salt of the compound, or a solvate of any of the foregoing, wherein X is NR⁵;
44) a compound represented by the general formula (III-I), or the compound according to 40) described above, a tautomer, a stereoisomer, or a pharmaceutically acceptable salt of the compound, or a solvate of any of the foregoing, wherein X is NH;
45) a compound represented by the general formula (III-I), or the compound according to 40) described above, a tautomer, a stereoisomer, or a pharmaceutically acceptable salt of the compound, or a solvate of any of the foregoing, wherein X is N(alkyl group having 1 to 8 carbon atoms);
46) a compound represented by the general formula (III-I), or the compound according to any one of 40) to 45) described above, a tautomer, a stereoisomer, or a pharmaceutically acceptable salt of the compound, or a solvate of any of the foregoing, wherein Y is CH₂;
47) a compound represented by the general formula (III-I), or the compound according to any one of 40) to 46) described above, a tautomer, a stereoisomer, or a pharmaceutically acceptable salt of the compound, or a solvate of any of the foregoing, wherein Z is carboxyl group;
48) a compound represented by the general formula (III-I), or the compound according to any one of 40) to 47) described above, a tautomer, a stereoisomer, or a pharmaceutically acceptable salt of the compound, or a solvate of any of the foregoing, wherein G is O;
49) a compound represented by the general formula (III-I), or the compound according to any one of 40) to 48) described above, a tautomer, a stereoisomer, or a pharmaceutically acceptable salt of the compound, or a solvate of any of the foregoing, wherein A is thiazole which may have a substituent selected from an alkyl group having 1 to 8 carbon atoms, an alkenyl group having 2 to 8 carbon atoms, an alkynyl group having 2 to 8 carbon atoms, an alkoxy group having 1 to 8 carbon atoms, a halogen atom, an alkyl group having 1 to 8 carbon atoms and substituted with a halogen atom, an alkoxy group having 1 to 8 carbon atoms and substituted with a halogen atom, hydroxyl group, nitro group, an acyl group having 2 to 8 carbon atoms, an aryl group having 6 to 10 carbon atoms, and a heterocyclic group having a 5- or 6-membered ring;
50) a compound represented by the general formula (III-I), or the compound according to any one of 40) to 49) described above, a tautomer, a stereoisomer, or a pharmaceutically acceptable salt of the compound, or a solvate of any of the foregoing, wherein B is ethylene chain;
51) a compound represented by the general formula (III-I), or the compound according to any one of 40) to 50) described above, a tautomer, a stereoisomer, or a pharmaceutically acceptable salt of the compound, or a solvate of any of the foregoing, wherein R¹ and R² may be the same or different, and are hydrogen atom, an alkyl group having 1 to 8 carbon atoms, an alkenyl group having 2 to 8 carbon atoms, an alkoxy group having 1 to 8 carbon atoms, a halogen atom, an alkyl group having 1 to 8 carbon atoms and substituted with a halogen atom, or an alkoxy group having 1 to 8 carbon atoms and substituted with a halogen atom;
52) a compound represented by the general formula (III-I), or the compound according to any one of 40) to 50) described above, a tautomer, a stereoisomer, or a pharmaceutically acceptable salt of the compound, or a solvate of any of the foregoing, wherein R¹ and R² may be the same or different, and are hydrogen atom, an alkyl group having 1 to 8 carbon atoms, a halogen atom, or an alkyl group having 1 to 8 carbon atoms and substituted with a halogen atom;
53) a compound represented by the general formula (III-I), or the compound according to any one of 40) to 52) described above, a tautomer, a stereoisomer, or a pharmaceutically acceptable salt of the compound, or a solvate of any of the foregoing, wherein R³ and R⁴ are both hydrogen atoms;
54) a compound represented by the general formula (III-I), or the compound according to any one of 40) to 52) described above, a tautomer, a stereoisomer, or a pharmaceutically acceptable salt of the compound, or a solvate of any of the foregoing, wherein m is 0;
55) a compound represented by the general formula (III-II), a tautomer, a stereoisomer, or a pharmaceutically acceptable salt of the compound, or a solvate of any of the foregoing, wherein G^{a} is O;
56) a compound represented by the general formula (III-II), or the compound according to 55) described above, a tautomer, a stereoisomer, or a pharmaceutically acceptable salt of the compound, or a solvate of any of the foregoing, wherein A^{a} is thiazole which may have a substituent selected from an alkyl group having 1 to 8 carbon atoms, an alkoxy group having 1 to 8 carbon atoms, a halogen atom, an alkyl group having 1 to 8 carbon atoms and substituted with a halogen atom, an alkoxy group having 1 to 8 carbon atoms and substituted with a halogen atom, hydroxyl group, nitro group, and an acyl group having 2 to 8 carbon atoms;
57) a compound represented by the general formula (III-II), or the compound according to 55) or 56) described above, a tautomer, a stereoisomer, or a pharmaceutically acceptable salt of the compound, or a solvate of any of the foregoing, wherein B^{a} is ethylene chain;
58) a compound represented by the general formula (III-II), or the compound according to any one of 55) to 57) described above, a tautomer, a stereoisomer, or a pharmaceutically acceptable salt of the compound, or a solvate of any of the foregoing, wherein R^{1a} and R^{2a} may be the same or different, and are hydrogen atom, an alkyl group having 1 to 8 carbon atoms, an alkoxy group having 1 to 8 carbon atoms, a halogen atom, an alkyl group having 1 to 8 carbon atoms and substituted with a halogen atom, or an alkoxy group having 1 to 8 carbon atoms and substituted with a halogen atom;
59) a compound represented by the general formula (III-III), a tautomer, a stereoisomer, or a pharmaceutically acceptable salt of the compound, or a solvate of any of the foregoing, wherein G^{b} is O;
60) a compound represented by the general formula (III-III), or the compound according to 59) described above, a tautomer, a stereoisomer, or a pharmaceutically acceptable salt of the compound, or a solvate of any of the foregoing, wherein A^{b} is thiazole which may have a substituent selected from an alkyl group having 1 to 8 carbon atoms, an alkoxy group having 1 to 8 carbon atoms, a halogen atom, an alkyl group having 1 to 8 carbon atoms and substituted with a halogen atom, an alkoxy group having 1 to 8 carbon atoms and substituted with a halogen atom, hydroxyl group, nitro group, and an acyl group having 2 to 8 carbon atoms;
61) a compound represented by the general formula (III-III), or the compound according to 59) or 60) described above, a tautomer, a stereoisomer, or a pharmaceutically acceptable salt of the compound, or a solvate of any of the foregoing, wherein B^{b} is ethylene chain;
62) a compound represented by the general formula (III-III), or the compound according to any one of 59) to 61) described above, a tautomer, a stereoisomer, or a pharmaceutically acceptable salt of the compound, or a solvate of any of the foregoing, wherein R^{1b} and R^{2b} may be the same or different, and are hydrogen atom, an alkyl group having 1 to 8 carbon atoms, an alkoxy group having 1 to 8 carbon atoms, a halogen atom, an alkyl group having 1 to 8 carbon atoms and substituted with a halogen atom, or an alkoxy group having 1 to 8 carbon atoms and substituted with a halogen atom;
63) a compound represented by the general formula (IV-II), a tautomer, a stereoisomer, or a pharmaceutically acceptable salt of the compound, or a solvate of any of the foregoing, wherein R¹¹ is hydrogen atom, an alkyl group having 1 to 8 carbon atoms, or an alkyl group having 1 to 8 carbon atoms and substituted with a halogen atom;
64) a compound represented by the general formula (IV-II), a tautomer, a stereoisomer, or a pharmaceutically acceptable salt of the compound, or a solvate of any of the foregoing, wherein R¹² is an alkyl group having 1 to 8 carbon atoms, or an alkyl group having 1 to 8 carbon atoms and substituted with a halogen atom;
65) a compound represented by the general formula (IV-II), a tautomer, a stereoisomer, or a pharmaceutically acceptable salt of the compound, or a solvate of any of the foregoing, wherein R¹³ and R¹⁴ are hydrogen atoms;
66) a compound represented by the general formula (IV-II), a tautomer, a stereoisomer, or a pharmaceutically acceptable salt of the compound, or a solvate of any of the foregoing, wherein R¹⁵ and R¹⁶ may be the same or different, and are hydrogen atom, or an alkyl group having 1 to 8 carbon atoms;
67) a compound represented by the general formula (IV-II), a tautomer, a stereoisomer, or a pharmaceutically acceptable salt of the compound, or a solvate of any of the foregoing, wherein Y¹ is oxygen atom, N(alkyl group having 1 to 8 carbon atoms), or an atomic bond;
68) a compound represented by the general formula (IV-II), a tautomer, a stereoisomer, or a pharmaceutically acceptable salt of the compound, or a solvate of any of the foregoing, wherein A¹ is oxygen atom, CH₂, N-OH, or N(O-benzyl group);
69) a compound represented by the general formula (IV-II), a tautomer, a stereoisomer, or a pharmaceutically acceptable salt of the compound, or a solvate of any of the foregoing, wherein Q¹ is an alkyl group having 1 to 8 carbon atoms, or an alkyl group having 1 to 8 carbon atoms and substituted with a halogen atom;
70) a compound represented by the general formula (IV-II), a tautomer, a stereoisomer, or a pharmaceutically acceptable salt of the compound, or a solvate of any of the foregoing, wherein r is 2;
71) a compound represented by the general formula (IV-II), a tautomer, a stereoisomer, or a pharmaceutically acceptable salt of the compound, or a solvate of any of the foregoing, wherein s is 1 or 2;
72) a compound represented by the general formula (IV-III), a tautomer, a stereoisomer, or a pharmaceutically acceptable salt of the compound, or a solvate of any of the foregoing, wherein R²¹ is hydrogen atom, an alkyl group having 1 to 8 carbon atoms, or an alkyl group having 1 to 8 carbon atoms and substituted with a halogen atom;
73) a compound represented by the general formula (IV-III), a tautomer, a stereoisomer, or a pharmaceutically acceptable salt of the compound, or a solvate of any of the foregoing, wherein R²² is an alkyl group having 1 to 8 carbon atoms, or an alkyl group having 1 to 8 carbon atoms and substituted with a halogen atom;
74) a compound represented by the general formula (IV-III), a tautomer, a stereoisomer, or a pharmaceutically acceptable salt of the compound, or a solvate of any of the foregoing, wherein R²³ and R²⁴ are hydrogen atoms;
75) a compound represented by the general formula (IV-III), a tautomer, a stereoisomer, or a pharmaceutically acceptable salt of the compound, or a solvate of any of the foregoing, wherein R²⁵ and R²⁶ may be the same or different, and are hydrogen atom, or an alkyl group having 1 to 8 carbon atoms;
76) a compound represented by the general formula (IV-III), a tautomer, a stereoisomer, or a pharmaceutically acceptable salt of the compound, or a solvate of any of the foregoing, wherein Y² is oxygen atom, N(alkyl group having 1 to 8 carbon atoms), or an atomic bond;
77) a compound represented by the general formula (IV-III), a tautomer, a stereoisomer, or a pharmaceutically acceptable salt of the compound, or a solvate of any of the foregoing, wherein Q² is an alkyl group having 1 to 8 carbon atoms, or an alkyl group having 1 to 8 carbon atoms and substituted with a halogen atom;
78) a compound represented by the general formula (IV-III), a tautomer, a stereoisomer, or a pharmaceutically acceptable salt of the compound, or a solvate of any of the foregoing, wherein t is 2; and
79) a compound represented by the general formula (IV-III), a tautomer, a stereoisomer, or a pharmaceutically acceptable salt of the compound, or a solvate of any of the foregoing, wherein u is 1 or 2.

In a more preferred embodiment, the compound represented by the general formula (I) usable for the present invention is any one of the compounds of (a) to (j) described below:
(a) [3-[2-[4-isopropyl-2-(4-trifluoromethyl)phenyl-5-thiazolyl]ethyl]-5-methyl-1,2-benzisoxazol-6-yl]oxyacetic acid;
(b) 2-[[3-[2-[4-isopropyl-2-(4-trifluoromethyl)phenyl-5-thiazolyl]ethyl]-5-methyl-1,2-benzisoxazol-6-yl]oxy]-2-methylpropionic acid;
(c) [3-[2-[4-isopropyl-2-(4-trifluoromethyl)phenyl-5-thiazolyl]ethyl]-5-methyl-1,2-benzisoxazol-6-yl]thioacetic acid;
(d) [3-[2-[4-isopropyl-2-(4-trifluoromethyl)phenyl-5-thiazolyl]ethyl]-5-methyl-1,2-benzisoxazol-6-yl]aminoacetic acid;
(e) [3-[2-[2-(2,4-dichlorophenyl)-5-isopropyl-4-oxazolyl]ethyl]-5-methyl-1,2-benzisoxazol-6-yl]oxyacetic acid;
(f) 2-[[3-[2-[4-isopropyl-2-(4-trifluoromethyl)phenyl-5-thiazolyl]ethyl]-5-methyl-1,2-benzisoxazol-6-yl]oxy]-2-methylpropionic acid 2-piperidinoethyl ester hydrochloride;
(g) [[7-allyl-3-[2-[2-(2,4-dichlorophenyl)-5-isopropyl-4-oxazolyl]ethyl]-1,2-benzisoxazol-6-yl]oxy]acetic acid;
(h) 2-[[7-allyl-3-[2-[2-(2,4-dichlorophenyl)-5-isopropyl-4-oxazolyl]ethyl]-1,2-benzisoxazol-6-yl]oxy]-2-methylpropionic acid;
(i) [[7-propyl-3-[2-[2-(2,4-dichlorophenyl)-5-isopropyl-4-oxazolyl]ethyl]-1,2-benzisoxazol-6-yl]oxy]acetic acid; and
(j) 2-[[7-allyl-3-[2-[2-[(4-trifluoromethyl)phenyl]-4-isopropyl-5-thiazolyl]ethyl]-1,2-benzisoxazol-6-yl]oxy]-2-methylpropionic acid,
   a tautomer, a stereoisomer, or a pharmaceutically acceptable salt of the compound, or a solvate of any of the foregoing.

In a more preferred embodiment, the compound represented by the general formula (II) usable for the present invention is any one of:
(1) 2-[4-[3-[2-(2,4-dichlorophenyl)-5-isopropyl-4-oxazolyl]propionyl]-2-methylphenoxy]-2-methylpropionic acid;
(2) [4-[3-[2-(2,4-dichlorophenyl)-5-isopropyl-4-oxazolyl]propionyl]-2-methylphenoxy]acetic acid;
(3) [4-[3-[2-(4-trifluoromethyl)phenyl-4-isopropyl-5-thiazolyl]propionyl]-2-methylphenoxy]acetic acid;
(4) 2-[4-[3-[2-(4-trifluoromethyl)phenyl-4-isopropyl-5-thiazolyl]propionyl]-2-methylphenoxy]-2-methylpropionic acid;
(5) [2-allyl-4-[3-[2-(2,4-dichlorophenyl)-5-isopropyl-4-oxazolyl]propionyl]phenoxy]acetic acid;
(6) [4-[3-[2-(2-hydroxy-4-chlorophenyl)-5-isopropyl-4-oxazolyl]propionyl]-2-methylphenoxy]acetic acid;
(7) [4-[3-[2-(2,4-dichlorophenyl)-5-isopropyl-4-oxazolyl]propionyl]-2-methylphenylsulfanyl]acetic acid;
(8) 2-[4-[3-[2-(2-hydroxy-4-chlorophenyl)-5-isopropyl-4-oxazolyl]propionyl]-2-methylphenoxy]-2-methylpropionic acid;
(9) [4-[3-[2-(2,4-dichlorophenyl)-5-isopropyl-4-oxazolyl]-1-propenyl]-2-methylphenoxy]acetic acid;
(10) [4-[3-[2-(2,4-dichlorophenyl)-5-isopropyl-4-oxazolyl]-1-propenyl]-2-methylphenoxy]acetic acid;
(11) [4-[3-[4-hexyl-2-(4-trifluoromethyl)phenyl-5-thiazolyl]propionyl]-2-methylphenoxy]acetic acid;
(12) 2-[4-[3-[4-hexyl-2-(4-trifluoromethyl)phenyl-5-thiazolyl]propionyl]-2-methylphenoxy]-2-methylpropionic acid;
(13) 2-[4-[3-[4-isopropyl-2-(4-trifluoromethyl)phenyl-5-thiazolyl]-1-propenyl]-2-methylphenoxy]-2-methylpropionic acid;
(14) [4-[3-[4-isopropyl-2-(4-trifluoromethyl)phenyl-5-thiazolyl]propionyl]-3-methylphenoxy]acetic acid;
(15) [4-[3-[2-(2,4-dichlorophenyl)-5-isopropyl-4-oxazolyl]propionyl]-3-methylphenoxy]acetic acid;
(16) [4-[3-[4-isopropyl-2-(4-trifluoromethyl)phenyl-5-thiazolyl]propionyl]-3-methylphenoxy]-2-methylpropionic acid;
(17) 2-[4-[3-[2-(2,4-dichlorophenyl)-5-isopropyl-4-oxazolyl]propionyl]-3-methylphenoxy]-2-methylpropionic acid;
(18) [4-[3-[4-isopropyl-2-(4-trifluoromethyl)phenyl-5-thiazolyl]propionyl]-2-propylphenoxy]acetic acid;
(19) 2-allyl-4-[3-[4-isopropyl-2-(4-trifluoromethyl)phenyl-5-thiazolyl]propionyl]phenoxyacetic acid;
(20) [4-[4-[2-(2,4-dichlorophenyl)-5-isopropyl-4-oxazolyl]-1-buten-2-yl]-2-methylphenoxy]acetic acid;
(21) 2-[4-[4-[2-(2,4-dichlorophenyl)-5-isopropyl-4-oxazolyl]-1-buten-2-yl]-2-methylpropionic acid;
(22) [4-[3-[2-(2,4-dichlorophenyl)-5-isopropyl-4-oxazolyl]-2-methylpropionyl]-2-methylphenoxy]acetic acid;
(23) 2-[4-[3-[2-(2,4-dichlorophenyl)-5-isopropyl-4-oxazolyl]-2-methylpropionyl]-2-methylphenoxy]-2-methylpropionic acid;
(24) [4-[3-[4-isopropyl-2-(4-trifluoromethyl)phenyl-5-thiazolyl]propenoyl]-2-methylphenoxy]acetic acid;
(25) 2-[4-[3-[4-isopropyl-2-(4-trifluoromethyl)phenyl-5-thiazolyl]propenoyl]-2-methylphenoxy]-2-methylpropionic acid;
(26) [4-[3-[4-isopropyl-2-(4-methoxyphenyl)-5-thiazolyl]propionyl]-2-methylphenoxy]propionic acid;
(27) [4-[3-[2-(3,5-dichlorophenyl)-4-isopropylthiazol-5-yl]propionyl]-2-methylphenoxy]acetic acid;
(28) 2-[4-[3-[2-(3,5-difluorophenyl)-4-isopropyl-5-thiazolyl]propionyl]-2-methylphenoxy]-2-methylpropionic acid;
(29) [4-[3-[4-isopropyl-2-(2-naphthyl)-5-thiazolyl]propionyl]-2-methylphenoxy]acetic acid;
(30) 2-[4-[3-[4-isopropyl-2-(2-naphthyl)-5-thiazolyl]propionyl]-2-methylphenoxy]-2-methylpropionic acid;
(31) [4-[3-[2-(4-butylphenyl)-4-isopropyl-5-thiazolyl]propionyl]-2-methylphenoxy]acetic acid;
(32) 2-[4-[3-[2-(4-butylphenyl)-4-isopropyl-5-thiazolyl]propionyl]-2-methylphenoxyl-2-methylpropionic acid;
(33) [4-[3-[4-isopropyl-2-(4-trifluoromethyl)phenyl-5-thiazolyl]propionyl]-2-chlorophenoxy]acetic acid;
(34) [4-[3-[2-(4-trifluoromethyl)phenyl-4-isopropyl-5-thiazolyl]propionyl]-2-chlorophenoxy]-2-methylpropionic acid;
(35) [4-[3-[2-(2,4-dichlorophenyl)-5-isopropyl-4-oxazolyl]propionyl]-2-chlorophenoxy]acetic acid;
(36) 2-[4-[3-[2-(2,4-dichlorophenyl)-5-isopropyl-4-oxazolyl]propionyl]-2-chlorophenoxy]-2-methylpropionic acid;
(37) [4-[3-[5-isopropyl-2-(4-trifluoromethyl)phenyl-4-thiazolyl]propionyl]-2-methylphenoxy]acetic acid;
(38) 2-[4-[3-[5-isopropyl-2-(4-trifluoromethyl)phenyl-4-thiazolyl]propionyl]-2-methylphenoxy]-2-methylpropionic acid;
(39) [4-[3-[2-(2,4-dichlorophenyl)-5-isopropyl-4-thiazolyl]propionyl]-2-methylphenoxy]acetic acid;
(40) 2-[4-[3-[2-(2,4-dichlorophenyl)-5-isopropyl-4-thiazolyl]propionyl]-2-methylphenoxy]-2-methylpropionic acid;
(41) [5-[3-[4-isopropyl-2-(4-trifluoromethyl)phenyl-5-thiazolyl]propionyl]-2-methylphenoxy]acetic acid;
(42) 2-[5-[3-[4-isopropyl-2-(4-trifluoromethyl)phenyl-5-thiazolyl]propionyl]-2-methylphenoxy]-2-methylpropionic acid;
(43) 2-[4-[3-[4-isopropyl-2-(4-trifluoromethyl)phenyl-5-thiazolyl]propionyl]-2-methylphenoxy]propionic acid;
(44) 4-[3-[4-methyl-2-(4-trifluoromethyl)phenyl-5-thiazolyl]propionyl]-2-methylphenoxy]acetic acid;
(45) 2-[4-[3-[4-hexyl-2-(4-trifluoromethyl)phenyl-5-thiazolyl]-1-propenyl]-2-methylphenoxy]-2-methylpropionic acid;
(46) 2-[5-[3-[4-hexyl-2-(4-trifluoromethyl)phenyl-5-thiazolyl]propionyl]-2-methylphenoxy]-2-methylpropionic acid;
(47) [4-[3-[4-ethyl-2-(4-trifluoromethyl)phenyl-5-thiazolyl]propionyl]-2-methylphenoxy]acetic acid;
(48) 2-[4-[3-[4-ethyl-2-(4-trifluoromethyl)phenyl-5-thiazolyl]propionyl]-2-methylphenoxy]-2-methylpropionic acid;
(49) [4-[3-[4-isopropyl-2-(4-methylphenyl)-5-thiazolyl]propionyl]-2-methylphenoxy]acetic acid; and
(50) 2-[4-[3-[4-isopropyl-2-(4-methylphenyl)-5-thiazolyl]propionyl]-2-methylphenoxy]-2-methylpropionic acid,
a tautomer, a stereoisomer, or a pharmaceutically acceptable salt of the compound, or a solvate of any of the foregoing.

In a more preferred embodiment, the compound represented by the general formula (III-I) usable for the present invention is any one of:
(1) 3-[4-[3-[4-hexyl-2-(4-methylphenyl)thiazol-5-yl]propionyl]-2-methylphenyl]propionic acid;
(2) 3-[4-[3-[3-isopropyl-5-[4-(trifluoromethyl)phenyllthiophen-2-yl]propionyl]-2-methylphenyl]propionic acid;
(3) 3-[4-[3-(5-isopropyl-2-phenyl-4-oxazolyl)propionyl]-2-methylphenyl]propionic acid;
(4) 3-[4-[3-[4-isopropyl-2-[4-(trifluoromethyl)phenyl]thiazol-5-yl]propenoyl]-2-methylphenyl]acrylic acid;
(5) 3-[4-[3-[4-isopropyl-2-[4-(trifluoromethyl)phenyl]thiazol-5-yl]propionyl]-2-methylphenyl]propionic acid;
(6) 3-[4-[1-[2-[4-isopropyl-2-[4-(trifluoromethyl)phenyl]thiazol-5-yl]ethyl]vinyl]-2-methylphenyl]propionic acid;
(7) N-[4-[3-[4-isopropyl-2-[4-(trifluoromethyl)phenyl]thiazol-5-yl]propionyl]phenyl]-N-methylglycine;
(8) N-[4-[3-[4-isopropyl-2-[4-(trifluoromethyl)pheny1]thiazol-5-yl]propionyl]phenyl]glycine;
(9) N-[4-[3-[3-isopropyl-5-[4-(trifluoromethyl)phenyl]thiophen-2-yl]propionyl]phenyl]-N-methylglycine;
(10) N-[4-[3-[2-(4-chloro-2-hydroxyphenyl)-5-isopropyl-4-oxazolyl]propionyl]phenyl]-N-methylglycine;
(11) 3-[4-[3-[4-isopropyl-2-[4-(trifluoromethyl)phenyl]-5-thiazolyl]propionyl]-2-ethylphenyl]propionic acid;
(12) 3-[4-[3-[2-(4-chloro-2-hydroxyphenyl)-5-isopropyl-4-oxazolyl]propionyl]-2-methylphenyl]propionic acid; and
(13) 3-[4-[3-[5-isopropyl-2-(2-hydroxyphenyl)-4-oxazolyl]propionyl]-2-methylphenyl]propionic acid,
a tautomer, a stereoisomer, or a pharmaceutically acceptable salt of the compound, or a solvate of any of the foregoing.

In a more preferred embodiment, the compound represented by the general formula (IV-1) usable for the present invention is any one of:
(1) 2-methyl-4-[3-(3-methylbenzothiophen-2-yl)propionyl]phenoxyacetic acid;
(2) 2-methyl-2-[2-methyl-4-[3-(3-methylbenzothiophen-2-yl)propionyl]phenoxy]propionic acid;
(3) 2-methyl-4-[3-[3-methyl-5-(trifluoromethyl)benzothiophen-2-yl]propionyl]phenoxyacetic acid;
(4) 2-methyl-2-[2-methyl-4-[3-[3-methyl-5-(trifluoromethyl)benzothiophen-2-yl]propionyl]phenoxy]propionic acid;
(5) 2-methyl-4-[3-[3-methyl-6-(trifluoromethyl)benzothiophen-2-yl]propionyl]phenoxyacetic acid;
(6) 2-methyl-2-[2-methyl-4-[3-[3-methyl-6-(trifluoromethyl)benzothiophen-2-yl]propionyl]phenoxy]propionic acid;
(7) 3-[4-[3-[3-methyl-6-(trifluoromethyl)benzothiophen-2-yl]propionyl]-2-methylphenyl]propionic acid;
(8) 3-[4-[3-[3-ethyl-6-(trifluoromethyl)benzothiophen-2-yl]propionyl]-2-methylphenyl]propionic acid;
(9) 3-[2-methyl-4-[3-[3-propyl-6-(trifluoromethyl)benzothiophen2-yl]propionyl]phenyl]propionic acid;
(10) 3-[2-methyl-4-[3-[3-butyl-6-(trifluoromethyl)benzothiophen-2-yl]propionyl]phenyl]propionic acid;
(11) 3-[2-methyl-4-[3-[3-isobutyl-6-(trifluoromethyl)benzothiophen-2-yl]propionyl]phenyl]propionic acid;
(12) 3-[2-methyl-4-[3-[3-isopropyl-6-(trifluoromethyl)benzothiophen-2-yl]propionyl]phenyl]propionic acid;
(13) 3-[4-[1-hydroxyimino-3-[3-isopropyl-6-(trifluoromethyl)benzothiophen-2-yl]propyl]-2-methylphenyl]propionic acid;
(14) 3-[4-[1-[2-[3-isopropyl-6-(trifluoromethyl)benzothiophen-2-yl]ethyl]vinyl]-2-methylphenyl]propionic acid;
(15) 4-[3-[3-isopropyl-6-(trifluoromethyl)benzothiophen-2-yl]propionyl]-2-methylphenoxyacetic acid;
(16) 4-[1-hydroxyimino-3-[3-isopropyl-6-(trifluoromethyl)benzothiophen-2-yl]propyl]-2-methylphenoxyacetic acid;
(17) 4-[3-[3-isopropyl-6-(trifluoromethyl)benzothiophen-2-yl]-1-methoxyiminopropyl]-2-methylphenoxyacetic acid;
(18) 4-[1-benzyloxyimino-3-[3-isopropyl-6-(trifluoromethyl)benzothiophen-2-yl]propyl]-2-methylphenoxyacetic acid;
(19) [3-[2-[3-isopropyl-6-(trifluoromethyl)benzothiophen-2-yl]ethyl]-5-methylbenzoisoxazol-6-yloxy]acetic acid;
(20) N-[3-[2-[3-isopropyl-6-(trifluoromethyl)benzothiophen-2-yl]ethyl]-5-methylbenzisoxazol-6-yl]-N-methylglycine;
(21) 3-[3-[2-[3-isopropyl-6-(trifluoromethyl)benzothiophen-2-yl]ethyl]-5-methylbenzoisoxazol-6-yl]propionic acid;
(22) 5-hydroxy-2-methyl-4-[3-[3-propyl-6-(trifluoromethyl)benzothiophen-2-yl]propionyl]phenoxyacetic acid;
(23) 5-hydroxy-4-[1-hydroxyimino-3-[3-propyl-6-(trifluoromethyl)benzothiophen-2-yl]propyl]-2-methylphenoxyacetic acid;
(24) N-[5-methyl-3-[2-[3-propyl-6-(trifluoromethyl)benzothiophen-2-yl]ethyl]benzoisoxazol-6-yl]-N-methylglycine;
(25) [5-methyl-3-[2-[3-propyl-6-(trifluoromethyl)benzothiophen-2-yl]ethyl]benzoisoxazol-6-yloxy] acetic acid;
(26) 3-[5-methyl-3-[2-[3-propyl-6-(trifluoromethyl)benzothiophen-2-yl]ethyl]benzoisoxazol-6-yl]propionic acid;
(27) 2-[3-[2-[3-isopropyl-6-(trifluoromethyl)benzothiophen-2-yl]ethyl]-5-methylbenzoisoxazol-6-yloxy]propionic acid; and
(28) N-[3-[2-[3-isopropyl-6-(trifluoromethyl)benzothiophen-2-yl]ethyl]benzoisoxazol-6-yl]-N-methylglycine,
a tautomer, a stereoisomer, or a pharmaceutically acceptable salt of the compound, or a solvate of any of the foregoing.

The compounds usable for the present invention can be synthesized according to the methods described in WO03/033493, WO03/016291, WO2007/119887, and WO2009/128558.

The compounds represented by the general formula (I), (II), (III-I), or (IV-I), tautomers, stereoisomers, and pharmaceutically acceptable salts of the compounds, and solvates of any of the foregoing can exert a PPARδ agonist effect.

The pharmaceutical composition provided by the present invention can contain a compound represented by the general formula (I), (II), (III-I), or (IV-I), a tautomer, a stereoisomer, or a pharmaceutically acceptable salt of the compound, or a solvate of any of the foregoing as an active ingredient, and the content thereof can be 0.000001 to 3% by weight, preferably 0.00001 to 1% by weight, more preferably 0.0001 to 1% by weight, further preferably 0.001 to 0.5% by weight.

The pharmaceutical composition provided by the present invention may be administered in an arbitrary administration scheme. Examples of the administration scheme include, for example, parenteral administration of injection, transdermal preparation, external preparation, or the like, and oral administration of tablet, granule, or the like. Such preparations as described above can be appropriately prepared by those skilled in the art from any of the aforementioned compounds usable for the present invention alone or a combination of any of the compounds and one or two or more selected from pharmaceutically acceptable carrier, binder, stabilizer, excipient, diluent, pH adjustor, disintegrating agent, solubilizing agent, dissolving aid, lubricant, corrigent, perfume, isotonic agent, suspending agent, and the like.

The pharmaceutical composition provided by the present invention is preferably an external preparation, and it is topically administered to the skin, and more preferably directly administered to a wound part. Examples of such an external preparation as described above include cream, ointment, solution, gel, lotion, cataplasm, plaster, tape, and patch. Such external preparations as described above can also be appropriately prepared by those skilled in the art, and can be produced by appropriately using, in addition to any of the aforementioned compounds that can be used for the present invention, for example, an oily component (white petrolatum etc.), moisturizer, emulsifier, solubilizer, thickener, perfume, alcohol, and the like in combination.

Although the frequency of administration can be appropriately determined, it may be preferably administered 1 to 3 times/day, preferably once a day. For example, by one time of administration, 1 to 10 g, preferably 10 mg to 1 g, of a pharmaceutical composition comprising 0.000001 to 3% by weight, preferably 0.00001 to 1% by weight, more preferably 0.0001 to 1% by weight, further preferably 0.001 to 0.5% by weight of a compound represented by the general formula (I), (II), (III-I) or (IV-I), a tautomer, a stereoisomer, or a pharmaceutically acceptable salt of the compound, or a solvate of any of the foregoing as an active ingredient is administered.

The pharmaceutical composition provided by the present invention is useful as a therapeutic agent of wound. Examples of wound include incised wound, laceration, stab wound, bite, racoma, gun shot wound, contusion, burn, pressure ulcer, diabetic ulcer, and chemical damage.

A wound can be healed with the pharmaceutical composition provided by the present invention. The period required for healing (for example, the period of wound healing corresponding to the inflammation phase and proliferation phase of the wound healing process) may also be shortened with the pharmaceutical composition provided by the present invention. Promotion of healing and/or suppression of exacerbation of wound (for example, expansion of wound surface during the inflammation phase and/or proliferation phase) can also be attained with the pharmaceutical composition provided by the present invention.

In one embodiment of the present invention, there is provided a method for treatment of a wound, which comprises administering a compound represented by the general formula (I), (II), (III-I), or (IV-I), a tautomer, a stereoisomer, or a pharmaceutically acceptable salt of the compound, or a solvate of any of the foregoing. The object of the administration is a mammal, preferably human.

In one embodiment of the present invention, there is provided a compound represented by the general formula (I), (II), (III), or (IV-I), a tautomer, a stereoisomer, or a pharmaceutically acceptable salt of the compound, or a solvate of any of the foregoing for use in treatment of a wound.

In one embodiment of the present invention, there is provided use of a compound represented by the general formula (I), (II), (III-I), or (IV-I), a tautomer, a stereoisomer, or a pharmaceutically acceptable salt of the compound, or a solvate of any of the foregoing for manufacture of a composition for treatment of a wound.

Examples of the embodiments of the present invention include the following (1) to (4-42).
(1) A pharmaceutical composition for wound treatment, which comprises a compound represented by the following general formula (I): 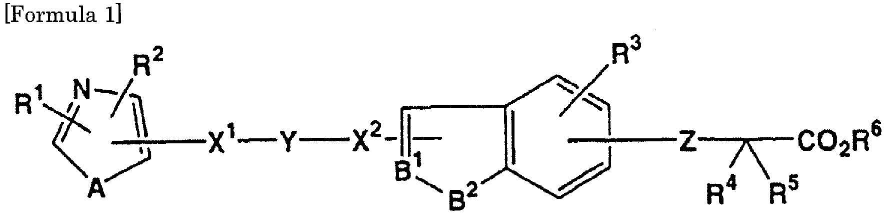 wherein
   A represents O, S, or NR⁷, wherein R⁷ represents hydrogen atom, or an alkyl group having 1 to 8 carbon atoms,
   B¹ represents CW or N, wherein W represents hydrogen atom, or an atomic bond,
   B² represents O, S, or NR⁸, wherein R⁸ represents hydrogen atom, or an alkyl group having 1 to 8 carbon atoms,
   X¹ and X² represent O, S, NH, NHC(=O), C(=O), C(=N-OR⁹), CH(OR¹⁰), C=C, C≡C, or an atomic bond, wherein R⁹ and R¹⁰ represent hydrogen atom, or an alkyl group having 1 to 8 carbon atoms,
   Y represents an alkylene chain having 1 to 8 carbon atoms which may have an alkyl group having 1 to 8 carbon atoms, or an alkyl group having 1 to 8 carbon atoms and substituted with 1 to 3 halogen atoms as a substituent,
   Z represents NH, O, or S,
   R¹ represents an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent, or a heterocyclic group having a 5- to 8-membered ring comprising 1 to 3 heteroatoms selected from nitrogen atom, oxygen atom, and sulfur atom, and the remainder carbon atoms as ring-constituting atoms (benzene ring may further condense to this heterocyclic ring),
   R² represents an alkyl group having 2 to 8 carbon atoms, an alkyl group having 1 to 8 carbon atoms and substituted with 1 to 3 halogen atoms, a cycloalkyl group having 3 to 7 carbon atoms, an alkenyl group having 2 to 8 carbon atoms, an alkynyl group having 2 to 8 carbon atoms, an alkyl group substituted with an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent (the alkyl moiety thereof has 1 to 4 carbon atoms), or an alkyl group substituted with a 5- to 8-membered heterocyclic ring (the heterocyclic ring thereof comprises 1 to 3 heteroatoms selected from nitrogen atom, oxygen atom, and sulfur atom, and the remainder carbon atoms as ring-constituting atoms, and the alkyl moiety thereof has 1 to 4 carbon atoms),
   R³ represents hydrogen atom, a halogen atom, trifluoromethyl group, an alkyl group having 1 to 8 carbon atoms, an alkenyl group having 2 to 8 carbon atoms, or an alkynyl group having 2 to 8 carbon atoms,
   R⁴ and R⁵ represent hydrogen atom, an alkyl group having 1 to 8 carbon atoms, or an alkyl group having 1 to 8 carbon atoms and substituted with 1 to 3 halogen atoms, and
   R⁶ represents hydrogen atom, an alkyl group having 1 to 8 carbon atoms and substituted with an amino group, an alkyl group having 1 to 8 carbon atoms, or an alkali metal,
   provided that Z and R³ bond to the benzene ring, and X² does not bond to the benzene ring, a tautomer, a stereoisomer, or a pharmaceutically acceptable salt of the compound, or a solvate of any of the foregoing.
(2) The pharmaceutical composition according to (1), which comprises a compound represented by the general formula (I), a tautomer, a stereoisomer, or a pharmaceutically acceptable salt of the compound, or a solvate of any of the foregoing, wherein B¹ is N, and B² is O.
(3) The pharmaceutical composition according to (1) or (2), which comprises a compound represented by the general formula (I), a tautomer, a stereoisomer, or a pharmaceutically acceptable salt of the compound, or a solvate of any of the foregoing, wherein A is S.
(4) The pharmaceutical composition according to (1) or (2), which comprises a compound represented by the general formula (I), a tautomer, a stereoisomer, or a pharmaceutically acceptable salt of the compound, or a solvate of any of the foregoing, wherein A is O.
(5) The pharmaceutical composition according to any one of (1) to (4), which comprises a compound represented by the general formula (I), a tautomer, a stereoisomer, or a pharmaceutically acceptable salt of the compound, or a solvate of any of the foregoing, wherein Z is O.
(6) The pharmaceutical composition according to any one of (1) to (4), which comprises a compound represented by the general formula (I), a tautomer, a stereoisomer, or a pharmaceutically acceptable salt of the compound, or a solvate of any of the foregoing, wherein Z is NH.
(7) The pharmaceutical composition according to any one of (1) to (4), which comprises a compound represented by the general formula (I), a tautomer, a stereoisomer, or a pharmaceutically acceptable salt of the compound, or a solvate of any of the foregoing, wherein Z is S.
(8) The pharmaceutical composition according to any one of (1) to (7), which comprises a compound represented by the general formula (I), a tautomer, a stereoisomer, or a pharmaceutically acceptable salt of the compound, or a solvate of any of the foregoing, wherein R¹ is a phenyl group which may have an alkyl group substituted with 1 to 3 halogen atoms.
(9) The pharmaceutical composition according to any one of (1) to (8), which comprises a compound represented by the general formula (I), a tautomer, a stereoisomer, or a pharmaceutically acceptable salt of the compound, or a solvate of any of the foregoing, wherein X¹ and X² are atomic bonds, and Y is ethylene group.
(10) The pharmaceutical composition according to any one of (1) to (9), which comprises a compound represented by the general formula (I), a tautomer, a stereoisomer, or a pharmaceutically acceptable salt of the compound, or a solvate of any of the foregoing, wherein R² is isopropyl group.
(11) The pharmaceutical composition according to any one of (1) to (10), which comprises a compound represented by the general formula (I), a tautomer, a stereoisomer, or a pharmaceutically acceptable salt of the compound, or a solvate of any of the foregoing, wherein R³ is an alkyl group having 1 to 3 carbon atoms.
(12) The pharmaceutical composition according to any one of (1) to (11), which comprises a compound represented by the general formula (I), a tautomer, a stereoisomer, or a pharmaceutically acceptable salt of the compound, or a solvate of any of the foregoing, wherein R⁴ and R⁵ are hydrogen atoms.
(13) The pharmaceutical composition according to any one of (1) to (12), which comprises a compound represented by the general formula (I), a tautomer, a stereoisomer, or a pharmaceutically acceptable salt of the compound, or a solvate of any of the foregoing, wherein R⁶ is hydrogen atom.
(14) A pharmaceutical composition for wound treatment, which comprises any one of the compounds of (a) to (j) described below:
   (a) [3-[2-[4-isopropyl-2-(4-trifluoromethyl)phenyl-5-thiazolyl]ethyl]-5-methyl-1,2-benzisoxazol-6-yl]oxyacetic acid;
   (b) 2-[[3-[2-[4-isopropyl-2-(4-trifluoromethyl)phenyl-5-thiazolyl]ethyl]-5-methyl-1,2-benzisoxazol-6-yl]oxy]-2-methylpropionic acid;
   (c) [3-[2-[4-isopropyl-2-(4-trifluoromethyl)phenyl-5-thiazolyl]ethyl]-5-methyl-1,2-benzisoxazol-6-yl]thioacetic acid;
   (d) [3-[2-[4-isopropyl-2-(4-trifluoromethyl)phenyl-5-thiazolyl]ethyl]-5-methyl-1,2-benzisoxazol-6-yl]aminoacetic acid;
   (e) [3-[2-[2-(2,4-dichlorophenyl)-5-isopropyl-4-oxazolyl]ethyl]-5-methyl-1,2-benzisoxazol-6-yl]oxyacetic acid;
   (f) 2-[[3-[2-[4-isopropyl-2-(4-trifluoromethyl)phenyl-5-thiazolyl]ethyl]-5-methyl-1,2-benzisoxazol-6-yl]oxy]-2-methylpropionic acid 2-piperidinoethyl ester hydrochloride;
   (g) [[7-allyl-3-[2-[2-(2,4-dichlorophenyl)-5-isopropyl-4-oxazolyl]ethyl]-1,2-benzisoxazol-6-yl]oxy]acetic acid;
   (h) 2-[[7-allyl-3-[2-[2-(2,4-dichlorophenyl)-5-isopropyl-4-oxazolyl]ethyl]-1,2-benzisoxazol-6-yl]oxy]-2-methylpropionic acid;
   (i) [[7-propyl-3-[2- [2-(2,4-dichlorophenyl)-5-isopropyl-4-oxazolyl]ethyl]- 1,2-benzisoxazol-6-yl]oxy]acetic acid; and
   (j) 2-[[7-allyl-3-[2-[2-[(4-trifluoromethyl)phenyl]-4-isopropyl-5-thiazolyl]ethyl]-1,2-benzisoxazol-6-yl]oxy]-2-methylpropionic acid,
   a tautomer, a stereoisomer, or a pharmaceutically acceptable salt of the compound, or a solvate of any of the foregoing.
(15) A pharmaceutical composition for wound treatment, which comprises a compound represented by the following general formula (II): 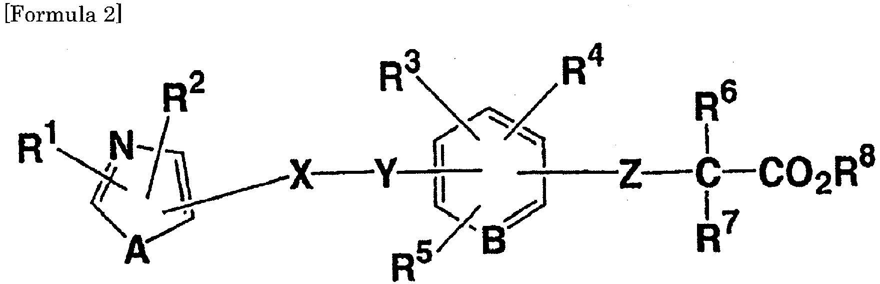
   wherein R¹ represents a phenyl group, naphthyl group, pyridyl group, thienyl group, furyl group, quinolyl group, or benzothienyl group which may have a group or an atom selected from an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent, R² represents an alkyl group having 1 to 8 carbon atoms, an alkyl group having 1 to 8 carbon atoms and substituted with a halogen atom, an alkenyl group having 2 to 8 carbon atoms, an alkynyl group having 2 to 8 carbon atoms, a cycloalkyl group having a 3- to 7-membered ring, an alkyl group having 1 to 8 carbon atoms and substituted with a cycloalkyl group having a 3- to 7-membered ring, or a phenyl group which may have a group or an atom selected from an alkoxy group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkynyl group having 2 to 8 carbon atoms, a halogen atom, an acyl group having 2 to 7 carbon atoms, a benzoyl group, a hydroxyl group, a nitro group, an amino group, a phenyl group, and a pyridyl group, or a naphthyl group, or an alkyl group having 1 to 6 carbon atoms and substituted with a pyridyl group, A represents oxygen atom, sulfur atom, or NR⁹, wherein R⁹ represents hydrogen atom, or an alkyl group having 1 to 8 carbon atoms, X represents an alkylene chain having 1 to 8 carbon atoms which may have a group selected from an alkyl group having 1 to 8 carbon atoms, an alkoxy group having 1 to 8 carbon atoms, and hydroxyl group as a substituent, and may contain a double bond, Y represents C(=O), C(=NOR¹⁰), CH(OR¹¹), CH=CH, C≡C, or C(=CH₂), wherein R¹⁰ and R¹¹ represent hydrogen atom, an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, or pyridyl group, B represents CH or nitrogen atom, Z represents oxygen atom or sulfur atom, R⁶ and R⁷ represent hydrogen atom, an alkyl group having 1 to 8 carbon atoms, or an alkyl group having 1 to 8 carbon atoms and substituted with a halogen atom, and R⁸ represents hydrogen atom, or an alkyl group having 1 to 8 carbon atoms,
   provided that at least one of R³, R⁴, and R⁵ is not hydrogen atom, a tautomer, a stereoisomer, or a pharmaceutically acceptable salt of the compound, or a solvate of any of the foregoing.
(16) The pharmaceutical composition according to (15), which comprises a compound represented by the general formula (II), a tautomer, a stereoisomer, or a pharmaceutically acceptable salt of the compound, or a solvate of any of the foregoing, wherein R¹ is a phenyl group which may have a group or an atom selected from an alkyl group having 1 to 8 carbon atoms, an alkyl group having 1 to 8 carbon atoms and substituted with 1 to 3 halogen atoms, an alkoxy group having 1 to 8 carbon atoms, an alkoxy group having 1 to 8 carbon atoms and substituted with 1 to 3 halogen atoms, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent.
(17) The pharmaceutical composition according to (15) or (16), which comprises a compound represented by the general formula (II), a tautomer, a stereoisomer, or a pharmaceutically acceptable salt of the compound, or a solvate of any of the foregoing, wherein R² is an alkyl group having 2 to 8 carbon atoms as a substituent.
(18) The pharmaceutical composition according to any one of (15) to (17), which comprises a compound represented by the general formula (II), a tautomer, a stereoisomer, or a pharmaceutically acceptable salt of the compound, or a solvate of any of the foregoing, wherein the substitution position of R¹ is the 2-position.
(19) The pharmaceutical composition according to any one of (15) to (18), which comprises a compound represented by the general formula (II), a tautomer, a stereoisomer, or a pharmaceutically acceptable salt of the compound, or a solvate of any of the foregoing, wherein A is oxygen atom or sulfur atom.
(20) The pharmaceutical composition according to any one of (15) to (19), which comprises a compound represented by the general formula (II), a tautomer, a stereoisomer, or a pharmaceutically acceptable salt of the compound, or a solvate of any of the foregoing, wherein X is an alkylene chain having 1 to 8 carbon atoms.
(21) The pharmaceutical composition according to any one of (15) to (20), which comprises a compound represented by the general formula (II), a tautomer, a stereoisomer, or a pharmaceutically acceptable salt of the compound, or a solvate of any of the foregoing, wherein Y is C(=O).
(22) The pharmaceutical composition according to any one of (15) to (21), which comprises a compound represented by the general formula (II), a tautomer, a stereoisomer, or a pharmaceutically acceptable salt of the compound, or a solvate of any of the foregoing, wherein R³, R⁴, and R⁵ are hydrogen atom, an alkyl group having 1 to 8 carbon atoms, or an alkyl group having 1 to 8 carbon atoms and substituted with a halogen atom.
(23) The pharmaceutical composition according to any one of (15) to (22), which comprises a compound represented by the general formula (II), a tautomer, a stereoisomer, or a pharmaceutically acceptable salt of the compound, or a solvate of any of the foregoing, wherein B is CH.
(24) The pharmaceutical composition according to any one of (15) to (23), which comprises a compound represented by the general formula (II), a tautomer, a stereoisomer, or a pharmaceutically acceptable salt of the compound, or a solvate of any of the foregoing, wherein Z is oxygen atom.
(25) The pharmaceutical composition according to any one of (15) to (24), which comprises a compound represented by the general formula (II), a tautomer, a stereoisomer, or a pharmaceutically acceptable salt of the compound, or a solvate of any of the foregoing, wherein R⁶ and R⁷ are hydrogen atom, or an alkyl group having 1 to 4 carbon atoms.
(26) The pharmaceutical composition according to any one of (15) to (25), which comprises a compound represented by the general formula (II), a tautomer, a stereoisomer, or a pharmaceutically acceptable salt of the compound, or a solvate of any of the foregoing, wherein R⁸ is hydrogen atom.
(27) The pharmaceutical composition according to (15), which comprises a compound represented by the general formula (II), a tautomer, a stereoisomer, or a pharmaceutically acceptable salt of the compound, or a solvate of any of the foregoing, wherein R¹ is a phenyl group or naphthyl group which may have a group or an atom selected from an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent, R² is an alkyl group having 2 to 8 carbon atoms, A is oxygen atom or sulfur atom, X is an alkylene chain having 1 to 8 carbon atoms which may have an alkyl group having 1 to 8 carbon atoms as a substituent, and may contain a double bond, Y is C(=O), CH=CH, or C(=CH₂), R³, R⁴, and R⁵ are hydrogen atom, an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, or pyridyl group, B is CH, Z is oxygen atom or sulfur atom, R⁶ and R⁷ are hydrogen atom, or an alkyl group having 1 to 8 carbon atoms, and R⁸ is hydrogen atom, or an alkyl group having 1 to 8 carbon atoms.
(28) The pharmaceutical composition according to any one of (27), which comprises a compound represented by the general formula (II), a tautomer, a stereoisomer, or a pharmaceutically acceptable salt of the compound, or a solvate of any of the foregoing, wherein X is an alkylene chain having 1 to 8 carbon atoms.
(29) The pharmaceutical composition according to (27) or (28), which comprises a compound represented by the general formula (II), a tautomer, a stereoisomer, or a pharmaceutically acceptable salt of the compound, or a solvate of any of the foregoing, wherein the substitution position of R¹ is the 2-position.
(30) The pharmaceutical composition according to any one of (27) to (29), which comprises a compound represented by the general formula (II), a tautomer, a stereoisomer, or a pharmaceutically acceptable salt of the compound, or a solvate of any of the foregoing, wherein R⁸ is hydrogen atom.
(31) The pharmaceutical composition according to any one of (27) to (30), which comprises a compound represented by the general formula (II), a tautomer, a stereoisomer, or a pharmaceutically acceptable salt of the compound, or a solvate of any of the foregoing, wherein a substituent other than hydrogen atom as R³, R⁴, or R⁵ substitutes at the ortho-position of -Z-CR⁶R⁷CO₂R⁸.
(32) A pharmaceutical composition for wound treatment, which comprises any one of the compounds of 1) to 50) described below:
   1) 2-[4-[3-[2-(2,4-dichlorophenyl) 5-isopropyl-4-oxazolyl]propionyl]-2-methylphenoxy]-2-methylpropionic acid;
   2) [4-[3-[2-(2,4-dichlorophenyl)-5-isopropyl-4-oxazolyl]propionyl]-2-methylphenoxy]acetic acid;
   3) [4-[3-[2-(4-trifluoromethyl)phenyl-4-isopropyl-5-thiazolyl]propionyl]-2-methylphenoxy]acetic acid;
   4) 2-[4-[3-[2-(4-trifluoromethyl)phenyl-4-isopropyl-5-thiazolyl]propionyl]-2-methylphenoxy]-2-methylpropionic acid;
   5) [2-allyl-4-[3-[2-(2,4-dichlorophenyl)-5-isopropyl-4-oxazolyl]propionyl]phenoxy]acetic acid;
   6) [4-[3-[2-(2-hydroxy-4-chlorophenyl)-5-isopropyl-4-oxazolyl]propionyl]-2-methylphenoxy]acetic acid;
   7) [4-[3-[2-(2,4-dichlorophenyl)-5-isopropyl-4-oxazolyl]propionyl]-2-methylphenylsulfanyl]acetic acid;
   8) 2-[4-[3-[2-(2-hydroxy-4-chlorophenyl)-5-isopropyl-4-oxazolyl]propionyl]-2-methylphenoxy]-2-methylprop ionic acid;
   9) [4-[3-[2-(2,4-dichlorophenyl)-5-isopropyl-4-oxazolyl]-1-propenyl]-2-methylphenoxy]acetic acid;
   10) [4-[3-[2-(2,4-dichlorophenyl)-5-isopropyl-4-oxazolyl]-1-propenyl]-2-methylphenoxy]acetic acid;
   11) [4-[3-[4-hexyl-2-(4-trifluoromethyl)phenyl-5-thiazolyl]propionyl]-2-methylphenoxy]acetic acid;
   12) 2-[4-[3-[4-hexyl-2-(4-trifluoromethyl)phenyl-5-thiazolyl]propionyl]-2-methylphenoxy]-2-methylpropionic acid;
   13) 2-[4-[3-[4-isopropyl-2-(4-trifluoromethyl)phenyl-5-thiazolyl]-1-propenyl]-2-methylphenoxy]-2-methylpropionic acid;
   14) [4-[3-[4-isopropyl-2-(4-trifluoromethyl)phenyl-5-thiazolyl]propionyl]-3-methylphenoxy]acetic acid;
   15) [4-[3-[2-(2,4-dichlorophenyl)-5-isopropyl-4-oxazolyl]propionyl]-3-methylphenoxy]acetic acid;
   16) [4-[3-[4-isopropyl-2-(4-trifluoromethyl)phenyl-5-thiazolyl]propionyl]-3-methylphenoxy]-2-methylpropionic acid;
   17) 2-[4-[3-[2-(2,4-dichlorophenyl)-5-isopropyl-4-oxazolyl]propionyl]-3-methylphenoxy]-2-methylpropionic acid;
   18) [4-[3-[4-isopropyl-2-(4-trifluoromethyl)phenyl-5-thiazolyl]propionyl]-2-propylphenoxy]acetic acid;
   19) 2-allyl-4-[3-[4-isopropyl-2-(4-trifluoromethyl)phenyl-5-thiazolyl]propionyl]phenoxyacetic acid;
   20) [4-[4-[2-(2,4-dichlorophenyl)-5-isopropyl-4-oxazolyl]-1-buten-2-yl]-2-methylphenoxy]acetic acid;
   21) 2-[4-[4-[2-(2,4-dichlorophenyl)-5-isopropyl-4-oxazolyl]-1-buten-2-yl-2-methylpropionic acid;
   22) [4-[3-[2-(2,4-dichlorophenyl)-5-isopropyl-4-oxazolyl]-2-methylpropionyl]-2-methylphenoxy]acetic acid;
   23) 2-[4-[3-[2-(2,4-dichlorophenyl)-5-isopropyl-4-oxazolyl]-2-methylpropionyl]-2-methylphenoxy]-2-methylpropionic acid;
   24) [4-[3-[4-isopropyl-2-(4-trifluoromethyl)phenyl-5-thiazolyl]propenoyl]-2-methylphenoxy]acetic acid;
   25) 2-[4-[3-[4-isopropyl-2-(4-trifluoromethyl)phenyl-5-thiazolyl]propenoyl]-2-methylphenoxy]-2-methylpropionic acid;
   26) [4-[3-[4-isopropyl-2-(4-methoxyphenyl)-5-thiazolyl]propionyl]-2-methylphenoxy]propionic acid;
   27) [4-[3-[2-(3,5-dichlorophenyl)-4-isopropylthiazol-5-yl]propionyl]-2-methylphenoxy]acetic acid;
   28) 2-[4-[3-[2-(3,5-difluorophenyl)-4-isopropyl-5-thiazolyl]propionyl]-2-methylphenoxy]-2-methylpropionic acid;
   29) [4-[3-[4-isopropyl-2-(2-naphthyl)-5-thiazolyl]propionyl]-2-methylphenoxyl]acetic acid;
   30) 2-[4-[3-[4-isopropyl-2-(2-naphthyl)-5-thiazolyl]propionyl]-2-methylphenoxy]-2-methylpropionic acid;
   31) [4-[3-[2-(4-butylphenyl)-4-isopropyl-5-thiazolyl]propionyl]-2-methylphenoxy]acetic acid;
   32) 2-[4-[3-[2-(4-butylphenyl)-4-isopropyl-5-thiazolyl]propionyl]-2-methylphenoxy]-2-methylpropionic acid;
   33) [4-[3-[4-isopropyl-2-(4-trifluoromethyl)phenyl-5-thiazolyl]propionyl]-2-chlorophenoxy]acetic acid;
   34) [4-[3-[2-(4-trifluoromethyl)phenyl-4-isopropyl-5-thiazolyl]propionyl]-2-chlorophenoxy]-2-methylprop ionic acid;
   35) [4-[3-[2-(2,4-dichlorophenyl)-5-isopropyl-1-4-oxazolyl]propionyl]-2-chlorophenoxy]acetic acid;
   36) 2-[4-[3-[2-(2,4-dichlorophenyl)-5-isopropyl-4-oxazolyl]propionyl]-2-chlorophenoxy]-2-methylpropionic acid;
   37) [4-[3-[5-isopropyl-2-(4-trifluoromethyl)phenyl-4-thiazolyl]propionyl]-2-methylphenoxy]acetic acid;
   38) 2-[4-[3-[5-isopropyl-2-(4-trifluoromethyl)phenyl-4-thiazolyl]propionyl]-2-methylphenoxy]-2-methylprop ionic acid;
   39) [4-[3-[2-(2,4-dichlorophenyl)-5-isopropyl-4-thiazolyl]propionyl]-2-methylphenoxy]acetic acid;
   40) 2-[4-[3-[2-(2,4-dichlorophenyl)-5-isopropyl-4-thiazolyl]propionyl]-2-methylphenoxy]-2-methylpropionic acid;
   41) [5-[3-[4-isopropyl-2-(4-trifluoromethyl)phenyl-5-thiazolyl]propionyl]-2-methylphenoxy]acetic acid;
   42) 2-[5-[3-[4-isopropyl-2-(4-trifluoromethyl)phenyl-5-thiazolyl]propionyl]-2-methylphenoxy]-2-methylpropionic acid;
   43) 2-[4-[3-[4-isopropyl-2-(4-trifluoromethyl)phenyl-5-thiazolyl]propionyl]-2-methylphenoxy]propionic acid;
   44) 4-[3-[4-methyl-2-(4-trifluoromethyl)phenyl-5-thiazolyl]propionyl]-2-methylphenoxy]acetic acid;
   45) 2-[4-[3-[4-hexyl-2-(4-trifluoromethyl)phenyl-5-thiazolyl]-1-propenyl]-2-methylphenoxy]-2-methylpropionic acid;
   46) 2-[5-[3-[4-hexyl-2-(4-trifluoromethyl)phenyl-5-thiazolyl]propionyl]-2-methylphenoxy]-2-methylpropionic acid;
   47) [4-[3-[4-ethyl-2-(4-trifluoromethyl)phenyl-5-thiazolyl]propionyl]-2-methylphenoxy] acetic acid;
   48) 2-[4-[3-[4-ethyl-2-(4-trifluoromethyl)phenyl-5-thiazolyl]propionyl]-2-methylphenoxy]-2-methylpropionic acid;
   49) [4-[3-[4-isopropyl-2-(4-methylphenyl)-5-thiazolyl]propionyl]-2-methylphenoxy]acetic acid; and
   50) 2-[4-[3-[4-isopropyl-2-(4-methylphenyl)-5-thiazolyl]propionyl]-2-methylphenoxy]-2-methylpropionic acid,
   a tautomer, a stereoisomer, or a pharmaceutically acceptable salt of the compound, or a solvate of any of the foregoinga.
(33) The pharmaceutical composition according to any one of (1) to (32), which is for topical administration to the skin.
(34) The pharmaceutical composition according to any one of (1) to (33), which promotes wound healing (for example, promotes wound healing during the inflammation phase and proliferation phase in the wound healing process).
(35) The pharmaceutical composition according to any one of (1) to (34), which suppresses exacerbation of wound (for example, expansion of wound surface during the inflammation phase and/or proliferation phase).
(36) The pharmaceutical composition according to any one of (1) to (35), which suppresses aggravation of wound surface and/or expansion of wound surface caused by exudate.
(37) The pharmaceutical composition according to any one of (1) to (36), which is for treatment of pressure ulcer or diabetic ulcer.
(38) A pharmaceutical composition for promoting wound healing (for example, a pharmaceutical composition for promoting wound healing during the inflammation phase and proliferation phase in the wound healing process), which comprises the compound, a tautomer, a stereoisomer, or a pharmaceutically acceptable salt of the compound, or a solvate of any of the foregoing described in any one of (1) to (32).
(39) A pharmaceutical composition for suppressing exacerbation of wound (for example, a pharmaceutical composition for suppressing expansion of wound surface during the inflammation phase and/or proliferation phase), which comprises the compound, a tautomer, a stereoisomer, or a pharmaceutically acceptable salt of the compound, or a solvate of any of the foregoing described in any one of (1) to (32).
(40) A pharmaceutical composition for suppressing aggravation of wound surface and/or expansion of wound surface caused by exudate, which comprises the compound, a tautomer, a stereoisomer, or a pharmaceutically acceptable salt of the compound, or a solvate of any of the foregoing pharmaceutical composition described in any one of (1) to (32).
(41) The pharmaceutical composition according to any one of (37) to (40), which is a pharmaceutical composition for topical administration to the skin.
(42) The pharmaceutical composition according to any one of (37) to (41), which is a pharmaceutical composition for treatment of pressure ulcer or diabetic ulcer.

(2-1) A compound represented by the following general formula (I), a tautomer, a stereoisomer, or a pharmaceutically acceptable salt of the compound, or a solvate of any of the foregoing: 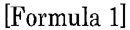 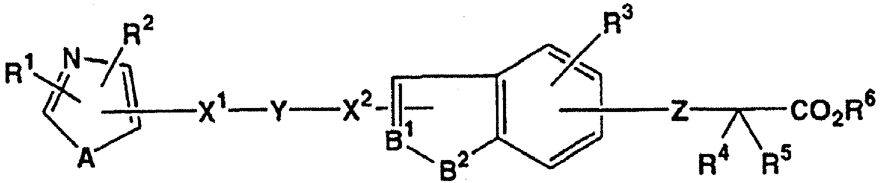 wherein, in the formula,
   A represents O, S, or NR⁷, wherein R⁷ represents hydrogen atom, or an alkyl group having 1 to 8 carbon atoms,
   represents CW or N, wherein W represents hydrogen atom, or an atomic bond,
   B² represents O, S, or NR⁸, wherein R⁸ represents hydrogen atom, or an alkyl group having 1 to 8 carbon atoms,
   X¹ and X² represent O, S, NH, NHC(=O), C(=O), C(=N-OR⁹), CH(OR¹⁰), C=C, C≡C, or an atomic bond, wherein R⁹ and R¹⁰ represent hydrogen atom, or an alkyl group having 1 to 8 carbon atoms,
   Y represents an alkylene chain having 1 to 8 carbon atoms which may have an alkyl group having 1 to 8 carbon atoms, or an alkyl group having 1 to 8 carbon atoms and substituted with 1 to 3 halogen atoms as a substituent,
   Z represents NH, O, or S,
   R¹ represents an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent, or a heterocyclic group having a 5- to 8-membered ring comprising 1 to 3 heteroatoms selected from nitrogen atom, oxygen atom, and sulfur atom, and the remainder carbon atoms as ring-constituting atoms (benzene ring may further condense to this heterocyclic ring),
   R² represents an alkyl group having 2 to 8 carbon atoms, an alkyl group having 1 to 8 carbon atoms and substituted with 1 to 3 halogen atoms, a cycloalkyl group having 3 to 7 carbon atoms, an alkenyl group having 2 to 8 carbon atoms, an alkynyl group having 2 to 8 carbon atoms, an alkyl group substituted with an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent (the alkyl moiety thereof has 1 to 4 carbon atoms), or an alkyl group substituted with a 5- to 8-membered heterocyclic ring (the heterocyclic ring thereof comprises 1 to 3 heteroatoms selected from nitrogen atom, oxygen atom, and sulfur atom, and the remainder carbon atoms as ring-constituting atoms, and the alkyl moiety thereof has 1 to 4 carbon atoms),
   R³ represents hydrogen atom, a halogen atom, trifluoromethyl group, an alkyl group having 1 to 8 carbon atoms, an alkenyl group having 2 to 8 carbon atoms, or an alkynyl group having 2 to 8 carbon atoms,
   R⁴ and R⁵ represent hydrogen atom, an alkyl group having 1 to 8 carbon atoms, or an alkyl group having 1 to 8 carbon atoms and substituted with 1 to 3 halogen atoms, and
   R⁶ represents hydrogen atom, an alkyl group having 1 to 8 carbon atoms and substituted with an amino group, an alkyl group having 1 to 8 carbon atoms, or an alkali metal,
   provided that Z and R³ bond to the benzene ring, and X² does not bond to the benzene ring,
   for use in the treatment of wound.
(2-2) The compound, a tautomer, a stereoisomer, or a pharmaceutically acceptable salt of the compound, or a solvate of any of the foregoing according to (2-1) for the use according to (2-1), which is a compound represented by the general formula (I), a tautomer, a stereoisomer, or a pharmaceutically acceptable salt of the compound, or a solvate of any of the foregoing, wherein is N, and B² is O.
(2-3) The compound, a tautomer, a stereoisomer, or a pharmaceutically acceptable salt of the compound, or a solvate of any of the foregoing according to (2-1) or (2-2) for the use according to (2-1) or (2-2), which is a compound represented by the general formula (I), a tautomer, a stereoisomer, or a pharmaceutically acceptable salt of the compound, or a solvate of any of the foregoing, wherein A is S.
(2-4) The compound, a tautomer, a stereoisomer, or a pharmaceutically acceptable salt of the compound, or a solvate of any of the foregoing according to (2-1) or (2-2) for the use according to (2-1) or (2-2), which is a compound represented by the general formula (I), a tautomer, a stereoisomer, or a pharmaceutically acceptable salt of the compound, or a solvate of any of the foregoing, wherein A is O.
(2-5) The compound, a tautomer, a stereoisomer, or a pharmaceutically acceptable salt of the compound, or a solvate of any of the foregoing according to any one of (2-1) to (2-4) for the use according to any one of (2-1) to (2-4), which is a compound represented by the general formula (I), a tautomer, a stereoisomer, or a pharmaceutically acceptable salt of the compound, or a solvate of any of the foregoing, wherein Z is O.
(2-6) The compound, a tautomer, a stereoisomer, or a pharmaceutically acceptable salt of the compound, or a solvate of any of the foregoing according to any one of (2-1) to (2-4) for the use according to any one of (2-1) to (2-4), which is a compound represented by the general formula (I), a tautomer, a stereoisomer, or a pharmaceutically acceptable salt of the compound, or a solvate of any of the foregoing, wherein Z is NH.
(2-7) The compound, a tautomer, a stereoisomer, or a pharmaceutically acceptable salt of the compound, or a solvate of any of the foregoing according to any one of (2-1) to (2-4) for the use according to any one of (2-1) to (2-4), which is a compound represented by the general formula (I), a tautomer, a stereoisomer, or a pharmaceutically acceptable salt of the compound, or a solvate of any of the foregoing, wherein Z is S.
(2-8) The compound, a tautomer, a stereoisomer, or a pharmaceutically acceptable salt of the compound, or a solvate of any of the foregoing according to any one of (2-1) to (2-7) for the use according to any one of (2-1) to (2-7), which is a compound represented by the general formula (I), a tautomer, a stereoisomer, or a pharmaceutically acceptable salt of the compound, or a solvate of any of the foregoing, wherein R¹ is a phenyl group which may have an alkyl group substituted with 1 to 3 halogen atoms.
(2-9) The compound, a tautomer, a stereoisomer, or a pharmaceutically acceptable salt of the compound, or a solvate of any of the foregoing according to any one of (2-1) to (2-8) for the use according to any one of (2-1) to (2-8), which is a compound represented by the general formula (I), a tautomer, a stereoisomer, or a pharmaceutically acceptable salt of the compound, or a solvate of any of the foregoing, wherein X¹ and X² are atomic bonds, and Y is ethylene group.
(2-10) The compound, a tautomer, a stereoisomer, or a pharmaceutically acceptable salt of the compound, or a solvate of any of the foregoing according to any one of (2-1) to (2-9) for the use according to any one of (2-1) to (2-9), which is a compound represented by the general formula (I), a tautomer, a stereoisomer, or a pharmaceutically acceptable salt of the compound, or a solvate of any of the foregoing, wherein R² is isopropyl group.
(2-11) The compound, a tautomer, a stereoisomer, or a pharmaceutically acceptable salt of the compound, or a solvate of any of the foregoing according to any one of (2-1) to (2-10) for the use according to any one of (2-1) to (2-10), which is a compound represented by the general formula (I), a tautomer, a stereoisomer, or a pharmaceutically acceptable salt of the compound, or a solvate of any of the foregoing, wherein R³ is an alkyl group having 1 to 3 carbon atoms.
(2-12) The compound, a tautomer, a stereoisomer, or a pharmaceutically acceptable salt of the compound, or a solvate of any of the foregoing according to any one of (2-1) to (2-11) for the use according to any one of (2-1) to (2-11), which is a compound represented by the general formula (I), a tautomer, a stereoisomer, or a pharmaceutically acceptable salt of the compound, or a solvate of any of the foregoing, wherein R⁴ and R⁵ are hydrogen atoms.
(2-13) The compound, a tautomer, a stereoisomer, or a pharmaceutically acceptable salt of the compound, or a solvate of any of the foregoing according to any one of (2-1) to (2-12) for the use according to any one of (2-1) to (2-12), which is a compound represented by the general formula (I), a tautomer, a stereoisomer, or a pharmaceutically acceptable salt of the compound, or a solvate of any of the foregoing, wherein R⁶ is hydrogen atom.
(2-14) A compound for use in treatment of wound, which is any one of the compounds of (a) to (j) described below:
   (a) [3-[2-[4-isopropyl-2-(4-trifluoromethyl)phenyl-5-thiazolyl]ethyl]-5-methyl-1,2-benzisoxazol-6-yl]oxyacetic acid;
   (b) 2-[[3-[2-[4-isopropyl-2-(4-trifluoromethyl)phenyl-5-thiazolyl]ethyl]-5-methyl-1,2-benzisoxazol-6-yl]oxy]-2-methylpropionic acid;
   (c) [3-[2-[4-isopropyl-2-(4-trifluoromethyl)phenyl-5-thiazolyl]ethyl]-5-methyl-1,2-benzisoxazol-6-yl]thioacetic acid;
   (d) [3-[2-[4-isopropyl-2-(4-trifluoromethyl)phenyl-5-thiazolyl]ethyl]-5-methyl-1,2-benzisoxazol-6-yl]aminoacetic acid;
   (e) [3-[2-[2-(2,4-dichlorophenyl)-5-isopropyl-4-oxazolyl]ethyl]-5-methyl-1,2-benzisoxazol-6-yl]oxyacetic acid;
   (f) 2-[[3-[2-[4-isopropyl-2-(4-trifluoromethyl)phenyl-5-thiazolyl]ethyl]-5-methyl-1,2-benzisoxazol-6-yl]oxy]-2-methylpropionic acid 2-piperidinoethyl ester hydrochloride;
   (g) [[7-allyl-3-[2-[2-(2,4-dichlorophenyl)-5-isopropyl-4-oxazolyl]ethyl]-1,2-benzisoxazol-6-yl]oxy]acetic acid;
   (h) 2-[[7-allyl-3-[2-[2-(2,4-dichlorophenyl)-5-isopropyl-4-oxazolyl]ethyl]-1,2-benzisoxazol-6-yl]oxy]-2-methylpropionic acid;
   (i) [[7-propyl-3-[2-[2-(2,4-dichlorophenyl)-5-isopropyl-4-oxazolyl]ethyl]-1,2-benzisoxazol-6-yl]oxy]acetic acid; and
   (j) 2-[[7-allyl-3-[2-[2-[(4-trifluoromethyl)phenyl]-4-isopropyl-5-thiazolyl]ethyl]-1,2-benzisoxazol-6-yl]oxy]-2-methylpropionic acid,
   a tautomer, a stereoisomer, or a pharmaceutically acceptable salt of the compound, or a solvate of any of the foregoing.
(2-15) A compound represented by the following general formula (II), a tautomer, a stereoisomer, or a pharmaceutically acceptable salt of the compound, or a solvate of any of the foregoing: 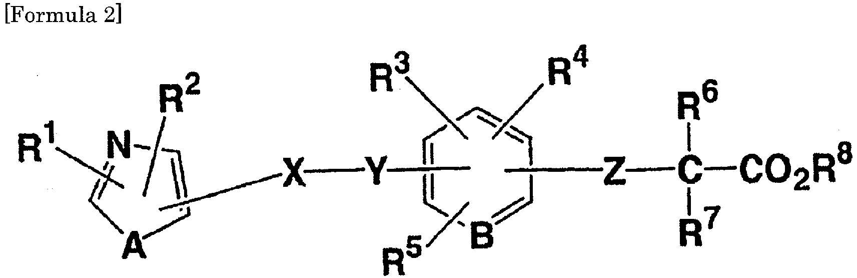
   wherein, in the formula, R¹ represents a phenyl group, naphthyl group, pyridyl group, thienyl group, furyl group, quinolyl group, or benzothienyl group which may have a group or an atom selected from an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent, R² represents an alkyl group having 1 to 8 carbon atoms, an alkyl group having 1 to 8 carbon atoms and substituted with a halogen atom, an alkenyl group having 2 to 8 carbon atoms, an alkynyl group having 2 to 8 carbon atoms, a cycloalkyl group having a 3- to 7-membered ring, an alkyl group having 1 to 8 carbon atoms and substituted with a cycloalkyl group having a 3- to 7-membered ring, a phenyl group, which may have a group or an atom selected from an alkoxy group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkynyl group having 2 to 8 carbon atoms, a halogen atom, an acyl group having 2 to 7 carbon atoms, a benzoyl group, a hydroxyl group, a nitro group, an amino group, a phenyl group, and a pyridyl group, a naphthyl group, or an alkyl group having 1 to 6 carbon atoms and substituted with apyridyl group, A represents oxygen atom, sulfur atom, or NR⁹, wherein R⁹ represents hydrogen atom, or an alkyl group having 1 to 8 carbon atoms, X represents an alkylene chain having 1 to 8 carbon atoms which may have a group selected from an alkyl group having 1 to 8 carbon atoms, an alkoxy group having 1 to 8 carbon atoms, and hydroxyl group as a substituent, and may contain a double bond, Y represents C(=O), C(=N-OR¹⁰), CH(OR¹¹), CH=CH, C≡C, or C(=CH₂), wherein R¹⁰ and R¹¹ represent hydrogen atom, an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, or pyridyl group, B represents CH or nitrogen atom, Z represents oxygen atom or sulfur atom, R⁶ and R⁷ represent hydrogen atom, an alkyl group having 1 to 8 carbon atoms, or an alkyl group having 1 to 8 carbon atoms and substituted with a halogen atom, and R⁸ represents hydrogen atom, or an alkyl group having 1 to 8 carbon atoms,
      provided that at least one of R³, R⁴, and R⁵ is not hydrogen atom, for use in the treatment of wound.
(2-16) The compound, a tautomer, a stereoisomer, or a pharmaceutically acceptable salt of the compound, or a solvate of any of the foregoing according to (2-15) for the use according to (2-15), which is a compound represented by the general formula (II), a tautomer, a stereoisomer, or a pharmaceutically acceptable salt of the compound, or a solvate of any of the foregoing, wherein R¹ is a phenyl group which may have a group or an atom selected from an alkyl group having 1 to 8 carbon atoms, an alkyl group having 1 to 8 carbon atoms and substituted with 1 to 3 halogen atoms, an alkoxy group having 1 to 8 carbon atoms, an alkoxy group having 1 to 8 carbon atoms and substituted with 1 to 3 halogen atoms, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent.
(2-17) The compound, a tautomer, a stereoisomer, or a pharmaceutically acceptable salt of the compound, or a solvate of any of the foregoing according to (2-15) or (2-16) for the use according to (2-15) or (2-16), which is a compound represented by the general formula (II), a tautomer, a stereoisomer, or a pharmaceutically acceptable salt of the compound, or a solvate of any of the foregoing, wherein R² is an alkyl group having 2 to 8 carbon atoms as a substituent.
(2-18) The compound, a tautomer, a stereoisomer, or a pharmaceutically acceptable salt of the compound, or a solvate of any of the foregoing according to any one of (2-15) to (2-17) for the use according to any one of (2-15) to (2-17), which is a compound represented by the general formula (II), a tautomer, a stereoisomer, or a pharmaceutically acceptable salt of the compound, or a solvate of any of the foregoing, wherein the substitution position of R¹ is the 2-position.
(2-19) The compound, a tautomer, a stereoisomer, or a pharmaceutically acceptable salt of the compound, or a solvate of any of the foregoing according to any one of (2-15) to (2-18) for the use according to any one of (2-15) to (2-18), which is a compound represented by the general formula (II), a tautomer, a stereoisomer, or a pharmaceutically acceptable salt of the compound, or a solvate of any of the foregoing, wherein A is oxygen atom or sulfur atom.
(2-20) The compound, a tautomer, a stereoisomer, or a pharmaceutically acceptable salt of the compound, or a solvate of any of the foregoing according to any one of (2-15) to (2-19) for the use according to any one of (2-15) to (2-19), which is a compound represented by the general formula (II), a tautomer, a stereoisomer, or a pharmaceutically acceptable salt of the compound, or a solvate of any of the foregoing, wherein X is an alkylene chain having 1 to 8 carbon atoms.
(2-21) The compound, a tautomer, a stereoisomer, or a pharmaceutically acceptable salt of the compound, or a solvate of any of the foregoing according to any one of (2-15) to (2-20) for the use according to any one of (2-15) to (2-20), which is a compound represented by the general formula (II), a tautomer, a stereoisomer, or a pharmaceutically acceptable salt of the compound, or a solvate of any of the foregoing, wherein Y is C(=O).
(2-22) The compound, a tautomer, a stereoisomer, or a pharmaceutically acceptable salt of the compound, or a solvate of any of the foregoing according to any one of (2-15) to (2-21) for the use according to any one of (2-15) to (2-21), which is a compound represented by the general formula (II), a tautomer, a stereoisomer, or a pharmaceutically acceptable salt of the compound, or a solvate of any of the foregoing, wherein R³, R⁴, and R⁵ are hydrogen atom, an alkyl group having 1 to 8 carbon atoms, or an alkyl group having 1 to 8 carbon atoms and substituted with a halogen atom.
(2-23) The compound, a tautomer, a stereoisomer, or a pharmaceutically acceptable salt of the compound, or a solvate of any of the foregoing according to any one of (2-15) to (2-22) for the use according to any one of (2-15) to (2-22), which is a compound represented by the general formula (II), a tautomer, a stereoisomer, or a pharmaceutically acceptable salt of the compound, or a solvate of any of the foregoing, wherein B is CH.
(2-24) The compound, a tautomer, a stereoisomer, or a pharmaceutically acceptable salt of the compound, or a solvate of any of the foregoing according to any one of (2-15) to (2-23) for the use according to any one of (2-15) to (2-23), which is a compound represented by the general formula (II), a tautomer, a stereoisomer, or a pharmaceutically acceptable salt of the compound, or a solvate of any of the foregoing, wherein Z is oxygen atom.
(2-25) The compound, a tautomer, a stereoisomer, or a pharmaceutically acceptable salt of the compound, or a solvate of any of the foregoing according to any one of (2-15) to (2-24) for the use according to any one of (2-15) to (2-24), which is a compound represented by the general formula (II), a tautomer, a stereoisomer, or a pharmaceutically acceptable salt of the compound, or a solvate of any of the foregoing, wherein R⁶ and R⁷ are hydrogen atom, or an alkyl group having 1 to 4 carbon atoms.
(2-26) The compound, a tautomer, a stereoisomer, or a pharmaceutically acceptable salt of the compound, or a solvate of any of the foregoing according to any one of (2-15) to (2-25) for the use according to any one of (2-15) to (2-25), which is a compound represented by the general formula (II), a tautomer, a stereoisomer, or a pharmaceutically acceptable salt of the compound, or a solvate of any of the foregoing, wherein R⁸ is hydrogen atom.
(2-27) The compound, a tautomer, a stereoisomer, or a pharmaceutically acceptable salt of the compound, or a solvate of any of the foregoing according to (2-15) for the use according to (2-15), which is a compound represented by the general formula (II), a tautomer, a stereoisomer, or a pharmaceutically acceptable salt of the compound, or a solvate of any of the foregoing, wherein R¹ is a phenyl group or naphthyl group which may have a group or an atom selected from an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent, R² is an alkyl group having 2 to 8 carbon atoms, A is oxygen atom or sulfur atom, X is an alkylene chain having 1 to 8 carbon atoms which may have an alkyl group having 1 to 8 carbon atoms as a substituent, and may contain a double bond, Y is C(=O), CH=CH, or C(=CH₂), R³, R⁴, and R⁵ are hydrogen atom, an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, or pyridyl group, B is CH, Z is oxygen atom or sulfur atom, R⁶ and R⁷ are hydrogen atom, or an alkyl group having 1 to 8 carbon atoms, and R⁸ is hydrogen atom, or an alkyl group having 1 to 8 carbon atoms.
(2-28) The compound, a tautomer, a stereoisomer, or a pharmaceutically acceptable salt of the compound, or a solvate of any of the foregoing according to any one of (2-27) for the use according to (2-27), which is a compound represented by the general formula (II), a tautomer, a stereoisomer, or a pharmaceutically acceptable salt of the compound, or a solvate of any of the foregoing, wherein X is an alkylene chain having 1 to 8 carbon atoms.
(2-29) The compound, a tautomer, a stereoisomer, or a pharmaceutically acceptable salt of the compound, or a solvate of any of the foregoing according to (2-27) or (2-28) for the use according to (2-27) or (2-28), which is a compound represented by the general formula (II), a tautomer, a stereoisomer, or a pharmaceutically acceptable salt of the compound, or a solvate of any of the foregoing, wherein the substitution position of R¹ is the 2-position.
(2-30) The compound, a tautomer, a stereoisomer, or a pharmaceutically acceptable salt of the compound, or a solvate of any of the foregoing according to any one of (2-27) to (2-29) for the use according to any one of (2-27) to (2-29), which is a compound represented by the general formula (II), a tautomer, a stereoisomer, or a pharmaceutically acceptable salt of the compound, or a solvate of any of the foregoing, wherein R⁸ is hydrogen atom.
(2-31) The compound, a tautomer, a stereoisomer, or a pharmaceutically acceptable salt of the compound, or a solvate of any of the foregoing according to any one of (2-27) to (2-30) for the use according to any one of (2-27) to (2-30), which is a compound represented by the general formula (II), a tautomer, a stereoisomer, or a pharmaceutically acceptable salt of the compound, or a solvate of any of the foregoing, wherein a substituent other than hydrogen atom as R³, R⁴, or R⁵ substitutes at the ortho-position of -Z-CR⁶R⁷CO₂R⁸.
(2-32) Any one of the compounds of 1) to 50) described below:
   1) 2-[4-[3-[2-(2,4-dichlorophenyl)-5-isopropyl-4-oxazolyl]propionyl]-2-methylphenoxy]-2-methylpropionic acid;
   2) [4-[3-[2-(2,4-dichlorophenyl)-5-isopropyl-4-oxazolyl]propionyl]-2-methylphenoxy]acetic acid;
   3) [4-[3-[2-(4-trifluoromethyl)phenyl-4-isopropyl-5-thiazolyl]propionyl]-2-methylphenoxy]acetic acid;
   4) 2-[4-[3-[2-(4-trifluoromethyl)phenyl-4-isopropyl-5-thiazolyl]propionyl]-2-methylphenoxy]-2-methylpropionic acid;
   5) [2-allyl-4-[3-[2-(2,4-dichlorophenyl)-5-isopropyl-4-oxazolyl]propionyl]phenoxy]acetic acid;
   6) [4-[3-[2-(2-hydroxy-4-chlorophenyl)-5-isopropyl-4-oxazolyl]propionyl]-2-methylphenoxy]acetic acid;
   7) [4-[3-[2-(2,4-dichlorophenyl)-5-isopropyl-4-oxazolyl]propionyl]-2-methylphenylsulfanyl]acetic acid;
   8) 2-[4-[3-[2-(2-hydroxy-4-chlorophenyl)-5-isopropyl-4-oxazolyl]propionyl]-2-methylphenoxy] -2-methylpropionic acid;
   9) [4-[3-[2-(2,4-dichlorophenyl)-5-isopropyl-4-oxazolyl]-1-propenyl]-2-methylphenoxy]acetic acid;
   10) [4-[3-[2-(2,4-dichlorophenyl)-5-isopropyl-4-oxazolyl]-1-propenyl]-2-methylphenoxy]acetic acid;
   11) [4-[3-[4-hexyl-2-(4-trifluoromethyl)phenyl-5-thiazolyl]propionyl]-2-methylphenoxy]acetic acid;
   12) 2-[4-[3-[4-hexyl-2-(4-trifluoromethyl)phenyl-5-thiazolyl]propionyl]-2-methylphenoxy]-2-methylpropionic acid;
   13) 2-[4-[3-[4-isopropyl-2-(4-trifluoromethyl)phenyl-5-thiazolyl]-1-propenyl]-2-methylphenoxy]-2-methylpropionic acid;
   14) [4-[3-[4-isopropyl-2-(4-trifluoromethyl)phenyl-5-thiazolyl]propionyl]-3-methylphenoxy]acetic acid;
   15) [4-[3-[2-(2,4-dichlorophenyl)-5-isopropyl-4-oxazolyl]propionyl]-3-methylphenoxy]acetic acid;
   16) [4-[3-[4-isopropyl-2-(4-trifluoromethyl)phenyl-5-thiazolyl]propionyl]-3-methylphenoxy]-2-methylpropionic acid;
   17) 2-[4-[3-[2-(2,4-dichlorophenyl)-5-isopropyl-4-oxazolyl]propionyl]-3-methylphenoxy]-2-methylpropionic acid;
   18) [4-[3-[4-isopropyl-2-(4-trifluoromethyl)phenyl-5-thiazolyl]propionyl]-2-propylphenoxy]acetic acid;
   19) 2-allyl-4-[3-[4-isopropyl-2-(4-trifluoromethyl)phenyl-5-thiazolyl]propionyl]phenoxyacetic acid;
   20) [4-[4-[2-(2,4-dichlorophenyl)-5-isopropyl-4-oxazolyl]-1-buten-2-yl]-2-methylphenoxy]acetic acid;
   21) 2-[4-[4-[2-(2,4-dichlorophenyl)-5-isopropyl-4-oxazolyl]-1-buten-2-yl]-2-methylpropionic acid;
   22) [4-[3-[2-(2,4-dichlorophenyl)-5-isopropyl-4-oxazolyl]-2-methylpropionyl]-2-methylphenoxy]acetic acid;
   23) 2-[4-[3-[2-(2,4-dichlorophenyl)-5-isopropyl-4-oxazolyl]-2-methylpropionyl]-2-methylphenoxy]-2-methylpropionic acid;
   24) [4-[3-[4-isopropyl-2-(4-trifluoromethyl)phenyl-5-thiazolyl]propenoyl]-2-methylphenoxy]acetic acid;
   25) 2-[4-[3-[4-isopropyl-2-(4-trifluoromethyl)phenyl-5-thiazolyl]propenoyl]-2-methylphenoxy]-2-methylpropionic acid;
   26) [4-[3-[4-isopropyl-2-(4-methoxyphenyl)-5-thiazolyl]propionyl]-2-methylphenoxy]propionic acid;
   27) [4-[3-[2-(3,5-dichlorophenyl)-4-isopropylthiazol-5-yl]propionyl]-2-methylphenoxy]acetic acid;
   28) 2-[4-[3-[2-(3,5-difluorophenyl)-4-isopropyl-5-thiazolyl]propionyl]-2-methylphenoxy]-2-methylpropionic acid;
   29) [4-[3-[4-isopropyl-2-(2-naphthyl)-5-thiazolyl]propionyl]-2-methylphenoxy]acetic acid;
   30) 2-[4-[3-[4-isopropyl-2-(2-naphthyl)-5-thiazolyl]propionyl]-2-methylphenoxy]-2-methylpropionic acid;
   31) [4-[3-[2-(4-butylphenyl)-4-isopropyl-5-thiazolyl]propionyl]-2-methylphenoxy]acetic acid;
   32) 2-[4-[3-[2-(4-butylphenyl)-4-isopropyl-5-thiazolyl]propionyl]-2-methylphenoxy]-2-methylpropionic acid;
   33) [4-[3-[4-isopropyl-2-(4-trifluoromethyl)phenyl-5-thiazolyl]propionyl]-2-chlorophenoxy]acetic acid;
   34) [4-[3-[2-(4-trifluoromethyl)phenyl-4-isopropyl-5-thiazolyl]propionyl]-2-chlorophenoxy]-2-methylprop ionic acid;
   35) [4-[3-[2-(2,4-dichlorophenyl)-5-isopropyl-4-oxazolyl]propionyl]-2-chlorophenoxy]acetic acid;
   36) 2-[4-[3-[2-(2,4-dichlorophenyl)-5-isopropyl-4-oxazolyl]propionyl]-2-chlorophenoxy]-2-methylpropionic acid;
   37) [4-[3-[5-isopropyl-2-(4-trifluoromethyl)phenyl-4-thiazolyl]propionyl]-2-methylphenoxy]acetic acid;
   38) 2-[4-[3-[5-isopropyl-2-(4-trifluoromethyl)phenyl-4-thiazolyl]propionyl]-2-methylphenoxy]-2-methylpropionic acid;
   39) [4-[3-[2-(2,4-dichlorophenyl)-5-isopropyl-4-thiazolyl]propionyl]-2-methylphenoxy]acetic acid;
   40) 2-[4-[3-[2-(2,4-dichlorophenyl)-5-isopropyl-4-thiazolyl]propionyl]-2-methylphenoxy]-2-methylpropionic acid;
   41) [5-[3-[4-isopropyl-2-(4-trifluoromethyl)phenyl-5-thiazolyl]propionyl]-2-methylphenoxy]acetic acid;
   42) 2-[5-[3-[4-isopropyl-2-(4-trifluoromethyl)phenyl-5-thiazolyl]propionyl]-2-methylphenoxy]-2-methylpropionic acid;
   43) 2-[4-[3-[4-isopropyl-2-(4-trifluoromethyl)phenyl-5-thiazolyl]propionyl]-2-methylphenoxy]propionic acid;
   44) 4-[3-[4-methyl-2-(4-trifluoromethyl)phenyl-5-thiazolyl]propionyl]-2-methylphenoxy]acetic acid;
   45) 2-[4-[3-[4-hexyl-2-(4-trifluoromethyl)phenyl-5-thiazolyl]-1-propenyl]-2-methylphenoxy]-2-methylpropionic acid;
   46) 2-[5-[3-[4-hexyl-2-(4-trifluoromethyl)phenyl-5-thiazolyl]propionyl]-2-methylphenoxy]-2-methylpropionic acid;
   47) [4-[3-[4-ethyl-2-(4-trifluoromethyl)phenyl-5-thiazolyl]propionyl]-2-methylphenoxy]acetic acid;
   48) 2-[4-[3-[4-ethyl-2-(4-trifluoromethyl)phenyl-5-thiazolyl]propionyl]-2-methylphenoxy]-2-methylpropionic acid;
   49) [4-[3-[4-isopropyl-2-(4-methylphenyl)-5-thiazolyl]propionyl]-2-methylphenoxy]acetic acid; and
   50) 2-[4-[3-[4-isopropyl-2-(4-methylphenyl)-5-thiazolyl]propionyl]-2-methylphenoxy]-2-methylpropionic acid,
   a tautomer, a stereoisomer, or a pharmaceutically acceptable salt of the compound, or a solvate of any of the foregoing, which is used for treatment of wound.
(2-33) The compound, a tautomer, a stereoisomer, or a pharmaceutically acceptable salt of the compound, or a solvate of any of the foregoing according to any one of (2-1) to (2-32) for the use mentioned in any one of (2-1) to (2-32), which is a compound, a tautomer, a stereoisomer, or a pharmaceutically acceptable salt of the compound, or a solvate of any of the foregoing used for topical administration to the skin.
(2-34) The compound, a tautomer, a stereoisomer, or a pharmaceutically acceptable salt of the compound, or a solvate of any of the foregoing according to any one of (2-1) to (2-33) for the use mentioned in any one of (2-1) to (2-33), which is a compound, a tautomer, a stereoisomer, or a pharmaceutically acceptable salt of the compound, or a solvate of any of the foregoing which promotes wound healing (for example, promotes wound healing during the inflammation phase and proliferation phase in the wound healing process).
(2-35) The compound, a tautomer, a stereoisomer, or a pharmaceutically acceptable salt of the compound, or a solvate of any of the foregoing according to any one of (2-1) to (2-34) for the use according to any one of (2-1) to (2-34), which is a compound, a tautomer, a stereoisomer, or a pharmaceutically acceptable salt of the compound, or a solvate of any of the foregoing which suppresses exacerbation of wound (for example, expansion of wound surface during the inflammation phase and/or proliferation phase).
(2-36) The compound, a tautomer, a stereoisomer, or a pharmaceutically acceptable salt of the compound, or a solvate of any of the foregoing according to any one of (2-1) to (2-35) for the use according to any one of (2-1) to (2-35), which is a compound, a tautomer, a stereoisomer, or a pharmaceutically acceptable salt of the compound, or a solvate of any of the foregoing for suppressing aggravation of wound surface and/or expansion of wound surface caused by exudate.
(2-37) The compound, a tautomer, a stereoisomer, or a pharmaceutically acceptable salt of the compound, or a solvate of any of the foregoing according to any one of (2-1) to (2-36) for the use according to any one of (2-1) to (2-36), which is a compound, a tautomer, a stereoisomer, or a pharmaceutically acceptable salt of the compound, or a solvate of any of the foregoing for treatment of pressure ulcer or diabetic ulcer.
(2-38) The compound, a tautomer, a stereoisomer, or a pharmaceutically acceptable salt of the compound, or a solvate of any of the foregoing according to any one of (2-1) to (2-32), which is used for promoting wound healing (for example, promoting wound healing during the inflammation phase and proliferation phase in the wound healing process).
(2-39) The compound, a tautomer, a stereoisomer, or a pharmaceutically acceptable salt of the compound, or a solvate of any of the foregoing according to any one of (2-1) to (2-32), which is used for suppressing exacerbation of wound (for example, expansion of wound surface during the inflammation phase and/or proliferation phase).
(2-40) The compound, a tautomer, a stereoisomer, or a pharmaceutically acceptable salt of the compound, or a solvate of any of the foregoing according to any one of (2-1) to (2-32), which is used for suppressing aggravation of wound surface and/or expansion of wound surface caused by exudate.
(2-41) The compound, a tautomer, a stereoisomer, or a pharmaceutically acceptable salt of the compound, or a solvate of any of the foregoing according to any one of (2-38) to (2-40), which is used for topical administration to the skin.
(2-42) The compound, a tautomer, a stereoisomer, or a pharmaceutically acceptable salt of the compound, or a solvate of any of the foregoing according to any one of (2-37) to (2-41), which is used for treatment of pressure ulcer or diabetic ulcer.

(3-1) Use of a compound represented by the following general formula (I), a tautomer, a stereoisomer, or a pharmaceutically acceptable salt of the compound, or a solvate of any of the foregoing for manufacture of a pharmaceutical composition for wound treatment: 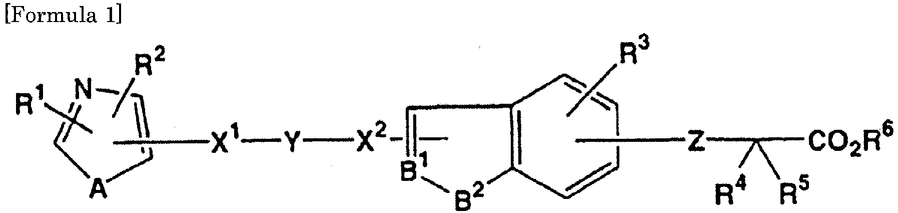 wherein
   A represents O, S, or NR⁷, wherein R⁷ represents hydrogen atom, or an alkyl group having 1 to 8 carbon atoms,
   represents CW or N, wherein W represents hydrogen atom, or an atomic bond,
   B² represents O, S, or NR⁸, wherein R⁸ represents hydrogen atom, or an alkyl group having 1 to 8 carbon atoms,
   X¹ and X² represent O, S, NH, NHC(=O), C(=O), C(=N-OR⁹), CH(OR¹⁰), C=C, C≡C, or an atomic bond, wherein R⁹ and R¹⁰ represent hydrogen atom, or an alkyl group having 1 to 8 carbon atoms,
   Y represents an alkylene chain having 1 to 8 carbon atoms which may have an alkyl group having 1 to 8 carbon atoms, or an alkyl group having 1 to 8 carbon atoms and substituted with 1 to 3 halogen atoms as a substituent,
   Z represents NH, O, or S,
   R¹ represents an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent, or a heterocyclic group having a 5- to 8-membered ring comprising 1 to 3 heteroatoms selected from nitrogen atom, oxygen atom, and sulfur atom, and the remainder carbon atoms as ring-constituting atoms (benzene ring may further condense to this heterocyclic ring),
   R² represents an alkyl group having 2 to 8 carbon atoms, an alkyl group having 1 to 8 carbon atoms and substituted with 1 to 3 halogen atoms, a cycloalkyl group having 3 to 7 carbon atoms, an alkenyl group having 2 to 8 carbon atoms, an alkynyl group having 2 to 8 carbon atoms, an alkyl group substituted with an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent (the alkyl moiety thereof has 1 to 4 carbon atoms), or an alkyl group substituted with a 5- to 8-membered heterocyclic ring (the heterocyclic ring thereof comprises 1 to 3 heteroatoms selected from nitrogen atom, oxygen atom, and sulfur atom, and the remainder carbon atoms as ring-constituting atoms, and the alkyl moiety thereof has 1 to 4 carbon atoms),
   R³ represents hydrogen atom, a halogen atom, trifluoromethyl group, an alkyl group having 1 to 8 carbon atoms, an alkenyl group having 2 to 8 carbon atoms, or an alkynyl group having 2 to 8 carbon atoms,
   R⁴ and R⁵ represent hydrogen atom, an alkyl group having 1 to 8 carbon atoms, or an alkyl group having 1 to 8 carbon atoms and substituted with 1 to 3 halogen atoms, and
   R⁶ represents hydrogen atom, an alkyl group having 1 to 8 carbon atoms and substituted with an amino group, an alkyl group having 1 to 8 carbon atoms, or an alkali metal,
   provided that Z and R³ bond to the benzene ring, and X² does not bond to the benzene ring.
(3-2) The use according to (3-1), wherein, in the general formula (I), is N, and B² is O.
(3-3) The use according to (3-1) or (3-2), wherein, in the general formula (I), A is S.
(3-4) The use according to (3-1) or (3-2), wherein, in the general formula (I), A is O.
(3-5) The use according to any one of (3-1) to (3-4), wherein, in the general formula (I), Z is O.
(3-6) The use according to any one of (3-1) to (3-4), wherein, in the general formula (I), Z is NH.
(3-7) The use according to any one of (3-1) to (3-4), wherein, in the general formula (I), Z is S.
(3-8) The use according to any one of (3-1) to (3-7), wherein, in the general formula (I), R¹ is a phenyl group which may have an alkyl group substituted with 1 to 3 halogen atoms.
(3-9) The use according to any one of (3-1) to (3-8), wherein, in the general formula (I), X¹ and X² are atomic bonds, and Y is ethylene group.
(3-10) The use according to any one of (3-1) to (3-9), wherein, in the general formula (I), R² is isopropyl group.
(3-11) The use according to any one of (3-1) to (3-10), wherein, in the general formula (I), R³ is an alkyl group having 1 to 3 carbon atoms.
(3-12) The use according to any one of (3-1) to (3-11), wherein, in the general formula (I), R⁴ and R⁵ are hydrogen atoms.
(3-13) The use according to any one of (3-1) to (3-12), wherein, in the general formula (I), R⁶ is hydrogen atom.
(3-14) Use of any one of the compounds of (a) to (j) described below:
   (a) [3-[2-[4-isopropyl-2-(4-trifluoromethyl)phenyl-5-thiazolyl]-ethyl]-5-methyl-1,2-benzisoxazol-6-yl]oxyacetic acid;
   (b) 2-[[3-[2-[4-isopropyl-2-(4-trifluoromethyl)phenyl-5-thiazolyl]ethyl]-5-methyl-1,2-benzisoxazol-6-yl]oxy]-2-methylpropionic acid;
   (c) [3-[2-[4-isopropyl-2-(4-trifluoromethyl)phenyl-5-thiazolyl]ethyl]-5-methyl-1,2-benzisoxazol-6-yl]thioacetic acid;
   (d) [3-[2-[4-isopropyl-2-(4-trifluoromethyl)phenyl-5-thiazolyl]ethyl]-5-methyl-1,2-benzisoxazol-6-yl]aminoacetic acid;
   (e) [3-[2-[2-(2,4-dichlorophenyl)-5-isopropyl-4-oxazolyl]ethyl]-5-methyl-1,2-benzisoxazol-6-yl]oxyacetic acid;
   (f) 2-[[3-[2-[4-isopropyl-2-(4-trifluoromethyl)phenyl-5-thiazolyl]ethyl]-5-methyl-1,2-benzisoxazol-6-yl]oxy]-2-methylpropionic acid 2-piperidinoethyl ester hydrochloride;
   (g) [[7-allyl-3-[2-[2-(2,4-dichlorophenyl)-5-isopropyl-4-oxazolyl]ethyl]-1,2-benzisoxazol-6-yl]oxy]acetic acid;
   (h) 2-[[7-allyl-3-[2-[2-(2,4-dichlorophenyl)-5-isopropyl-4-oxazolyl]ethyl]-1,2-benzisoxazol-6-yl]oxy]-2-methylpropionic acid;
   (i) [[7-propyl-3-[2-[2-(2,4-dichlorophenyl)-5-isopropyl-4-oxazolyl]ethyl]-1,2 -benzisoxazol-6-yl]oxy]acetic acid; and
   (j) 2-[[7-allyl-3-[2-[2-[(4-trifluoromethyl)phenyl]-4-isopropyl-5-thiazolyl]ethyl]-1,2-benzisoxazol-6-yl]oxy]-2-methylpropionic acid,
   a tautomer, a stereoisomer, or a pharmaceutically acceptable salt of the compound, or a solvate of any of the foregoing,
   for manufacture of a pharmaceutical composition for wound treatment.
(3-15) Use of a compound represented by the following general formula (II), a tautomer, a stereoisomer, or a pharmaceutically acceptable salt of the compound, or a solvate of any of the foregoing for manufacture of a pharmaceutical composition for wound treatment: 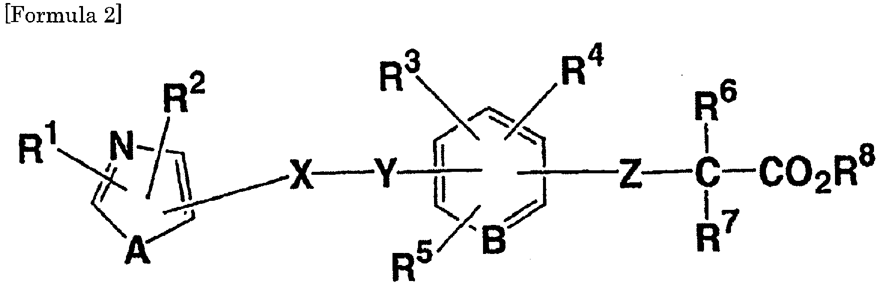
   wherein R¹ represents a phenyl group, naphthyl group, pyridyl group, thienyl group, furyl group, quinolyl group, or benzothienyl group which may have a group or an atom selected from an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent, R² represents an alkyl group having 1 to 8 carbon atoms, an alkyl group having 1 to 8 carbon atoms and substituted with a halogen atom, an alkenyl group having 2 to 8 carbon atoms, an alkynyl group having 2 to 8 carbon atoms, a cycloalkyl group having a 3- to 7-membered ring, an alkyl group having 1 to 8 carbon atoms and substituted with a cycloalkyl group having a 3- to 7-membered ring, a phenyl group, which may have a group or an atom selected from an alkoxy group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkynyl group having 2 to 8 carbon atoms, a halogen atom, an acyl group having 2 to 7 carbon atoms, a benzoyl group, a hydroxyl group, a nitro group, an amino group, a phenyl group, and a pyridyl group, a naphthyl group, or an alkyl group having 1 to 6 carbon atoms and substituted with a pyridyl group, A represents oxygen atom, sulfur atom, or NR⁹, wherein R⁹ represents hydrogen atom, or an alkyl group having 1 to 8 carbon atoms, X represents an alkylene chain having 1 to 8 carbon atoms which may have a group selected from an alkyl group having 1 to 8 carbon atoms, an alkoxy group having 1 to 8 carbon atoms, and hydroxyl group as a substituent, and may contain a double bond, Y represents C(=O), C(=NOR¹⁰), CH(OR¹¹), CH=CH, C≡C, or C(=CH₂), wherein R¹⁰ and R¹¹ represent hydrogen atom, an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, or pyridyl group, B represents CH or nitrogen atom, Z represents oxygen atom or sulfur atom, R⁶ and R⁷ represent hydrogen atom, an alkyl group having 1 to 8 carbon atoms, or an alkyl group having 1 to 8 carbon atoms and substituted with a halogen atom, and R⁸ represents hydrogen atom, or an alkyl group having 1 to 8 carbon atoms,
   provided that at least one of R³, R⁴, and R⁵ is not hydrogen atom.
(3-16) The use according to (3-15), wherein, as for the general formula (II), R¹ is a phenyl group which may have a group or an atom selected from an alkyl group having 1 to 8 carbon atoms, an alkyl group having 1 to 8 carbon atoms and substituted with 1 to 3 halogen atoms, an alkoxy group having 1 to 8 carbon atoms, an alkoxy group having 1 to 8 carbon atoms and substituted with 1 to 3 halogen atoms, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent.
(3-17) The use according to (3-15) or (3-16), wherein, as for the general formula (II), R² is an alkyl group having 2 to 8 carbon atoms as a substituent.
(3-18) The use according to any one of (3-15) to (3-17), wherein, as for the general formula (II), the substitution position of R¹ is the 2-position.
(3-19) The use according to any one of (3-15) to (3-18), wherein, as for the general formula (II), A is oxygen atom or sulfur atom.
(3-20) The use according to any one of (3-15) to (3-19), wherein, as for the general formula (II), X is an alkylene chain having 1 to 8 carbon atoms.
(3-21) The use according to any one of (3-15) to (3-20), wherein, as for the general formula (II), Y is C(=O).
(3-22) The use according to any one of (3-15) to (3-21), wherein, as for the general formula (II), R³, R⁴, and R⁵ are hydrogen atom, an alkyl group having 1 to 8 carbon atoms, or an alkyl group having 1 to 8 carbon atoms and substituted with a halogen atom.
(3-23) The use according to any one of (3-15) to (3-22), wherein, as for the general formula (II), B is CH.
(3-24) The use according to any one of (3-15) to (3-23), wherein, as for the general formula (II), Z is oxygen atom.
(3-25) The use according to any one of (3-15) to (3-24), wherein, as for the general formula (II), R⁶ and are hydrogen atom, or an alkyl group having 1 to 4 carbon atoms.
(3-26) The use according to any one of (3-15) to (3-25), wherein, as for the general formula (II), R⁸ is hydrogen atom.
(3-27) The use according to (3-15), wherein, as for the general formula (II), R¹ is a phenyl group or naphthyl group which may have a group or an atom selected from an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent, R² is an alkyl group having 2 to 8 carbon atoms, A is oxygen atom or sulfur atom, X is an alkylene chain having 1 to 8 carbon atoms which may have an alkyl group having 1 to 8 carbon atoms as a substituent, and may contain a double bond, Y is C(=O), CH=CH, or C(=CH₂), R³, R⁴, and R⁵ are hydrogen atom, an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, or pyridyl group, B is CH, Z is oxygen atom or sulfur atom, R⁶ and R⁷ are hydrogen atom, or an alkyl group having 1 to 8 carbon atoms, and R⁸ is hydrogen atom, or an alkyl group having 1 to 8 carbon atoms.
(3-28) The use according to (3-27), wherein, as for the general formula (II), X is an alkylene chain having 1 to 8 carbon atoms.
(3-29) The use according to (3-27) or (3-28), wherein, as for the general formula (II), the substitution position of R¹ is the 2-position.
(3-30) The use according to any one of (3-27) to (3-30), wherein, as for the general formula (II), R⁸ is hydrogen atom.
(3-31) The use according to any one of (3-27) to (3-30), wherein, as for the general formula (II), a substituent other than hydrogen atom as R³, R⁴, or R⁵ substitutes at the ortho-position of -Z-CR⁶R⁷CO₂R⁸.
(3-32) Use of any one of the compounds of 1) to 50) described below:
   1) 2-[4-[3-[2-(2,4-dichlorophenyl)-5-isopropyl-4-oxazolyl]proplonyl]-2-methylphenoxy]-2-methylpropionic acid;
   2) [4-[3-[2-(2,4-dichlorophenyl)-5-isopropyl-4-oxazolyl]propionyl]-2-methylphenoxy] acetic acid;
   3) [4-[3-[2-(4-trifluoromethyl)phenyl-4-isopropyl-5-thiazolyl]propionyl]-2-methylphenoxy]acetic acid;
   4) 2-[4-[3-[2-(4-trifluoromethyl)phenyl-4-isopropyl-5-thiazolyl]propionyl]-2-methylphenoxy]-2-methylpropionic acid;
   5) [2-allyl-4-[3-[2-(2,4-dichlorophenyl)-5-isopropyl-4-oxazolyl]propionyl]phenoxy]acetic acid;
   6) [4-[3-[2-(2-hydroxy-4-chlorophenyl)-5-isopropyl-4-oxazolyl]propionyl]-2-methylphenoxy]acetic acid;
   7) [4-[3-[2-(2,4-dichlorophenyl)-5-isopropyl-4-oxazolyl]propionyl]-2-methylphenylsulfanyl]acetic acid;
   8) 2-[4-[3-[2-(2-hydroxy-4-chlorophenyl)-5-isopropyl-4-oxazolyl]propionyl]-2-methylphenoxy]-2-methylpropionic acid;
   9) [4-[3-[2-(2,4-dichlorophenyl)-5-isopropyl-4-oxazolyl]-1-propenyl]-2-methylphenoxy]acetic acid;
   10) [4-[3-[2-(2,4-dichlorophenyl)-5-isopropyl-4-oxazolyl]-1-propenyl]-2-methylphenoxy]acetic acid;
   11) [4-[3-[4-hexyl-2-(4-trifluoromethyl)phenyl-5-thiazolyl]propionyl]-2-methylphenoxy]acetic acid;
   12) 2-[4-[3-[4-hexyl-2-(4-trifluoromethyl)phenyl-5-thiazolyl]propionyl]-2-methylphenoxy]-2-methylpropionic acid;
   13) 2-[4-[3-[4-isopropyl-2-(4-trifluoromethyl)phenyl-5-thiazolyl]-1-propenyl]-2-methylphenoxy]-2-methylpropionic acid;
   14) [4-[3-[4-isopropyl-2-(4-trifluoromethyl)phenyl-5-thiazolyl]propionyl]-3-methylphenoxy]acetic acid;
   15) [4-[3-[2-(2,4-dichlorophenyl)-5-isopropyl-4-oxazolyl]propionyl]-3-methylphenoxy]acetic acid;
   16) [4-[3-[4-isopropyl-2-(4-trifluoromethyl)phenyl-5-thiazolyl]propionyl]-3-methylphenoxy]-2-methylpropionic acid;
   17) 2-[4-[3-[2-(2,4-dichlorophenyl)-5-isopropyl-4-oxazolyl]propionyl]-3-methylphenoxyl-2-methylpropionic acid;
   18) [4-[3-[4-isopropyl-2-(4-trifluoromethyl)phenyl-5-thiazolyl]propionyl]-2-propylphenoxy]acetic acid;
   19) 2-allyl-4-[3-[4-isopropyl-2-(4-trifluoromethyl)phenyl-5-thiazolyl]propionyl]phenoxyacetic acid;
   20) [4-[4-[2-(2,4-dichlorophenyl)-5-isopropyl-4-oxazolyl]-1-buten-2-yl]-2-methylphenoxy]acetic acid;
   21) 2-[4-[4-[2-(2,4-dichlorophenyl)-5-isopropyl-4-oxazolyl]-1-buten-2-yl]-2-methylpropionic acid;
   22) [4-[3-[2-(2,4-dichlorophenyl)-5-isopropyl-4-oxazolyl]-2-methylpropionyl]-2-methylphenoxy]acetic acid;
   23) 2-[4-[3-[2-(2,4-dichlorophenyl)-5-isopropyl-4-oxazolyl]-2-methylpropionyl]-2-methylphenoxy]-2-methylpropionic acid;
   24) [4-[3-[4-isopropyl-2-(4-trifluoromethyl)phenyl-5-thiazolyl]propenoyl]-2-methylphenoxy]acetic acid;
   25) 2-[4-[3-[4-isopropyl-2-(4-trifluoromethyl)phenyl-5-thiazolyl]propenoyl]-2-methylphenoxy]-2-methylpropionic acid;
   26) [4-[3-[4-isopropyl-2-(4-methoxyphenyl)-5-thiazolyl]propionyl]-2-methylphenoxy]propionic acid;
   27) [4-[3-[2-(3,5-dichlorophenyl)-4-isopropylthiazol-5-yl]propionyl]-2-methylphenoxy]acetic acid;
   28) 2-[4-[3-[2-(3,5-difluorophenyl)-4-isopropyl-5-thiazolyl]propionyl]-2-methylphenoxy]-2-methylpropionic acid;
   29) [4-[3-[4-isopropyl-2-(2-naphthyl)-5-thiazolyl]propionyl]-2-methylphenoxy]acetic acid;
   30) 2-[4-[3-[4-isopropyl-2-(2-naphthyl)-5-thiazolyl]propionyl]-2-methylphenoxy]-2-methylpropionic acid;
   31) [4-[3-[2-(4-butylphenyl)-4-isopropyl-5-thiazolyl]propionyl]-2-methylphenoxy]acetic acid;
   32) 2-[4-[3-[2-(4-butylphenyl)-4-isopropyl-5-thiazolyl]propionyl]-2-methylphenoxy]-2-methylpropionic acid;
   33) [4-[3-[4-isopropyl-2-(4-trifluoromethyl)phenyl-5-thiazolyl]propionyl]-2-chlorophenoxy]acetic acid;
   34) [4-[3-[2-(4-trifluoromethyl)phenyl-4-isopropyl-5-thiazolyl]propionyl]-2-chlorophenoxy]-2-methylpropionic acid;
   35) [4-[3-[2-(2,4-dichlorophenyl)-5-isopropyl-4-oxazolyl]propionyl]-2-chlorophenoxy]acetic acid;
   36) 2-[4-[3-[2-(2,4-dichlorophenyl)-5-isopropyl-4-oxazolyl]propionyl]-2-chlorophenoxy]-2-methylpropionic acid;
   37) [4-[3-[5-isopropyl-2-(4-trifluoromethyl)phenyl-4-thiazolyl]propionyl]-2-methylphenoxy]acetic acid;
   38) 2-[4-[3-[5-isopropyl-2-(4-trifluoromethyl)phenyl-4-thiazolyl]propionyl]-2-methylphenoxy]-2-methylpropionic acid;
   39) [4-[3-[2-(2,4-dichlorophenyl)-5-isopropyl-4-thiazolyl]propionyl]-2-methylphenoxy]acetic acid;
   40) 2-[4-[3-[2-(2,4-dichlorophenyl)-5-isopropyl-4-thiazolyl]propionyl]-2-methylphenoxy]-2-methylpropionic acid;
   41) [5-[3-[4-isopropyl-2-(4-trifluoromethyl)phenyl-5-thiazolyl]propionyl]-2-methylphenoxy]acetic acid;
   42) 2-[5-[3-[4-isopropyl-2-(4-trifluoromethyl)phenyl-5-thiazolyl]propionyl]-2-methylphenoxy]-2-methylpropionic acid;
   43) 2-[4-[3-[4-isopropyl-2-(4-trifluoromethyl)phenyl-5-thiazolyl]propionyl]-2-methylphenoxy]propionic acid;
   44) 4-[3-[4-methyl-2-(4-trifluoromethyl)phenyl-5-thiazolyl]propionyl]-2-methylphenoxy]acetic acid;
   45) 2-[4-[3-[4-hexyl-2-(4-trifluoromethyl)phenyl-5-thiazolyl]-1-propenyl]-2-methylphenoxy]-2-methylpropionic acid;
   46) 2-[5-[3-[4-hexyl-2-(4-trifluoromethyl)phenyl-5-thiazolyl]propionyl]-2-methylphenoxy]-2-methylpropionic acid;
   47) [4-[3-[4-ethyl-2-(4-trifluoromethyl)phenyl-5-thiazolyl]propionyl]-2-methylphenoxy]acetic acid;
   48) 2-[4-[3-[4-ethyl-2-(4-trifluoromethyl)phenyl-5-thiazolyl]propionyl]-2-methylphenoxy]-2-methylpropionic acid;
   49) [4-[3-[4-isopropyl-2-(4-methylphenyl)-5-thiazolyl]propionyl]-2-methylphenoxy]acetic acid; and
   50) 2-[4-[3-[4-isopropyl-2-(4-methylphenyl)-5-thiazolyl]propionyl]-2-methylphenoxy]-2-methylpropionic acid,
   a tautomer, a stereoisomer, or a pharmaceutically acceptable salt of the compound, or a solvate of any of the foregoing,
   for manufacture of a pharmaceutical composition for wound treatment.
(3-33) The use according to any one of (3-1) to (3-32), wherein the pharmaceutical composition is for topical administration to the skin.
(3-34) The use according to any one of (3-1) to (3-33), wherein the pharmaceutical composition promotes wound healing (for example, promotes wound healing during the inflammation phase and proliferation phase in the wound healing process).
(3-35) The use according to any one of (3-1) to (3-34), wherein the pharmaceutical composition suppresses exacerbation of wound (for example, expansion of wound surface during the inflammation phase and/or proliferation phase).
(3-36) The use according to any one of (3-1) to (3-35), wherein the pharmaceutical composition suppresses aggravation of wound surface and/or expansion of wound surface caused by exudate.
(3-37) The use according to any one of (3-1) to (3-36), wherein the pharmaceutical composition is for treatment of pressure ulcer or diabetic ulcer.
(3-38) The use of a compound, a tautomer, a stereoisomer, or a pharmaceutically acceptable salt of the compound, or a solvate of any of the foregoing according to any one of (3-1) to (3-32), which is for manufacture of a pharmaceutical composition for promoting wound healing (for example, a pharmaceutical composition for promoting wound healing during the inflammation phase and proliferation phase in the wound healing process).
(3-39) The use of a compound, a tautomer, a stereoisomer, or a pharmaceutically acceptable salt of the compound, or a solvate of any of the foregoing according to any one of (3-1) to (3-32), which is for manufacture of a pharmaceutical composition for suppressing exacerbation of wound (for example, a pharmaceutical composition for suppressing expansion of wound surface during the inflammation phase and/or proliferation phase).
(3-40) The use of a compound, a tautomer, a stereoisomer, or a pharmaceutically acceptable salt of the compound, or a solvate of any of the foregoing according to any one of (3-1) to (3-32), which is for manufacture of a pharmaceutical composition for suppressing aggravation of wound surface and/or expansion of wound surface caused by exudate.
(3-41) The use according to any one of (3-37) to (3-40), wherein the pharmaceutical composition is for topical administration to the skin.
(3-42) The use according to any one of (3-37) to (3-41), wherein the pharmaceutical composition is for treatment of pressure ulcer or diabetic ulcer.

(4-1) A method for treatment of wound in a mammalian subject (for example, human), which comprises administering a pharmaceutical composition comprising an effective amount of a compound represented by the following general formula (I), a tautomer, a stereoisomer, or a pharmaceutically acceptable salt of the compound, or a solvate of any of the foregoing to a subject in need thereof: 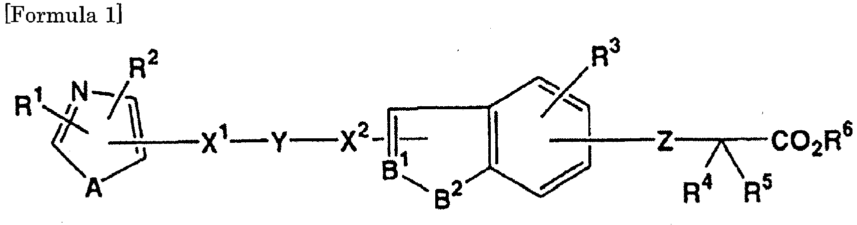 wherein
   A represents O, S, or NR⁷, wherein R⁷ represents hydrogen atom, or an alkyl group having 1 to 8 carbon atoms,
   B¹ represents CW or N, wherein W represents hydrogen atom, or an atomic bond,
   B² represents O, S, or NR⁸, wherein R⁸ represents hydrogen atom, or an alkyl group having 1 to 8 carbon atoms,
   X¹ and X² represent O, S, NH, NHC(=O), C(=O), C(=N-OR⁹), CH(OR¹⁰), C=C, C=C, or an atomic bond, wherein R⁹ and R¹⁰ represent hydrogen atom, or an alkyl group having 1 to 8 carbon atoms,
   Y represents an alkylene chain having 1 to 8 carbon atoms which may have an alkyl group having 1 to 8 carbon atoms, or an alkyl group having 1 to 8 carbon atoms and substituted with 1 to 3 halogen atoms as a substituent,
   Z represents NH, O, or S,
   R¹ represents an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent, or a heterocyclic group having a 5- to 8-membered ring comprising 1 to 3 heteroatoms selected from nitrogen atom, oxygen atom, and sulfur atom, and the remainder carbon atoms as ring-constituting atoms (benzene ring may further condense to this heterocyclic ring),
   R² represents an alkyl group having 2 to 8 carbon atoms, an alkyl group having 1 to 8 carbon atoms and substituted with 1 to 3 halogen atoms, a cycloalkyl group having 3 to 7 carbon atoms, an alkenyl group having 2 to 8 carbon atoms, an alkynyl group having 2 to 8 carbon atoms, an alkyl group substituted with an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent (the alkyl moiety thereof has 1 to 4 carbon atoms), or an alkyl group substituted with a 5- to 8-membered heterocyclic ring (the heterocyclic ring thereof comprises 1 to 3 heteroatoms selected from nitrogen atom, oxygen atom, and sulfur atom, and the remainder carbon atoms as ring-constituting atoms, and the alkyl moiety thereof has 1 to 4 carbon atoms),
   R³ represents hydrogen atom, a halogen atom, trifluoromethyl group, an alkyl group having 1 to 8 carbon atoms, an alkenyl group having 2 to 8 carbon atoms, or an alkynyl group having 2 to 8 carbon atoms,
   R⁴ and R⁵ represent hydrogen atom, an alkyl group having 1 to 8 carbon atoms, or an alkyl group having 1 to 8 carbon atoms and substituted with 1 to 3 halogen atoms, and
   R⁶ represents hydrogen atom, an alkyl group having 1 to 8 carbon atoms and substituted with an amino group, an alkyl group having 1 to 8 carbon atoms, or an alkali metal,
   provided that Z and R³ bond to the benzene ring, and X² does not bond to the benzene ring.
(4-2) The method according to (4-1), wherein, in the general formula (I), B¹ is N, and B² is O.
(4-3) The method according to (4-1) or (4-2), wherein, in the general formula (I), A is S.
(4-4) The method according to (4-1) or (4-2), wherein, in the general formula (I), A is O.
(4-5) The method according to any one of (4-1) to (4-4), wherein, in the general formula (I), Z is O.
(4-6) The method according to any one of (4-1) to (4-4), wherein, in the general formula (I), Z is NH.
(4-7) The method according to any one of (4-1) to (4-4), wherein, in the general formula (I), Z is S.
(4-8) The method according to any one of (4-1) to (4-7), wherein, in the general formula (I), R¹ is a phenyl group which may have an alkyl group substituted with 1 to 3 halogen atoms.
(4-9) The method according to any one of (4-1) to (4-8), wherein, in the general formula (I), X¹ and X² are atomic bonds, and Y is ethylene group.
(4-10) The method according to any one of (4-1) to (4-9), wherein, in the general formula (I), R² is isopropyl group.
(4-11) The method according to any one of (4-1) to (4-10), wherein, in the general formula (I), R³ is an alkyl group having 1 to 3 carbon atoms.
(4-12) The method according to any one of (4-1) to (4-11), wherein, in the general formula (I), R⁴ and R⁵ are hydrogen atoms.
(4-13) The method according to any one of (4-1) to (4-12), wherein, in the general formula (I), R⁶ is hydrogen atom.
(4-14) A method for treatment of wound in a mammalian subject (for example, human), which comprises administering a pharmaceutical composition comprising an effective amount of any one of the compounds of (a) to (j) described below:
   (a) [3-[2-[4-isopropyl-2-(4-trifluoromethyl)phenyl-5-thiazolyl]ethyl]-5-methyl-1,2-benzisoxazol-6-yl]oxyacetic acid;
   (b) 2-[[3-[2-[4-isopropyl-2-(4-trifluoromethyl)phenyl-5-thiazolyl]ethyl]-5-methyl-1,2-benzisoxazol-6-yl]oxy]-2-methylprop ionic acid;
   (c) [3-[2-[4-isopropyl-2-(4-trifluoromethyl)phenyl-5-thiazolyl]ethyl]-5-methyl-1,2-benzisoxazol-6-yl]thioacetic acid;
   (d) [3-[2-[4-isopropyl-2-(4-trifluoromethyl)phenyl-5-thiazolyl]ethyl]-5-methyl-1,2-benzisoxazol-6-yl]aminoacetic acid;
   (e) [3-[2-[2-(2,4-dichlorophenyl)-5-isopropyl-4-oxazolyl]ethyl]-5-methyl-1,2-benzisoxazol-6-yl]oxyacetic acid;
   (f) 2-[[3-[2-[4-isopropyl-2-(4-trifluoromethyl)phenyl-5-thiazolyl]ethyl]-5-methyl-1,2-benzisoxazol-6-yl]oxy]-2-methylpropionic acid 2-piperidinoethyl ester hydrochloride;
   (g) [[7-allyl-3-[2-[2-(2,4-dichlorophenyl)-5-isopropyl-4-oxazolyl]ethyl]-1,2-benzisoxazol-6-yl]oxy]acetic acid;
   (h) 2-[[7-allyl-3-[2-[2-(2,4-dichlorophenyl)-5-isopropyl-4-oxazolyl]ethyl]-1,2-benzisoxazol-6-yl]oxy]-2-methylpropionic acid;
   (i) [[7-propyl-3-[2-[2-(2,4-dichlorophenyl)-5-isopropyl-4-oxazolyl]ethyl]-1,2-benzisoxazol-6-yl]oxy]acetic acid; and
   (j) 2-[[7-allyl-3-[2-[2-[(4-trifluoromethyl)phenyl]-4-isopropyl-5-thiazolyl]ethyl]-1,2-benzisoxazol-6-yl]oxy]-2-methylpropionic acid,
   a tautomer, a stereoisomer, or a pharmaceutically acceptable salt of the compound, or a solvate of any of the foregoing to a subject in need thereof.
(4-15) A method for treatment of wound in a mammalian subject (for example, human), which comprises administering a pharmaceutical composition comprising an effective amount of a compound represented by the following general formula (II), a tautomer, a stereoisomer, or a pharmaceutically acceptable salt of the compound, or a solvate of any of the foregoing to a subject in need thereof: 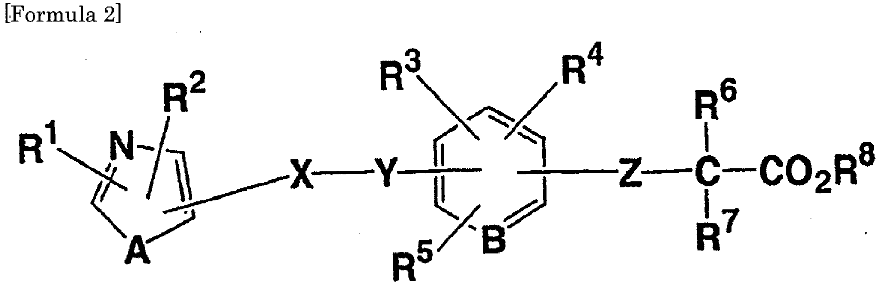
   wherein, in the formula, R¹ represents a phenyl group, naphthyl group, pyridyl group, thienyl group, furyl group, quinolyl group, or benzothienyl group which may have a group or an atom selected from an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent, R² represents an alkyl group having 1 to 8 carbon atoms, an alkyl group having 1 to 8 carbon atoms and substituted with a halogen atom, an alkenyl group having 2 to 8 carbon atoms, an alkynyl group having 2 to 8 carbon atoms, a cycloalkyl group having a 3- to 7-membered ring, an alkyl group having 1 to 8 carbon atoms and substituted with a cycloalkyl group having a 3- to 7-membered ring, a phenyl group which may have a group or an atom selected from an alkoxy group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkynyl group having 2 to 8 carbon atoms, a halogen atom, an acyl group having 2 to 7 carbon atoms, a benzoyl group, a hydroxyl group, a nitro group, an amino group, a phenyl group, and a pyridyl group, a naphthyl group, or an alkyl group having 1 to 6 carbon atoms and substituted with a pyridyl group, A represents oxygen atom, sulfur atom, or NR⁹, wherein R⁹ represents hydrogen atom, or an alkyl group having 1 to 8 carbon atoms, X represents an alkylene chain having 1 to 8 carbon atoms which may have a group selected from an alkyl group having 1 to 8 carbon atoms, an alkoxy group having 1 to 8 carbon atoms, and hydroxyl group as a substituent, and may contain a double bond, Y represents C(=O), C(=N-OR¹⁰), CH(OR¹¹), CH=CH, C≡C, or C(=CH₂), wherein R¹⁰ and R¹¹ represent hydrogen atom, an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, or pyridyl group, B represents CH or nitrogen atom, Z represents oxygen atom or sulfur atom, R⁶ and R⁷ represent hydrogen atom, an alkyl group having 1 to 8 carbon atoms, or an alkyl group having 1 to 8 carbon atoms and substituted with a halogen atom, and R⁸ represents hydrogen atom, or an alkyl group having 1 to 8 carbon atoms,
   provided that at least one of R³, R⁴, and R⁵ is not hydrogen atom.
(4-16) The method according to (4-15), wherein, in the general formula (II), R¹ is a phenyl group which may have a group or an atom selected from an alkyl group having 1 to 8 carbon atoms, an alkyl group having 1 to 8 carbon atoms and substituted with 1 to 3 halogen atoms, an alkoxy group having 1 to 8 carbon atoms, an alkoxy group having 1 to 8 carbon atoms and substituted with 1 to 3 halogen atoms, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent.
(4-17) The method according to (4-15) or (4-16), wherein, in the general formula (II), R² is an alkyl group having 2 to 8 carbon atoms as a substituent.
(4-18) The method according to any one of (4-15) to (4-17), wherein, in the general formula (II), the substitution position of R¹ is the 2-position.
(4-19) The method according to any one of (4-15) to (4-18), wherein, in the general formula (II), A is oxygen atom or sulfur atom.
(4-20) The method according to any one of (4-15) to (4-19), wherein, in the general formula (II), X is an alkylene chain having 1 to 8 carbon atoms.
(4-21) The method according to any one of (4-15) to (4-20), wherein, in the general formula (II), Y is C(=O).
(4-22) The method according to any one of (4-15) to (4-21), wherein, in the general formula (II), R³, R⁴, and R⁵ are hydrogen atom, an alkyl group having 1 to 8 carbon atoms, or an alkyl group having 1 to 8 carbon atoms and substituted with a halogen atom.
(4-23) The method according to any one of (4-15) to (4-22), wherein, in the general formula (II), B is CH.
(4-24) The method according to any one of (4-15) to (4-23), wherein, in the general formula (II), Z is oxygen atom.
(4-25) The method according to any one of (4-15) to (4-24), wherein, in the general formula (II), R⁶ and R⁷ are hydrogen atom, or an alkyl group having 1 to 4 carbon atoms.
(4-26) The method according to any one of (4-15) to (4-25), wherein, in the general formula (II), R⁸ is hydrogen atom.
(4-27) The method according to (4-15), wherein, in the general formula (II), R¹ is a phenyl group or naphthyl group which may have a group or an atom selected from an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent, R² is an alkyl group having 2 to 8 carbon atoms, A is oxygen atom or sulfur atom, X is an alkylene chain having 1 to 8 carbon atoms which may have an alkyl group having 1 to 8 carbon atoms as a substituent, and may contain a double bond, Y is C(=O), CH=CH, or C(=CH₂), R³, R⁴, and R⁵ are hydrogen atom, an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, or pyridyl group, B is CH, Z is oxygen atom or sulfur atom, R⁶ and R⁷ are hydrogen atom, or an alkyl group having 1 to 8 carbon atoms, and R⁸ is hydrogen atom, or an alkyl group having 1 to 8 carbon atoms.
(4-28) The method according to (4-27), wherein, in the general formula (II), X is an alkylene chain having 1 to 8 carbon atoms.
(4-29) The method according to (4-27) or (4-28), wherein, in the general formula (II), the substitution position of R¹ is the 2-position.
(4-30) The method according to any one of (4-27) to (4-30), wherein, in the general formula (II), R⁸ is hydrogen atom.
(4-31) The method according to any one of (4-27) to (4-30), wherein, in the general formula (II), a substituent other than hydrogen atom as R³, R⁴, or R⁵ substitutes at the ortho-position of -Z-CR⁶R⁷CO₂R⁸.
(4-32) A method for treatment of wound in a mammalian subject (for example, human), which comprises administering a pharmaceutical composition comprising an effective amount of any one of the compounds of 1) to 50) described below:
   1) 2-[4-[3-[2-(2,4-dichlorophenyl)-5-isopropyl-4-oxazolyl]propionyl]-2-methylphenoxy]-2-methylpropionic acid;
   2) [4-[3-[2-(2,4-dichlorophenyl)-5-isopropyl-4-oxazolyl]propionyl]-2-methylphenoxy]acetic acid;
   3) [4-[3-[2-(4-trifluoromethyl)phenyl-4-isopropyl-5-thiazolyl]propionyl]-2-methylphenoxy]acetic acid;
   4) 2-[4-[3-[2-(4-trifluoromethyl)phenyl-4-isopropyl-5-thiazolyl]propionyl]-2-methylphenoxy]-2-methylpropionic acid;
   5) [2-allyl-4-[3-[2-(2,4-dichlorophenyl)-5-isopropyl-4-oxazolyl]propionyl]phenoxy]acetic acid;
   6) [4-[3-[2-(2-hydroxy-4-chlorophenyl)-5-isopropyl-4-oxazolyl]propionyl]-2-methylphenoxy]acetic acid;
   7) [4-[3-[2-(2,4-dichlorophenyl)-5-isopropyl-4-oxazolyl]propionyl]-2-methylphenylsulfanyl]acetic acid;
   8) 2-[4-[3-[2-(2-hydroxy-4-chlorophenyl)-5-isopropyl-4-oxazolyl]propionyl]-2-methylphenoxy]-2-methylpropionic acid;
   9) [4-[3-[2-(2,4-dichlorophenyl)-5-isopropyl-4-oxazolyl]-1-propenyl]-2-methylphenoxy]acetic acid;
   10) [4-[3-[2-(2,4-dichlorophenyl)-5-isopropyl-4-oxazolyl]-1-propenyl]-2-methylphenoxy]acetic acid;
   11) [4-[3-[4-hexyl-2-(4-trifluoromethyl)phenyl-5-thiazolyl]propionyl]-2-methylphenoxy]acetic acid;
   12) 2-[4-[3-[4-hexyl-2-(4-trifluoromethyl)phenyl-5-thiazolyl]propionyl]-2-methylphenoxy]-2-methylpropionic acid;
   13) 2-[4-[3-[4-isopropyl-2-(4-trifluoromethyl)phenyl-5-thiazolyl]-1-propenyl]-2-methylphenoxy]-2-methylpropionic acid;
   14) [4-[3-[4-isopropyl-2-(4-trifluoromethyl)phenyl-5-thiazolyl]propionyl]-3-methylphenoxy]acetic acid;
   15) [4-[3-[2-(2,4-dichlorophenyl)-5-isopropyl-4-oxazolyl]propionyl]-3-methylphenoxy]acetic acid;
   16) [4-[3-[4-isopropyl-2-(4-trifluoromethyl)phenyl-5-thiazolyl]propionyl]-3-methylphenoxy]-2-methylpropionic acid;
   17) 2-[4-[3-[2-(2,4-dichlorophenyl)-5-isopropyl-4-oxazolyl]propionyl]-3-methylphenoxyl-2-methylpropionic acid;
   18) [4-[3-[4-isopropyl-2-(4-trifluoromethyl)phenyl-5-thiazolyl]propionyl]-2-propylphenoxy]acetic acid;
   19) 2-allyl-4-[3-[4-isopropyl-2-(4-trifluoromethyl)phenyl-5-thiazolyl]propionyl]phenoxyacetic acid;
   20) [4-[4-[2-(2,4-dichlorophenyl)-5-isopropyl-4-oxazolyl]-1-buten-2-yl]-2-methylphenoxy]acetic acid;
   21) 2-[4-[4-[2-(2,4-dichlorophenyl)-5-isopropyl-4-oxazolyl]-1-buten-2-yl]-2-methylpropionic acid;
   22) [4-[3-[2-(2,4-dichlorophenyl)-5-isopropyl-4-oxazolyl]-2-methylpropionyl]-2-methylphenoxy]acetic acid;
   23) 2-[4-[3-[2-(2,4-dichlorophenyl)-5-isopropyl-4-oxazolyl]-2-methylpropionyl]-2-methylphenoxy]-2-methylpropionic acid;
   24) [4-[3-[4-isopropyl-2-(4-trifluoromethyl)phenyl-5-thiazolyl]propenoyl]-2-methylphenoxy]acetic acid;
   25) 2-[4-[3-[4-isopropyl-2-(4-trifluoromethyl)phenyl-5-thiazolyl]propenoyl]-2-methylphenoxy]-2-methylpropionic acid;
   26) [4-[3-[4-isopropyl-2-(4-methoxyphenyl)-5-thiazolyl]propionyl]-2-methylphenoxy]propionic acid;
   27) [4-[3-[2-(3,5-dichlorophenyl)-4-isopropylthiazol-5-yl]propionyl]-2-methylphenoxy]acetic acid;
   28) 2-[4-[3-[2-(3,5-difluorophenyl)-4-isopropyl-5-thiazolyl]propionyl]-2-methylphenoxy]-2-methylpropionic acid;
   29) [4-[3-[4-isopropyl-2-(2-naphthyl)-5-thiazolyl]propionyl]-2-methylphenoxy]acetic acid;
   30) 2-[4-[3-[4-isopropyl-2-(2-naphthyl)-5-thiazolyl]propionyl]-2-methylphenoxyl-2-methylpropionic acid;
   31) [4-[3-[2-(4-butylphenyl)-4-isopropyl-5-thiazolyl]propionyl]-2-methylphenoxy]acetic acid;
   32) 2-[4-[3-[2-(4-butylphenyl)-4-isopropyl-5-thiazolyl]propionyl]-2-methylphenoxy]-2-methylpropionic acid;
   33) [4-[3-[4-isopropyl-2-(4-trifluoromethyl)phenyl-5-thiazolyl]propionyl]-2-chlorophenoxy]acetic acid;
   34) [4-[3-[2-(4-trifluoromethyl)phenyl-4-isopropyl-5-thiazolyl]propionyl]-2-chlorophenoxy]-2-methylpropionic acid;
   35) [4-[3-[2-(2,4-dichlorophenyl)-5-isopropyl-4-oxazolyl]propionyl]-2-chlorophenoxy]acetic acid;
   36) 2-[4-[3-[2-(2,4-dichlorophenyl)-5-isopropyl-4-oxazolyl]propionyl]-2-chlorophenoxy]-2-methylpropionic acid;
   37) [4-[3-[5-isopropyl-2-(4-trifluoromethyl)phenyl-4-thiazolyl]propionyl]-2-methylphenoxy]acetic acid;
   38) 2-[4-[3-[5-isopropyl-2-(4-trifluoromethyl)phenyl-4-thiazolyl]propionyl]-2-methylphenoxy]-2-methylpropionic acid;
   39) [4-[3-[2-(2,4-dichlorophenyl)-5-isopropyl-4-thiazolyl]propionyl]-2-methylphenoxy] acetic acid;
   40) 2-[4-[3-[2-(2,4-dichlorophenyl)-5-isopropyl-4-thiazolyl]propionyl]-2-methylphenoxy]-2-methylpropionic acid;
   41) [5-[3-[4-isopropyl-2-(4-trifluoromethyl)phenyl-5-thiazolyl]propionyl]-2-methylphenoxy]acetic acid;
   42) 2-[5-[3-[4-isopropyl-2-(4-trifluoromethyl)phenyl-5-thiazolyl]propionyl]-2-methylphenoxy]-2-methylpropionic acid;
   43) 2-[4-[3-[4-isopropyl-2-(4-trifluoromethyl)phenyl-5-thiazolyl]propionyl]-2-methylphenoxy]propionic acid;
   44) 4-[3-[4-methyl-2-(4-trifluoromethyl)phenyl-5-thiazolyl]propionyl]-2-methylphenoxy]acetic acid;
   45) 2-[4-[3-[4-hexyl-2-(4-trifluoromethyl)phenyl-5-thiazolyl]-1-propenyl]-2-methylphenoxy]-2-methylpropionic acid;
   46) 2-[5-[3-[4-hexyl-2-(4-trifluoromethyl)phenyl-5-thiazolyl]propionyl]-2-methylphenoxy]-2-methylpropionic acid;
   47) [4-[3-[4-ethyl-2-(4-trifluoromethyl)phenyl-5-thiazolyl]propionyl]-2-methylphenoxy]acetic acid;
   48) 2-[4-[3-[4-ethyl-2-(4-trifluoromethyl)phenyl-5-thiazolyl]propionyl]-2-methylphenoxy]-2-methylpropionic acid;
   49) [4-[3-[4-isopropyl-2-(4-methylphenyl)-5-thiazolyl]propionyl]-2-methylphenoxy]acetic acid; and
   50) 2-[4-[3-[4-isopropyl-2-(4-methylphenyl)-5-thiazolyl]propionyl]-2-methylphenoxy]-2-methylpropionic acid,
   a tautomer, a stereoisomer, or a pharmaceutically acceptable salt of the compound, or a solvate of any of the foregoing to a subject in need thereof.
(4-33) The method according to any one of (4-1) to (4-32), wherein the pharmaceutical composition is for topical administration to the skin.
(4-34) The method according to any one of (4-1) to (4-33), wherein the pharmaceutical composition promotes wound healing (for example, promotes wound healing during the inflammation phase and proliferation phase in the wound healing process).
(4-35) The method according to any one of (4-1) to (4-34), wherein the pharmaceutical composition suppresses exacerbation of wound (for example, expansion of wound surface during the inflammation phase and/or proliferation phase).
(4-36) The method according to any one of (4-1) to (4-35), wherein the pharmaceutical composition suppresses aggravation of wound surface and/or expansion of wound surface caused by exudate.
(4-37) The method according to any one of (4-1) to (4-36), wherein the pharmaceutical composition is for treatment of pressure ulcer or diabetic ulcer.
(4-38) A method for promoting wound healing (for example, promoting wound healing during the inflammation phase and proliferation phase in the wound healing process) in a mammalian subject (for example, human), which comprises administering a pharmaceutical composition comprising an effective amount of the compound, a tautomer, a stereoisomer, or a pharmaceutically acceptable salt of the compound, or a solvate of any of the foregoing described in any one of (4-1) to (4-32) to a subject in need thereof.
(4-39) A method for suppressing exacerbation of wound (for example, a method for suppressing expansion of wound surface during the inflammation phase and/or proliferation phase) in a mammalian subject (for example, human), which comprises administering a pharmaceutical composition comprising an effective amount of the compound, a tautomer, a stereoisomer, or a pharmaceutically acceptable salt of the compound, or a solvate of any of the foregoing described in any one of (4-1) to (4-32) to a subject in need thereof.
(4-40) A method for suppressing aggravation of wound surface and/or expansion of wound surface caused by exudate in a mammalian subject (for example, human), which comprises administering a pharmaceutical composition comprising an effective amount of the compound, a tautomer, a stereoisomer, or a pharmaceutically acceptable salt of the compound, or a solvate of any of the foregoing described in any one of (4-1) to (4-32) to a subject in need thereof.
(4-41) The method according to any one of (4-37) to (4-40), wherein the pharmaceutical composition is topically administered to the skin.
(4-42) The method according to any one of (4-37) to (4-41), which is for treating pressure ulcer or diabetic ulcer in a mammalian subject (for example, human).

Hereafter, the present invention will be explained in detail with reference to examples. However, the present invention is not limited to them.

### Examples

### 1. Effect of compound A ([3-[2-[4-isopropyl-2-(4-trifluoromethyl)phenyl-5-thiazolyl]ethyl]-5-methyl-1,2-benzisoxazol-6-yl]oxyacetic acid in rat pressure ulcer

### model

### Method for preparing ointment containing compound A

The compound A was weighed in the required amount, and polyethylene glycol 400 (PEG400) was added to the compound to prepare liquids having final concentrations of the compound of 0.01, 0.1, and 1.0 w/v %. In order to prepare ointments having final concentrations of the compound of 0.005, 0.05, and 0.5 w/w %, PEG4000 melted by warming at 65°C was added to the liquids, and the liquids were stirred until they solidified at room temperature to prepare ointments having concentrations of the compound of 0.005, 0.05, and 0.5 w/w %.

### Method of experiment

Pentobarbital sodium was intraperitoneally administered (50 mg/kg) to 8 weeks old Slc:SD rats, of which skins on the right third trochanter had been broadly depilated beforehand using an electric clipper and an electric shaver, 20 mg/kg of allobarbital was further intraperitoneally administered to anesthetize the rats, and the animals were fixed on wooden fixation plates in the prone position. Absorbent cotton was inserted between the femoral region and the fixation plate as a cushion, and a stainless steel bar (diameter 19 mm, length 50 cm) having a weight of 1.02 to 1.03 kg and attached with a rubber stopper (diameter 12 mm) was placed on the skin over the right third trochanter to give a pressure load (902.3 to 911.2 g/cm²) for 24 hours⁵⁾. After 24 hours, the pressure load was eliminated, a 5% glucose solution was orally administered to the rats, and two days after the elimination of the pressure load, the necrotic skin was surgically removed under isoflurane anesthesia to prepare a pressure ulcer model. As for the methods of the experiments, Hidetoshi Hamamoto et al., Clinical Pharmacology and Therapy (Yakuri to Rinsho), 15:255-262, 2005, Effect of MRX-201 on Wound Healing Dermal Burns and Decubitus Ulcer in Rat was referred to.

### Administration

After the preparation of the pressure ulcer model, the administration was performed once a day at a dose of 150 mg until healing was completed. The wound surface was cleaned with absorbent cotton dipped in physiological saline before the administration, and the ointment was applied to the whole wound surface with a spatula.

### Evaluation

Wound area was measured once a day after the start of the administration of the test substance until healing was completed. By using the measured wound area, wound area ratio (%) based on the wound area before the start of the administration was calculated. The number of days of the period from the start of the administration to healing was calculated as number of days required for healing.

### Statistical analysis

Homoscedasticity was examined by the Bartlett's method. When homoscedastic results were obtained, one-way layout analysis of variance was further performed, and when significant results were obtained, mean values were compared by the Tukey's method. When heteroscedastic results were obtained, Kruskal-Wallis H-test was performed, and when significant results were obtained, mean ranks were compared by the Tukey's method. For the Bartlett's method, one-way analysis of variance, and Kruskal-Wallis H-test, the statistical significance level was set at 5%, and for the Tukey's method, the statistical significance level was set at 5% and 1%.

### Test results

The wound area ratios of the vehicle-administered group and 0.005%, 0.05%, and 0.5% compound A-administered groups measured at various points are shown in Table 1. It was found that, compared with the vehicle-administered group, the wound areas became significantly smaller from the day 4 of the administration in the 0.005% compound A-administered group, and from the day 5 of the administration in the 0.05% and 0.5% compound A-administered groups. A tendency that exacerbation of wounds was suppressed by the administration of the compound A from the day 1 to day 3 of the administration was also observed. The test results demonstrated that administration of the compound A suppresses expansion of the wound surface during the inflammation phase and the proliferation phase in the wound healing process, and the administration of the compound A promotes wound healing during the inflammation phase and the proliferation phase in the wound healing process. It was also suggested that administration of the compound A possibly suppresses abnormal granulation or expansion of wound surface caused by excessive exudate in the inflammation phase and the proliferation phase.

**[Table 1]**

| Group | Vehicle | 0.005% compound A | 0.05% compound A | 0.5% compound A |
|---|---|---|---|---|
| Number of days of administration | | | | |
| 1 | 100.0±0.0 | 100.0±0.0 | 100.0±0.0 | 100.0±0.0 |
| 2 | 123.8±5.3 | 108.3±3.1 | 115.0±2.8 | 119.6±5.0 |
| 3 | 125.7±4.8 | 108.9±3.9 | 113.6±3.5 | 117.5±5.0 |
| 4 | 119.5±4.4 | 99.9±3.5 * | 104.9±3.1 | 109.5±4.6 |
| 5 | 111.9±4.4 | 92.1±3.7 ** | 95.1±3.7 * | 93.2±4.3 * |
| 6 | 100.7±3.7 | 76.4±5.5 ** | 84.4±2.8 | 84.9±4.3 |
| 7 | 91.5±3.6 | 66.4±4.7 ** | 73.5±2.7 * | 77.0±5.1 |
| 8 | 82.0±3.5 | 55.2±4.8 ** | 63.4±3.1 * | 65.3±4.4 |
| 9 | 66.7±3.8 | 45.3±4.3 ** | 49.8±4.2 * | 56.0±4.0 |
| 10 | 61.2±3.7 | 39.4±4.1 ** | 41.6±3.9 ** | 49.2±3.9 |
| 11 | 53.2±2.8 | 31.5±3.3 ** | 31.2±3.1 ** | 39.7±4.6 |
| 12 | 40.9±4.1 | 23.8±2.4 * | 22.3±3.6 ** | 28.3±3.4 |
| 13 | 37.1±4.4 | 19.6±2.3 * | 16.4±4.0 ** | 19.6±2.2 * |
| 14 | 30.0±3.0 | 14.4±2.5 * | 12.1±3.7 ** | 15.5±1.6 * |
| 15 | 23.4±2.7 | 10.3±2.1 | 8.6±3.2 ** | 8.0±0.9 * |
| 16 | 21.1±2.8 | 7.1±1.8 * | 7.5±3.3 ** | 5.6±0.7 * |
| 17 | 17.4±2.2 | 4.4±1.5 ** | 6.7±3.2 * | 3.7±0.5 ** |
| 18 | 15.3±2.3 | 4.9±1.6 | 7.8±3.8 | 2.2±0.6 ** |
| 19 | 12.6±2.1 | 3.3±1.5 * | 5.4±2.9 | 2.2±0.6 ** |
| 20 | 10±2.5 | 2.8±1.5 | 5.4±3.2 | 1.6±0.4 * |

There are shown values of the wound area ratios (%) of the rat calculated on the basis of the initial values of the same of the individual rats, which are taken as 100% (mean ± standard error for the rats (n = 10) up to the day 16 of the administration, and for the rats except for the healed rats from the day 17 and thereafter). The asterisks mentioned in the table indicate significant difference levels (*; p < 0.05, and **; p < 0.01) in contrast to the vehicle group.

The numbers of days required for healing are shown in Table 2. It was shown that, compared with the vehicle-administered group, the number of the days required for healing was significantly decreased in the 0.005% and 0.05% compound A⁻ administered groups.

**[Table 2]**

| Group | Number of days required for healing | Significant difference (vs. vehicle) |
|---|---|---|
| Vehicle | 24.8±0.8 | |
| 0.005% compound A | 20.1±1.4 | * |
| 0.05% compound A | 19.7±1.1 | * |
| 0.5% compound A | 21.0±1.2 | |

Here are shown the number of days required for healing of the pressure ulcer in the rat (mean ± standard error, n= 10). The asterisks mentioned in the table indicate that there was a significant difference (*; p < 0.05) in contrast to the vehicle-administered group. These test results revealed that transdermal administration of the compound A promotes recovery of wound surface.

### 2. Effect of compound A ([3-[2-[4-isopropyl-2-(4-trifluoromethyl)phenyl-5-thiazolyl]ethyl]-5-methyl-1,2-benzisoxazol-6-yl]oxyacetic acid on wound in diabetes mouse (db/db mouse) model

### Method for preparing ointment containing compound A

The compound A was weighed in the required amount, and polyethylene glycol 400 (PEG400) was added to the compound to prepare liquids having final concentrations of the compound of 0.001, 0.01, and 0.1 w/v %. In order to prepare ointments having final concentrations of the compound of 0.0005, 0.005, and 0.05 w/w %, PEG4000 melted by warming at 65°C was added to the liquids, and the liquids were stirred until they solidified at room temperature to prepare ointments having concentrations of the compound of 0.0005, 0.005, and 0.05 w/w %.

### Method of experiment

Diabetes mice (db/db mice) were anesthetized with isoflurane, and a 1.5-cm square of skin including the back median line as the center axis of each mouse was removed with ophthalmic scissors. A polyurethane film was stuck so as to cover the skin-lacking wound part and surrounding parts to prepare the wound.

### Administration

The administration was performed once a day at a volume of 0.05 mL. As for the administration method, the administration was performed with a disposable polypropylene injection syringe attached to a 23G needle by inserting the needle into a gap between the wound and the polyurethane film covering the wound part. On the day when the wound was produced and the days when the wound area was measured, the compound was administered to the wound part by using a disposable polypropylene injection syringe attached to a 23G needle before sticking the polyurethane film.

On days 14 to 20 of the administration (the day on which the administration was started is day 1 of the administration), the administration was performed by peeling off the polyurethane film under isoflurane anesthesia, cleaning the wound with absorbent cotton containing physiological saline, administering the ointment with a disposable polypropylene injection syringe, and then the polyurethane film was stuck on again.

### Evaluation

On days 1, 4, 8, 12, 16, and 21 of the administration, the wound part was traced. That is, after the polyurethane film was removed (in the case of the day 1 of the administration, before the administration and sticking of the film), a plastic sheet was placed on the wound part, the contour of the wound part was traced on the plastic sheet (tracing was performed by using a felt pen so that the external periphery of the wound part would coincide with the internal periphery of the line drawn with the felt pen), and a copy of the sheet on which tracing was performed was used as the source of raw data. The area of the inside of the traced line (mm²) was measured once using an area-line meter (PLANIX EX, Tamaya Technics Inc.) on the copy of the sheet on which tracing was performed. The results are expressed as ratio of change (%) calculated from the wound area (mm²) of each measurement day, and the wound area (mm²) of the day 1 of the wound creation (before administration).

### Statistical analysis

Significant difference test was performed by using a commercial statistical analysis program (SAS System, SAS Institute Japan, Inc.). Statistical significance levels lower than 5% were considered to be significant, and the results are indicated as those of the level lower than 5% (*; p < 0.05) and those of the level lower than 1% (**; p < 0.01).

As for the test method, the multiple comparison of Dunnett's method was used for the comparison between the vehicle-administered group and compound A-administered groups.

### Test results

The wound area ratios of the vehicle-administered group, and 0.0005%, 0.005%, and 0.05% compound A-administered groups measured at various points are shown in Table 3. It was found that the wound area became significantly smaller in the 0.05% compound A-administered group compared with the vehicle-administered group on the day 8 and day 12 of the administration.

**[Table 3]**

| Group | Vehicle | 0.0005% compound A | 0.005% compound A | 0.05% compound A |
|---|---|---|---|---|
| Number of days of administration | | | | |
| 1 | 100.0±0.0 | 100.0±0.0 | 100.0±0.0 | 100.0±0.0 |
| 4 | 101.0±3.1 | 96.9±1.7 | 97.2±2.4 | 96.0±2.1 |
| 8 | 99.5±1.9 | 95.2±2.4 | 95.0±2.2 | 90.3±2.6* |
| 12 | 88.4±1.5 | 82.9±2.7 | 84.1±1.6 | 78.6±1.6** |
| 16 | 75.9±2.0 | 73.4±2.7 | 72.1±1.7 | 69.2±2.1 |
| 21 | 69.5±2.1 | 67.6±3.8 | 69.1±3.0 | 65.5±2.1 |

Here are shown values of the wound area ratios (%) of the mouse back calculated on the basis of the initial values of wound areas of the individual rats, which are taken as 100% (mean ± standard error, n = 7 or 8, i.e., each group consisted of 8 mice, with the exception of the data of the day 16 and thereafter for the vehicle-administered group which was 7 mice). The asterisks mentioned in the table indicate significant difference levels (*; p < 0.05, and **; p < 0.01) in contrast to the vehicle-administered group.

These test results revealed that transdermal administration of the compound A is useful for the treatment of diabetic ulcer.

## Claims

1. A pharmaceutical composition for use in the treatment of wound, which comprises a compound represented by the following general formula (I), a tautomer, a stereoisomer, or a pharmaceutically acceptable salt of the compound, or a solvate of any of the foregoing: 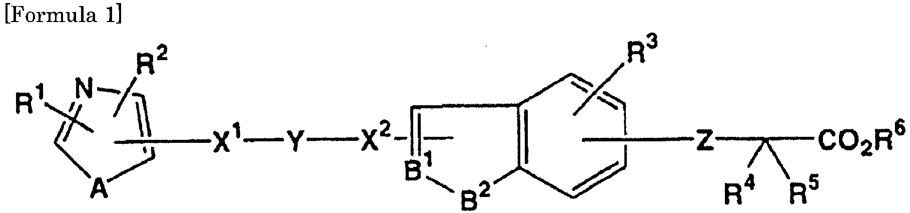 wherein
A represents O, S, or NR⁷, wherein R⁷ represents hydrogen atom, or an alkyl group having 1 to 8 carbon atoms,
B¹ represents CW or N, wherein W represents hydrogen atom, or an atomic bond,
B² represents O, S, or NR⁸, wherein R⁸ represents hydrogen atom, or an alkyl group having 1 to 8 carbon atoms,
X¹ and X² represent O, S, NH, NHC(=O), C(=O), C(=N-OR⁹), CH(OR¹⁰), C=C, C=C, or an atomic bond, wherein R⁹ and R¹⁰ represent hydrogen atom, or an alkyl group having 1 to 8 carbon atoms,
Y represents an alkylene chain having 1 to 8 carbon atoms which may have an alkyl group having 1 to 8 carbon atoms, or an alkyl group having 1 to 8 carbon atoms and substituted with 1 to 3 halogen atoms as a substituent,
Z represents NH, O, or S,
R¹ represents an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent, or a heterocyclic group having a 5- to 8-membered ring comprising 1 to 3 heteroatoms selected from nitrogen atom, oxygen atom, and sulfur atom, and the remainder carbon atoms as ring-constituting atoms (benzene ring may further condense to this heterocyclic ring),
R² represents an alkyl group having 2 to 8 carbon atoms, an alkyl group having 1 to 8 carbon atoms and substituted with 1 to 3 halogen atoms, a cycloalkyl group having 3 to 7 carbon atoms, an alkenyl group having 2 to 8 carbon atoms, an alkynyl group having 2 to 8 carbon atoms, an alkyl group substituted with an aryl group which may have a group or an atom selected from an alkyl group having 1 to 8 carbon atoms, an alkoxy group having 1 to 8 carbon atoms, an alkyl group having 1 to 8 carbon atoms and substituted with 1 to 3 halogen atoms, hydroxyl group, nitro group, amino group, phenyl group, pyridyl group, and a halogen atom as a substituent (the alkyl moiety thereof has 1 to 4 carbon atoms), or an alkyl group substituted with a 5- to 8-membered heterocyclic ring (the heterocyclic ring thereof comprises 1 to 3 heteroatoms selected from nitrogen atom, oxygen atom, and sulfur atom, and the remainder carbon atoms as ring-constituting atoms, and the alkyl moiety thereof has 1 to 4 carbon atoms),
R³ represents hydrogen atom, a halogen atom, trifluoromethyl group, an alkyl group having 1 to 8 carbon atoms, an alkenyl group having 2 to 8 carbon atoms, or an alkynyl group having 2 to 8 carbon atoms,
R⁴ and R⁵ represent hydrogen atom, an alkyl group having 1 to 8 carbon atoms, or an alkyl group having 1 to 8 carbon atoms and substituted with 1 to 3 halogen atoms, and
R⁶ represents hydrogen atom, an alkyl group having 1 to 8 carbon atoms and substituted with an amino group, an alkyl group having 1 to 8 carbon atoms, or an alkali metal,
provided that Z and R³ bond to the benzene ring, and X² does not bond to the benzene ring; or
a compound represented by the following general formula (II), a tautomer, a stereoisomer, or a pharmaceutically acceptable salt of the compound, or a solvate of any of the foregoing: 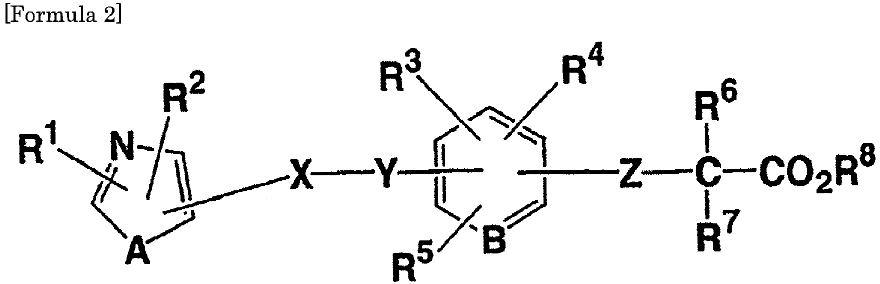
wherein R¹ represents a phenyl group, naphthyl group, pyridyl group, thienyl group, furyl group, quinolyl group, or benzothienyl group which may have a group or an atom selected from an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, and pyridyl group as a substituent, R² represents an alkyl group having 1 to 8 carbon atoms, an alkyl group having 1 to 8 carbon atoms and substituted with a halogen atom, an alkenyl group having 2 to 8 carbon atoms, an alkynyl group having 2 to 8 carbon atoms, a cycloalkyl group having a 3- to 7-membered ring, an alkyl group having 1 to 8 carbon atoms and substituted with a cycloalkyl group having a 3- to 7-membered ring, a phenyl group which may have a group or an atom selected from an alkoxy group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkynyl group having 2 to 8 carbon atoms, a halogen atom, an acyl group having 2 to 7 carbon atoms, a benzoyl group, a hydroxyl group, a nitro group, an amino group, a phenyl group, and a pyridyl group, a naphthyl group, or an alkyl group having 1 to 6 carbon atoms substituted with a pyridyl group, A represents oxygen atom, sulfur atom, or NR⁹, wherein R⁹ represents hydrogen atom, or an alkyl group having 1 to 8 carbon atoms, X represents an alkylene chain having 1 to 8 carbon atoms which may have a group selected from an alkyl group having 1 to 8 carbon atoms, an alkoxy group having 1 to 8 carbon atoms, and hydroxyl group as a substituent, and may contain a double bond, Y represents C(=O), C(=NOR¹⁰), CH(OR¹¹), CH=CH, C≡C, or C(=CH₂), wherein R¹⁰ and R¹¹ represent hydrogen atom, an alkyl group having 1 to 8 carbon atoms, an alkyl group having 1 to 8 carbon atoms and substituted with a halogen atom, an alkoxy group having 1 to 8 carbon atoms, an alkoxy group having 1 to 8 carbon atoms and substituted with a halogen atom, an alkenyl group having 2 to 8 carbon atoms, an alkynyl group having 2 to 8 carbon atoms, a halogen atom, an acyl group having 2 to 7 carbon atoms, benzoyl group, hydroxyl group, nitro group, amino group, phenyl group, or pyridyl group, B represents CH or nitrogen atom, Z represents oxygen atom or sulfur atom, R⁶ and R⁷ represent hydrogen atom, an alkyl group having 1 to 8 carbon atoms, or an alkyl group having 1 to 8 carbon atoms and substituted with a halogen atom, and R⁸ represents hydrogen atom, or an alkyl group having 1 to 8 carbon atoms,
provided that at least one of R³, R⁴, and R⁵ is not hydrogen atom.

2. The pharmaceutical composition according to claim 1 for the use according to claim 1, wherein the compound represented by the general formula (I) or the general formula (II), a tautomer, a stereoisomer, or a pharmaceutically acceptable salt of the compound, or a solvate of any of the foregoing is any one of the compounds of (a) to (j) described below:
(a) [3-[2-[4-isopropyl-2-(4-trifluoromethyl)phenyl-5-thiazolyl]ethyl]-5-methyl-1,2-benzisoxazol-6-yl]oxyacetic acid;
(b) 2-[[3-[2-[4-isopropyl-2-(4-trifluoromethyl)phenyl-5-thiazolyl]ethyl]-5-methyl-1,2-benzisoxazol-6-yl]oxy]-2-methylpropionic acid;
(c) [3-[2-[4-isopropyl-2-(4-trifluoromethyl)phenyl-5-thiazolyl]ethyl]-5-methyl-1,2-benzisoxazol-6-yl]thioacetic acid;
(d) [3-[2-[4-isopropyl-2-(4-trifluoromethyl)phenyl-5-thiazolyl]ethyl]-5-methyl-1,2-benzisoxazol-6-yl]aminoacetic acid;
(e) [3-[2-[2-(2,4-dichlorophenyl)-5-isopropyl-4-oxazolyl]ethyl]-5-methyl-1,2-benzisoxazol-6-yl]oxyacetic acid;
(f) 2-[[3-[2-[4-isopropyl-2-(4-trifluoromethyl)phenyl-5-thiazolyl]ethyl]-5-methyl-1,2-benzisoxazol-6-yl]oxy]-2-methylpropionic acid 2-piperidinoethyl ester hydrochloride;
(g) [[7-allyl-3-[2-[2-(2,4-dichlorophenyl)-5-isopropyl-4-oxazolyl]ethyl]-1,2-benzisoxazol-6-yl]oxy]acetic acid;
(h) 2-[[7-allyl-3-[2-[2-(2,4-dichlorophenyl)-5-isopropyl-4-oxazolyl]ethyl]-1,2-benzisoxazol-6-yl]oxy]-2-methylpropionic acid;
(i) [[7-propyl-3-[2-[2-(2,4-dichlorophenyl)-5-isopropyl-4-oxazolyl]ethyl]-1,2-benzisoxazol-6-yl]oxy]acetic acid; and
(j) 2-[[7-allyl-3-[2-[2-[(4-trifluoromethyl)phenyl]-4-isopropyl-5-thiazolyl]ethyl]-1,2-benzisoxazol-6-yl]oxy]-2-methylpropionic acid,
a tautomer, a stereoisomer, or a pharmaceutically acceptable salt of the compound, or a solvate of any of the foregoing.

3. The pharmaceutical composition according to claim 1 for the use according to claim 1, wherein the compound represented by the general formula (I) or the general formula (II), a tautomer, a stereoisomer, or a pharmaceutically acceptable salt of the compound, or a solvate of any of the foregoing is any one of the compounds of (1) to (50) described below:
(1) 2-[4-[3-[2-(2,4-dichlorophenyl)-5-isopropyl-4-oxazolyl]propionyl]-2-methylphenoxy]-2-methylpropionic acid;
(2) [4-[3-[2-(2,4-dichlorophenyl)-5-isopropyl-4-oxazolyl]propionyl]-2-methylphenoxy]acetic acid;
(3) [4-[3-[2-(4-trifluoromethyl)phenyl-4-isopropyl-5-thiazolyl]propionyl]-2-methylphenoxy]acetic acid;
(4) 2-[4-[3-[2-(4-trifluoromethyl)phenyl-4-isopropyl-5-thiazolyl]propionyl]-2-methylphenoxy]-2-methylprop ionic acid;
(5) [2-allyl-4-[3-[2-(2,4-dichlorophenyl)-5-isopropyl-4-oxazolyl]propionyl]phenoxy]acetic acid;
(6) [4-[3-[2-(2-hydroxy-4-chlorophenyl)-5-isopropyl-4-oxazolyl]propionyl]-2-methylphenoxy]acetic acid;
(7) [4-[3-[2-(2,4-dichlorophenyl)-5-isopropyl-4-oxazolyl]propionyl]-2-methylphenylsulfanyl]acetic acid;
(8) 2-[4-[3-[2-(2-hydroxy-4-chlorophenyl)-5-isopropyl-4-oxazolyl]propionyl]-2-methylphenoxy]-2-methylpropionic acid;
(9) [4-[3-[2-(2,4-dichlorophenyl)-5-isopropyl-4-oxazolyl]-1-propenyl]-2-methylphenoxy]acetic acid;
(10) [4-[3-[2-(2,4-dichlorophenyl)-5-isopropyl-4-oxazolyl]-1-propenyl]-2-methylphenoxy]acetic acid;
(11) [4-[3-[4-hexyl-2-(4-trifluoromethyl)phenyl-5-thiazolyl]propionyl]-2-methylphenoxy]acetic acid;
(12) 2-[4-[3-[4-hexyl-2-(4-trifluoromethyl)phenyl-5-thiazolyl]propionyl]-2-methylphenoxy]-2-methylpropionic acid;
(13) 2-[4-[3-[4-isopropyl-2-(4-trifluoromethyl)phenyl-5-thiazolyl]-1-propenyl]-2-methylphenoxy]-2-methylprop ionic acid;
(14) [4-[3-[4-isopropyl-2-(4-trifluoromethyl)phenyl-5-thiazolyl]propionyl]-3-methylphenoxy]acetic acid;
(15) [4-[3-[2-(2,4-dichlorophenyl)-5-isopropyl-4-oxazolyl]propionyl]-3-methylphenoxy]acetic acid;
(16) [4-[3-[4-isopropyl-2-(4-trifluoromethyl)phenyl-5-thiazolyl]propionyl]-3-methylphenoxy]-2-methylpropionic acid;
(17) 2-[4-[3-[2-(2,4-dichlorophenyl)-5-isopropyl-4-oxazolyl]propionyl]-3-methylphenoxy]-2-methylpropionic acid;
(18) [4-[3-[4-isopropyl-2-(4-trifluoromethyl)phenyl-5-thiazolyl]propionyl]-2-propylphenoxy]acetic acid;
(19) 2-allyl-4-[3-[4-isopropyl-2-(4-trifluoromethyl)phenyl-5-thiazolyl]propionyl]phenoxyacetic acid;
(20) [4-[4-[2-(2,4-dichlorophenyl)-5-isopropyl-4-oxazolyl]-1-buten-2-yl]-2-methylphenoxy]acetic acid;
(21) 2-[4-[4-[2-(2,4-dichlorophenyl)-5-isopropyl-4-oxazolyl-1-buten-2-yl]-2-methylpropionic acid;
(22) [4-[3-[2-(2,4-dichlorophenyl)-5-isopropyl-4-oxazolyl]-2-methylpropionyl]-2-methylphenoxy]acetic acid;
(23) 2-[4-[3-[2-(2,4-dichlorophenyl)-5-isopropyl-4-oxazolyl]-2-methylpropionyl]-2-methylphenoxy]-2-methylpropionic acid;
(24) [4-[3-[4-isopropyl-2-(4-trifluoromethyl)phenyl-5-thiazolyl]propenoyl]-2-methylphenoxy]acetic acid;
(25) 2-[4-[3-[4-isopropyl-2-(4-trifluoromethyl)phenyl-5-thiazolyl]propenoyl]-2-methylphenoxy]-2-methylpropionic acid;
(26) [4-[3-[4-isopropyl-2-(4-methoxyphenyl)-5-thiazolyl]propionyl]-2-methylphenoxy]propionic acid;
(27) [4-[3-[2-(3,5-dichlorophenyl)-4-isopropylthiazol-5-yl]propionyl]-2-methylphenoxy]acetic acid;
(28) 2-[4-[3-[2-(3,5-difluorophenyl)-4-isopropyl-5-thiazolyl]propionyl]-2-methylphenoxy]-2-methylpropionic acid;
(29) [4-[3-[4-isopropyl-2-(2-naphthyl)-5-thiazolyl]propionyl]-2-methylphenoxy]acetic acid;
(30) 2-[4-[3-[4-isopropyl-2-(2-naphthyl)-5-thiazolyl]propionyl]-2-methylphenoxy]-2-methylpropionic acid;
(31) [4-[3-[2-(4-butylphenyl)-4-isopropyl-5-thiazolyl]propionyl]-2-methylphenoxy]acetic acid;
(32) 2-[4-[3-[2-(4-butylphenyl)-4-isopropyl-5-thiazolyl]propionyl]-2-methylphenoxy]-2-methylpropionic acid;
(33) [4-[3-[4-isopropyl-2-(4-trifluoromethyl)phenyl-5-thiazolyl]propionyl]-2-chlorophenoxy]acetic acid;
(34) [4-[3-[2-(4-trifluoromethyl)phenyl-4-isopropyl-5-thiazolyl]propionyl]-2-chlorophenoxy]-2-methylpropionic acid;
(35) [4-[3-[2-(2,4-dichlorophenyl)-5-isopropyl-4-oxazolyl]propionyl]-2-chlorophenoxy]acetic acid;
(36) 2-[4-[3-[2-(2,4-dichlorophenyl)-5-isopropyl-4-oxazolyl]propionyl]-2-chlorophenoxy]-2-methylpropionic acid;
(37) [4-[3-[5-isopropyl-2-(4-trifluoromethyl)phenyl-4-thiazolyl]propionyl]-2-methylphenoxy]acetic acid;
(38) 2-[4-[3-[5-isopropyl-2-(4-trifluoromethyl)phenyl-4-thiazolyl]propionyl]-2-methylphenoxy]-2-methylpropionic acid;
(39) [4-[3-[2-(2,4-dichlorophenyl)-5-isopropyl-4-thiazolyl]propionyl]-2-methylphenoxy]acetic acid;
(40) 2-[4-[3-[2-(2,4-dichlorophenyl)-5-isopropyl-4-thiazolyl]propionyl]-2-methylphenoxy]-2-methylpropionic acid;
(41) [5-[3-[4-isopropyl-2-(4-trifluoromethyl)phenyl-5-thiazolyl]propionyl]-2-methylphenoxy]acetic acid;
(42) 2-[5-[3-[4-isopropyl-2-(4-trifluoromethyl)phenyl-5-thiazolyl]propionyl]-2-methylphenoxy]-2-methylprop ionic acid;
(43) 2-[4-[3-[4-isopropyl-2-(4-trifluoromethyl)phenyl-5-thiazolyl]propionyl]-2-methylphenoxy]propionic acid;
(44) 4-[3-[4-methyl-2-(4-trifluoromethyl)phenyl-5-thiazolyl]propionyl]-2-methylphenoxy]acetic acid;
(45) 2-[4-[3-[4-hexyl-2-(4-trifluoromethyl)phenyl-5-thiazolyl]-1-propenyl]-2-methylphenoxy]-2-methylpropionic acid;
(46) 2-[5-[3-[4-hexyl-2-(4-trifluoromethyl)phenyl-5-thiazolyl]propionyl]-2-methylphenoxy]-2-methylpropionic acid;
(47) [4-[3-[4-ethyl-2-(4-trifluoromethyl)phenyl-5-thiazolyl]propionyl]-2-methylphe noxy] acetic acid;
(48) 2-[4-[3-[4-ethyl-2-(4-trifluoromethyl)phenyl-5-thiazolyl]propionyl]-2-methylphenoxy]-2-methylprop ionic acid;
(49) [4-[3-[4-isopropyl-2-(4-methylphenyl)-5-thiazolyl]propionyl]-2-methylphenoxy]acetic acid; and
(50) 2-[4-[3-[4-isopropyl-2-(4-methylphenyl)-5-thiazolyl]propionyl]-2-methylphenoxy]-2-methylpropionic acid,
a tautomer, a stereoisomer, or a pharmaceutically acceptable salt of the compound, or a solvate of any of the foregoing.

4. The pharmaceutical composition according to any one of claims 1 to 3 for the use according to any one of claims 1 to 3, which is for topical administration to the skin.

5. The pharmaceutical composition according to any one of claims 1 to 4 for the use according to any one of claims 1 to 4, which promotes wound healing.

6. The pharmaceutical composition according to any one of claims 1 to 5 for the use according to any one of claims 1 to 5, which suppresses exacerbation of wound.

7. The pharmaceutical composition according to any one of claims 1 to 6 for the use according to any one of claims 1 to 6, which promotes wound healing during the inflammation phase and proliferation phase in the wound healing process.

8. The pharmaceutical composition according to any one of claims 1 to 7 for the use according to any one of claims 1 to 7, which suppresses expansion of wound surface during the inflammation phase and/or proliferation phase in the wound healing process.

9. The pharmaceutical composition according to any one of claims 1 to 8 for the use according to any one of claims 1 to 8, which suppresses aggravation of wound surface and/or expansion of wound surface caused by exudate.

10. The pharmaceutical composition according to any one of claims 1 to 9 for the use according to any one of claims 1 to 9, which is for treatment of pressure ulcer or diabetic ulcer.
